(19) Europäisches Patentamt
European Patent Office
Office européen des brevets

(11) EP 3 845 527 A1

(12) EUROPEAN PATENT APPLICATION
published in accordance with Art. 153(4) EPC

(43) Date of publication:
07.07.2021 Bulletin 2021/27

(21) Application number: 19855688.8

(22) Date of filing: 10.04.2019

(51) Int Cl.:
C07D 401/12 (2006.01) C07D 491/04 (2006.01)
A61K 31/506 (2006.01) A61P 35/00 (2006.01)

(86) International application number:
PCT/CN2019/081987

(87) International publication number:
WO 2020/042618 (05.03.2020 Gazette 2020/10)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
KH MA MD TN

(30) Priority: 27.08.2018 CN 201810979525

(71) Applicant: Beijing Yuezhikangtai Biomedicines Co., Ltd.
Beijing 102200 (CN)

(72) Inventors:
- DUAN, Maosheng
Beijing 102200 (CN)
- LIU, Jiale
Beijing 102200 (CN)
- TIAN, Shihong
Beijing 102200 (CN)
- DENG, Wei
Beijing 102200 (CN)
- YAN, Chao
Beijing 102200 (CN)
- ZHAO, Le
Beijing 102200 (CN)

(74) Representative: Viering, Jentschura & Partner mbB
Patent- und Rechtsanwälte
Hamborner Straße 53
40472 Düsseldorf (DE)

# (54) MULTI-SUBSTITUTED PYRIDONE DERIVATIVES AND MEDICAL USE THEREOF

(57) The present invention relates to multi-substituted pyridone derivatives and therapeutic use thereof. In particular, the present invention relates to a compound of formula (I), a preparation method therefor, a pharmaceutical composition comprising the same, as well as use thereof as a tyrosine kinase inhibitor, in particular, use thereof in treating a disease associated with tyrosine kinase activity. Each substituent in the formula (I) is defined as in the specification.

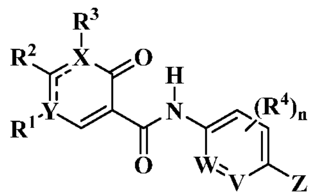

(I)

EP 3 845 527 A1

## Description

### TECHNICAL FIELD

The present invention relates to a multi-substituted pyridone derivative, a preparation method therefor, a pharmaceutical composition comprising the same, as well as use thereof as a tyrosine kinase inhibitor, in particular, use thereof in the manufacture of a medicament for treating a disease associated with tyrosine kinase activity.

### BACKGROUND

Receptor tyrosine kinases (RTKs) are multi-domain transmembrane proteins that function as sensors for receptors and ligands outside the cell membrane. When the ligand binds to the receptor, the receptor is induced to dimerize on the cell membrane and activate a kinase domain inside the membrane, resulting in tyrosine phosphorylation and further activation of a series of signaling pathways downstream. To date, nearly 60 receptor tyrosine kinases have been found in the human genome database, which broadly regulate metabolic process of cells, including survival, growth, differentiation, proliferation, adhesion, and death.

The TAM family of receptor tyrosine kinases has three members: Axl, Mer, and Tyro3. The TAM receptors have two ligands, Gas 6 and Protein S, *in vivo.* All of them can bind to Gas 6 with the binding affinities being Axl > Mer > Tyro3. Protein S binds only to Mer and Tyro3. The TAM receptors, upon autophosphorylation, result in signaling that regulates various cellular responses, including cell survival, induced differentiation, migration, and adhesion, and also controls vascular smooth muscle homeostasis, platelet function, microthrombus stability, red blood cell formation, etc. Furthermore, TAM receptors play a key role in immunity and inflammation. They promote the phagocytosis of apoptotic cells and stimulate the induced differentiation of NK cells.

Axl is an important regulator of cell survival, proliferation, aggregation, migration, and adhesion. It is widely expressed in cells and organs, such as monocytes, macrophages, platelets, endothelial cells, cerebellum, heart, skeletal muscle, liver, and kidney. Meanwhile, the Axl gene is overexpressed or ectopically expressed in a plurality of cancer cells, hematopoietic cells, interstitial cells, and endothelial cells. The overexpression of Axl is particularly prominent in various leukemias and most solid tumors. Furthermore, Axl is expressed even higher in metastatic or malignant tumors than in normal tissues or in primary tumors, and its overexpression is closely related to poor therapeutic effect of clinical treatment.

Among the three members of the TAM family of receptor tyrosine kinases, Axl and Tyro3 are most similar in gene structure, while Axl and Mer are most similar in amino acid sequence of the tyrosine kinase domain. Axl is an important regulator of cell survival, proliferation, aggregation, migration, and adhesion. It is widely expressed in cells and organs, such as monocytes, macrophages, platelets, endothelial cells, cerebellum, heart, skeletal muscle, liver, and kidney. Particularly, the Axl gene is overexpressed or ectopically expressed in a plurality of cancer cells. The overexpression of Axl is particularly prominent in various leukemias and most solid tumors. Furthermore, Axl is expressed even higher in cells of metastatic or malignant tumors than in normal tissues or in cells of primary tumors, and its overexpression is closely related to poor therapeutic effect of clinical treatment.

Axl is also involved in drug resistance caused by different mechanisms in a variety of tumor cells. Overexpression of Axl kinase has become an important marker for the development of drug resistance in cancer patients. Inhibition of Axl receptor tyrosine kinase can reduce the activation of pro-survival signals of tumor cells, block the invasion of tumors, and increase the sensitivity of targeted drug therapy, radiotherapy, and chemotherapy.

Mer was originally discovered from the lymphoblastoid expression library and identified as a class of phosphoproteins. It can regulate activation of macrophages, promote phagocytosis of apoptotic cells, boost platelet aggregation, and maintain the stability of blood clots *in vivo.* Mer is overexpressed or ectopically expressed in many types of cancers, such as leukemia, non-small cell lung cancer, melanoma, and prostate cancer, resulting in activation of several typical oncogenic signaling pathways.

Tyro3 was found in a research of PCR-based clone. Although its involvement in signaling pathways downstream requires further research, its involvement in PI3K-AKT and RAF-MAPK signaling pathways has been confirmed.

Several tinib tyrosine kinase inhibitors, such as cabozantinib and crizotinib, contain Axl kinase inhibitor activity. However, they are multi-target molecules with no selectivity. Therefore, no new TAM inhibitor is available as a therapeutic agent for patients at present.

c-MET belongs to the family of transmembrane receptor tyrosine kinases (RTKs) that have autophosphorylation activity. The c-MET receptor contains an intracellular tyrosine kinase catalytic domain with 4 key tyrosine residues that regulate enzymatic activity. These tyrosine residues form docking sites for several signaling proteins, resulting in biological responses. c-MET was first isolated from a cell line derived from human osteosarcoma and it is expressed predominantly on epithelial cells. During embryonic development and adulthood, the c-MET receptor is expressed on the surface of epithelial cells in many organs, including the liver, pancreas, prostate, kidney, muscle, and bone marrow.

HGF is the only ligand for c-MET. When c-MET binds to it, receptor dimerization is triggered, which activates tyrosine kinases in the c-MET intracytoplasmic protein kinase domain, resulting in the autophosphorylation of tyrosines (Tyrl349, Tyrl356) at c-MET carboxyl terminal. c-MET activation recruits adaptor proteins Gab1 and Grb2, and activates Shp2, Ras, and ERK/MAPK. Various effector proteins in cytoplasm are recruited to phosphorylated carboxyl terminal and rapidly phosphorylated so that various signaling pathways in cells, such as PI3K/AKT, Ras-Rac/Rho, MAPK and STAT3 pathways, are activated, promoting various biological functions, such as cell deformation, proliferation, anti-apoptosis, cell separation, movement, and invasion.

The normal HGF/c-MET signaling pathway is involved in various physiological processes in different cells and different differentiation stages of the cells, such as controlling the migration of the cells in the process of embryonic development and repairing after tissue damage. However, abnormal conditions of c-MET include overexpression, sustained activation of constitutive kinases, gene amplification, paracrine and autocrine activation by HGF, c-MET mutations and subsequent alterations, etc. The abnormal HGF/c-MET signaling pathways play a very important role in the development and progression of tumors and can induce the growth, invasion, drug resistance, and survival of the tumors. Therefore, effectively inhibiting the HGF/c-MET signaling pathways in tumor cells would have significant therapeutic effect on a variety of cancers.

Therefore, the invention provides new tyrosine kinase inhibitors. They can be used alone or in combination with other active drugs as new therapeutic agents for the treatment of cancer.

**SUMMARY**

The inventors, through intensive research, have designed and synthesized a series of pyridone-containing compounds, which exhibited inhibition activity against tyrosine kinases Axl and MET, and are suitable for use in the manufacture of medicaments for treating a disease associated with Axl and MET.

It is therefore an object of the present invention to provide a compound of formula (I),

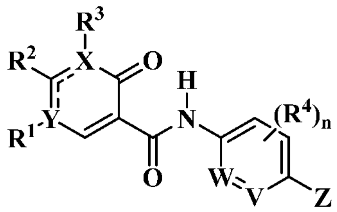

(I)

or a mesomer, a racemate, an enantiomer, or a diastereomer thereof, or a mixture thereof, or a pharmaceutically acceptable salt thereof,
wherein,

"⎓" represents a single bond or a double bond;
X and Y are each independently C or N;
W and V are each independently CH or N;
Z is

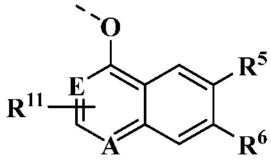 , 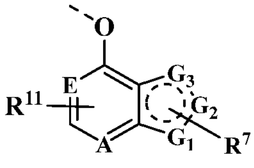 , 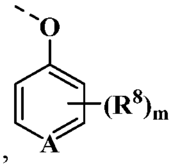 , or 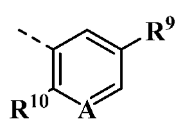 ;

A and E are each independently CH or N;
$G_1$, $G_2$, and $G_3$ are each independently C, N, O, or S;
$R^1$ is hydrogen, halogen, alkyl, alkenyl, alkynyl, cycloalkyl, or heterocyclyl, wherein the alkyl, alkenyl, alkynyl, cycloalkyl, or heterocyclyl is optionally further substituted with one or more groups selected from halogen, amino, nitro, cyano, hydroxyl, sulfydryl, carboxyl, an ester group, oxo, $NR^aR^b$, alkyl, alkoxy, cycloalkyl, heterocyclyl, aryl, and heteroaryl;
$R^2$ is hydrogen, halogen, hydroxyl, oxo, cyano, alkyl, cycloalkyl, heterocyclyl, $NR^aR^b$, $NHC(O)R^a$, or $NHS(O)_mR^a$,

wherein the alkyl, cycloalkyl, or heterocyclyl is optionally further substituted with one or more groups selected from halogen, hydroxyl, sulfydryl, cyano, alkyl, $OR^a$, $SR^a$, $NR^aR^b$, and $C(O)NR^aR^b$;

$R^3$ is alkenyl, alkynyl, aryl, or heteroaryl, wherein the alkenyl, alkynyl, aryl, or heteroaryl is optionally further substituted with $R^a$;

$R^4$ is hydrogen, halogen, cyano, alkyl, haloalkyl, alkoxy, or haloalkoxy;

$R^5$ and $R^6$ are each independently hydrogen, halogen, cyano, $OR^a$, $SR^a$, $O(CH_2)_pNR^aR^b$, $O(CH_2)_pOR^a$, $NR^aR^b$, $C(O)R^a$, $C(O)OR^a$, $OC(O)R^a$, $C(O)NR^aR^b$ and $OC(O)NR^aR^b$, or

$R^5$ and $R^6$ together with the atoms to which they are attached form oxacycloalkyl, in which the oxygen atom is attached to the phenyl ring;

$R^7$ is hydrogen, halogen, $NR^aR^b$, alkyl, cycloalkyl, heterocyclyl, aryl, or heteroaryl, wherein the alkyl, cycloalkyl, heterocyclyl, aryl, or heteroaryl is optionally further substituted with one or more groups selected from halogen, amino, nitro, cyano, hydroxyl, sulfydryl, carboxyl, an ester group, oxo, alkyl, alkoxy, cycloalkyl, heterocyclyl, aryl, and heteroaryl;

each $R^8$ is independently hydrogen, halogen, $NR^aR^b$, alkyl, cycloalkyl, heterocyclyl, aryl, and heteroaryl, wherein the alkyl, cycloalkyl, heterocyclyl, aryl, or heteroaryl is optionally further substituted with one or more groups selected from halogen, amino, nitro, cyano, hydroxyl, sulfydryl, carboxyl, an ester group, oxo, alkyl, alkoxy, cycloalkyl, heterocyclyl, aryl, and heteroaryl;

$R^9$ is aryl or heteroaryl, wherein the aryl or heteroaryl is optionally further substituted with one or more Q groups;

Q is halogen, alkyl, cycloalkyl, heterocyclyl, aryl, heteroaryl, $OR^a$, $SR^a$, $O(CH_2)_pNR^aR^b$, $O(CH_2)_pOR^a$, $NR^aR^b$, $C(O)R^a$, $C(O)OR^a$, $OC(O)R^a$, $C(O)NR^aR^b$, or $OC(O)NR^aR^b$, wherein the alkyl, cycloalkyl, heterocyclyl, aryl, or heteroaryl is optionally further substituted with one or more groups selected from halogen, amino, nitro, cyano, hydroxyl, sulfydryl, carboxyl, an ester group, oxo, alkyl, alkoxy, cycloalkyl, heterocyclyl, aryl, and heteroaryl;

$R^{10}$ is selected from hydrogen, halogen, alkyl, and $NR^aR^b$, wherein the alkyl is optionally further substituted with one or more halogens;

$R^{11}$ is hydrogen, halogen, cyano, amino, hydroxyl, alkyl, cycloalkyl, heterocyclyl, aryl, or heteroaryl, wherein the alkyl, cycloalkyl, heterocyclyl, aryl, or heteroaryl is optionally further substituted with one or more groups selected from halogen, amino, nitro, cyano, hydroxyl, sulfydryl, carboxyl, an ester group, oxo, alkyl, alkoxy, cycloalkyl, heterocyclyl, aryl, and heteroaryl;

$R^a$ and $R^b$ are each independently hydrogen, halogen, hydroxyl, nitro, cyano, oxo, alkyl, alkoxy, cycloalkyl, heterocyclyl, aryl, and heteroaryl, wherein the alkyl, alkoxy, cycloalkyl, heterocyclyl, aryl or heteroaryl is optionally further substituted with one or more groups selected from halogen, amino, nitro, cyano, hydroxyl, sulfydryl, carboxyl, an ester group, oxo, alkyl, alkoxy, cycloalkyl, heterocyclyl, aryl and heteroaryl, or

$R^a$ and $R^b$ together with the nitrogen atom to which they are attached form nitrogen-containing heterocyclyl, wherein the nitrogen-containing heterocyclyl is optionally further substituted with one or more groups selected from halogen, amino, nitro, cyano, oxo, hydroxyl, sulfydryl, carboxyl, an ester group, alkyl, alkoxy, cycloalkyl, heterocyclyl, aryl, and heteroaryl;

m is an integer from 1 to 4;

n is an integer from 0 to 4;

p is an integer from 1 to 6; and

any one or more H atoms in the compound of formula (I) are optionally further substituted with D atoms.

In a preferred embodiment, the compound of formula (I) according to the present invention is a compound of formula (II),

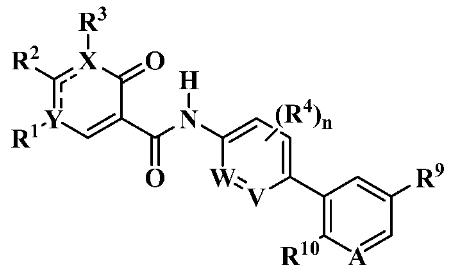

(II)

or a mesomer, a racemate, an enantiomer, or a diastereomer thereof, or a mixture thereof, or a pharmaceutically acceptable salt thereof,

wherein, , $R^1$, $R^2$, $R^3$, $R^4$, $R^9$, $R^{10}$, X, Y, W, V, A, and n are defined as in formula (I).

In a preferred embodiment, the compound of formula (I) according to the present invention is a compound of formula (III),

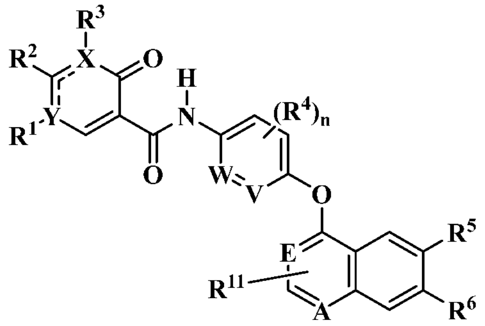

(III)

or a mesomer, a racemate, an enantiomer, or a diastereomer thereof, or a mixture thereof, or a pharmaceutically acceptable salt thereof,

wherein, , $R^1$, $R^2$, $R^3$, $R^4$, $R^5$, $R^6$, $R^{11}$, X, Y, W, V, A, E, and n are defined as in formula (I).

In a preferred embodiment, the compound of formula (II) according to the present invention is provided, wherein W and V are CH, and A is N.

In a preferred embodiment, the compound of formula (II) according to the present invention is a compound of formula (IV), (V), or (VI),

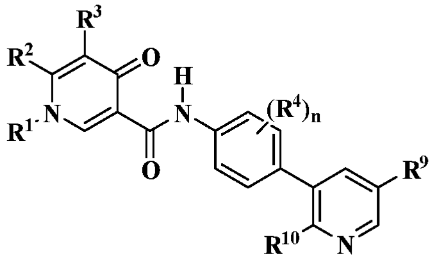

(IV)

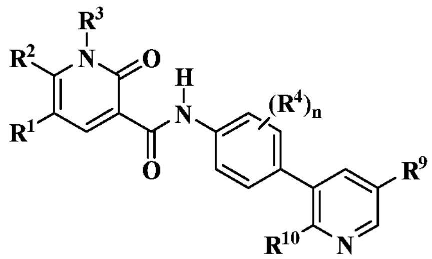

(V)

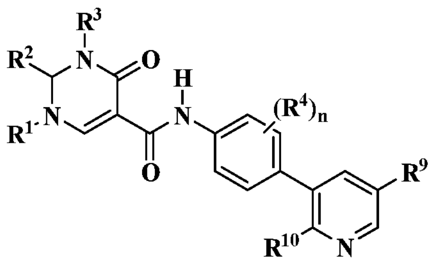

(VI)

wherein, $R^1$, $R^2$, $R^3$, $R^4$, $R^9$, $R^{10}$ and n are defined as in formula (I).

In another preferred embodiment, the compound of the formula (IV), (V) or (VI) according to the present invention is provided,

wherein $R^9$ is aryl or heteroaryl, and preferably $C_6$-$C_{10}$ aryl or 5-7 membered heteroaryl, wherein the aryl or heteroaryl is optionally further substituted with one or more Q groups;

Q is alkyl, cycloalkyl, heterocyclyl, $OR^a$, $SR^a$, $O(CH_2)_pNR^aR^b$, $O(CH_2)_pOR^a$, $NR^aR^b$, $OC(O)R^a$, or $OC(O)NR^aR^b$, wherein the alkyl, cycloalkyl or heterocyclyl is optionally further substituted with one or more groups selected from halogen, amino, nitro, cyano, hydroxyl, sulfydryl, carboxyl, an ester group, oxo, alkyl, alkoxy, cycloalkyl, heterocyclyl, aryl and heteroaryl;

$R^a$ and $R^b$ are each independently hydrogen or alkyl, wherein the alkyl is optionally further substituted with one or more groups selected from halogen, cycloalkyl, and heterocyclyl, or

$R^a$ and $R^b$ together with the nitrogen atom to which they are attached form nitrogen-containing heterocyclyl, preferably 5-7 membered nitrogen-containing heterocyclyl, wherein the nitrogen-containing heterocyclyl is optionally further substituted with one or more alkyl groups; and p is an integer from 1 to 6.

In another preferred embodiment, the compound of formula (IV), (V), or (VI) according to the present invention is provided, wherein $R^{10}$ is amino.

In a preferred embodiment, the compound of formula (III) according to the present invention is provided, wherein W and V are CH, E is CH, and A is N.

In another preferred embodiment, the compound of formula (III) according to the present invention is a compound of formula (VII), (VIII), or (IX),

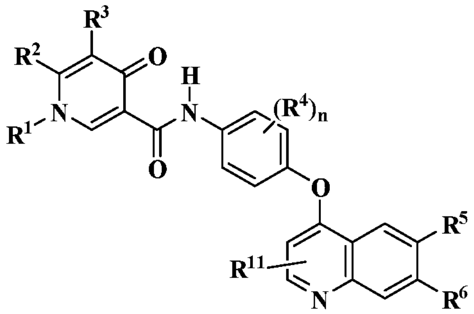

(VII)

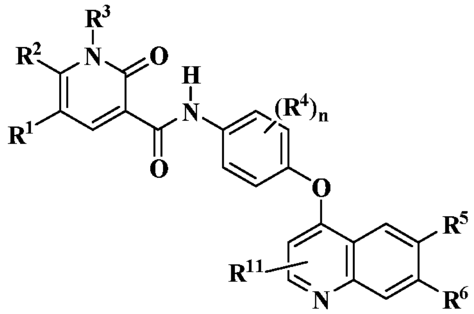

(VIII)

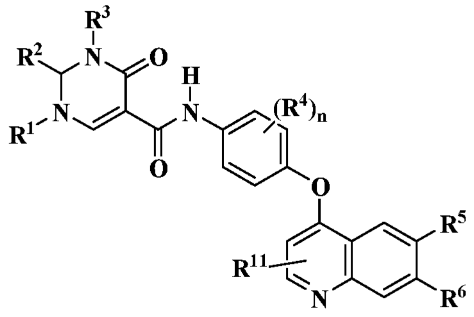

(IX)

wherein, $R^1$, $R^2$, $R^3$, $R^4$, $R^5$, $R^6$, $R^{11}$ and n are defined as in formula (I).

In another preferred embodiment, the compounds of formula (VII), (VIII), or (IX) according to the present invention are provided, wherein,

$R^5$ and $R^6$ are each independently cyano, $OR^a$, $SR^a$, $O(CH_2)_pNR^aR^b$, $O(CH_2)_pOR^a$, $NR^aR^b$, $OC(O)R^a$, $C(O)NR^aR^b$, or $OC(O)NR^aR^b$, or

$R^5$ and $R^6$ together with the atoms to which they are attached form oxacycloalkyl, in which the oxygen atom is attached to the phenyl ring;

$R^a$ and $R^b$ are each independently hydrogen and alkyl, wherein the alkyl is optionally further substituted with one or more groups selected from halogen, cycloalkyl, and heterocyclyl, or

$R^a$ and $R^b$ together with the nitrogen atom to which they are attached form nitrogen-containing heterocyclyl, preferably 5-7 membered nitrogen-containing heterocyclyl, wherein the nitrogen-containing heterocyclyl is optionally further substituted with one or more alkyl groups; and

p is an integer from 1 to 6.

In another preferred embodiment, the compounds of formula (VII), (VIII), or (IX) according to the present invention are provided, wherein $R^{11}$ is hydrogen or amino.

In another preferred embodiment, the compound of formula (I) according to the present invention is provided, wherein, $R^1$ is halogen, alkyl, alkenyl, cycloalkyl, and heterocyclyl, wherein the alkyl, alkenyl, cycloalkyl, or heterocyclyl is optionally further substituted with one or more groups selected from halogen, amino, nitro, cyano, hydroxyl, sulfydryl, carboxyl, an ester group, oxo, $NR^aR^b$, alkyl, alkoxy, cycloalkyl, heterocyclyl, aryl, and heteroaryl;

$R^a$ and $R^b$ are each independently hydrogen or alkyl, or

$R^a$ and $R^b$ together with the nitrogen atom to which they are attached form nitrogen-containing heterocyclyl, wherein the nitrogen-containing heterocyclyl is optionally further substituted with one or more alkyl groups; and

preferably, the cycloalkyl is $C_3$-$C_7$ cycloalkyl, and the heterocyclyl is 5-7 membered heterocyclyl, and more preferably, the cycloalkyl or heterocyclyl is

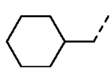 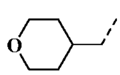 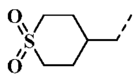 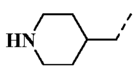 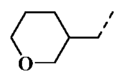 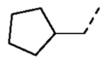 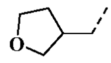

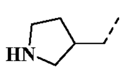 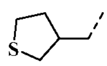 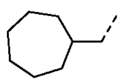 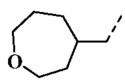 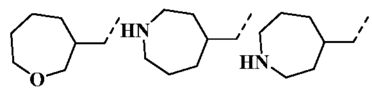 .

In another preferred embodiment, the compound of formula (I) according to the present invention is provided, wherein, $R^2$ is hydrogen, oxo, cyano, hydroxyl, alkyl, cycloalkyl, or heterocyclyl, and preferably oxo, cyano, hydroxyl, and alkyl, wherein the alkyl, cycloalkyl, or heterocyclyl is optionally further substituted with one or more groups selected from halogen, hydroxyl, sulfydryl, cyano, alkyl, $OR^a$, $SR^a$, $NR^aR^b$, and $C(O)NR^aR^b$; and

$R^a$ and $R^b$ are each independently hydrogen and alkyl, wherein the alkyl is optionally further substituted with one or more halogens, or

$R^a$ and $R^b$ together with the nitrogen atom to which they are attached form nitrogen-containing heterocyclyl, wherein the nitrogen-containing heterocyclyl is optionally further substituted with one or more alkyl groups.

In another preferred embodiment, the compound of formula (I) according to the present invention is provided, wherein $R^3$ is alkynyl, aryl, and heteroaryl, wherein the alkynyl, aryl, or heteroaryl is optionally further substituted with $R^a$;

$R^a$ is hydrogen, halogen, cyano, alkyl, alkoxy, or cycloalkyl, wherein the alkyl, alkoxy, or cycloalkyl is optionally further substituted with one or more halogens;

the aryl is preferably $C_6$-$C_{10}$ aryl, and more preferably phenyl; and

the heteroaryl is preferably 5-10 membered heteroaryl, and more preferably pyridinyl, pyridazinyl, thienyl, furanyl, imidazolyl, pyrazolyl, thiazolyl, or thiadiazolyl.

In another preferred embodiment, the compound of formula (I) according to the present invention is provided, wherein $R^4$ is hydrogen, halogen, cyano, alkyl, haloalkyl, alkoxy, and haloalkoxy, preferably halogen; and n is an integer from 0 to 2.

In another preferred embodiment, the compound of formula (I) according to the present invention is provided, wherein any hydrogen atom in the compound is substituted with a deuterium atom.

Exemplary compounds of the present invention include, but are not limited to:

| Example No. | Structure & Name |
|---|---|
| 1 | 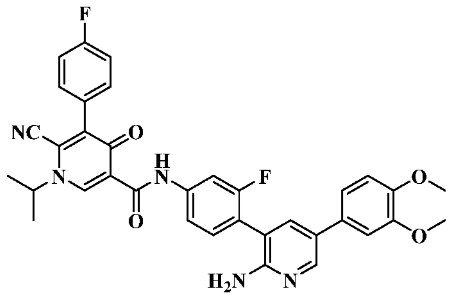 |
| | *N*-(4-(2-Amino-5-(3,4-dimethoxyphenyl)pyridin-3-yl)-3-fluorophenyl)-6-cyano-5-(4-fluorophenyl)-1-isopropyl-4-oxo-1,4-dihydropyridine-3-carboxamide |
| 2 | 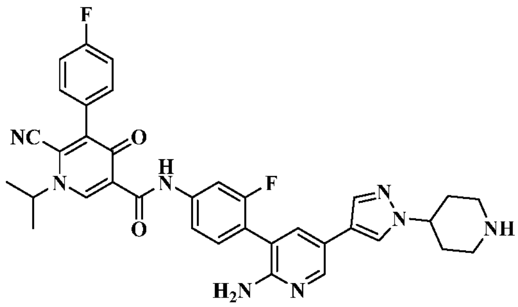 |
| | *N*-(4-(2-Amino-5-(1-(piperidin-4-yl)-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide |
| 3 | 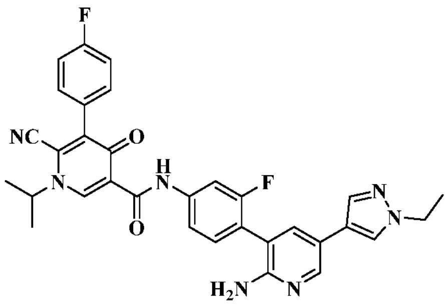 |
| | *N*-(4-(2-Amino-5-(1-ethyl-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide |

(continued)

| Example No. | Structure & Name |
|---|---|
| 4 | 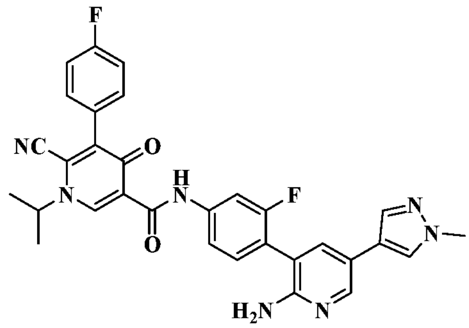 |
| | *N*-(4-(2-Amino-5-(1-methyl-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide |
| 5 | 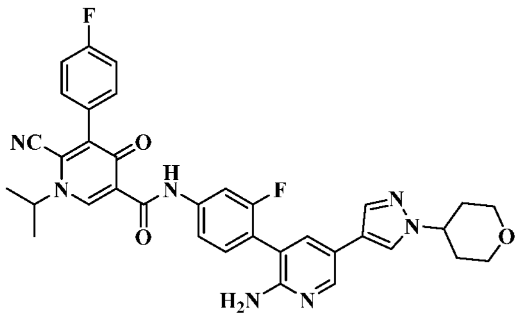 |
| | *N*-(4-(2-Amino-5-(1-(tetrahydro-2*H*-pyran-4-yl)-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide |
| 6 | 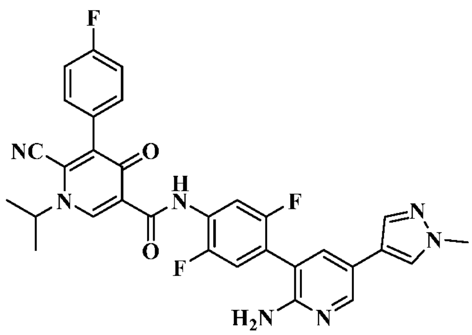 |
| | *N*-(4-(2-Amino-5-(1-methyl-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide |
| 7 | 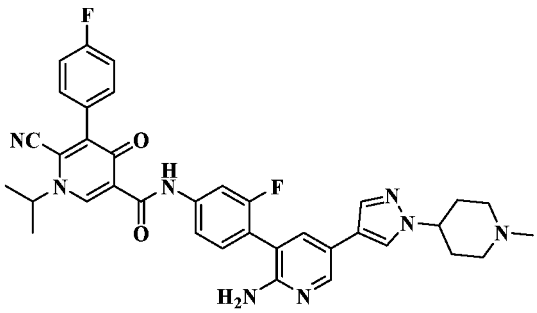 |
| | *N*-(4-(2-Amino-5-(1-(1-methylpiperidin-4-yl)-1*H*-pyrazo1-4-yl)pyridin-3-yl)-3-fluorophenyl)-6-cyano-5-(4-fluorophenyl)-1-isopropyl-4-oxo-1,4-dihydropyridine-3-carboxamide |

(continued)

| Example No. | Structure & Name |
|---|---|
| 8 | 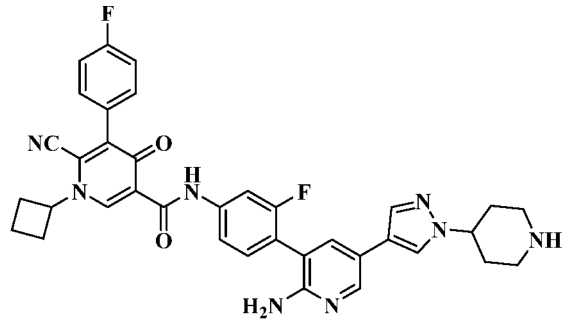 |
| | *N*-(4-(2-Amino-5-(1-(piperidin-4-yl)-1*H*-pyrazol-4-yl)pyridin-3-yl)-3-fluorophenyl)-6-cyano-5-(4-fluorophenyl)-1-cyclobutyl-4-oxo-1,4-dihydropyridine-3-carboxamide |
| 9 | 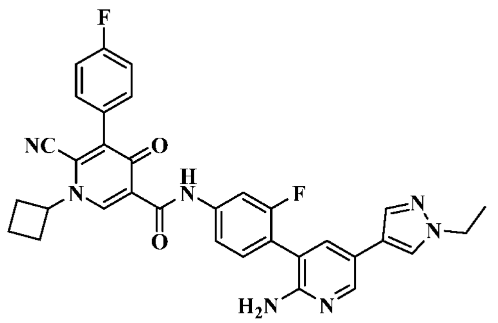 |
| | *N*-(4-(2-Amino-5-(1-ethyl-1*H*-pyrazol-4-yl)pyridin-3-yl)-3-fluorophenyl)-6-cyano-5-(4-fluorophenyl)-1-cyclobutyl-4-oxo-1,4-dihydropyridine-3-carboxamide |
| 10 | 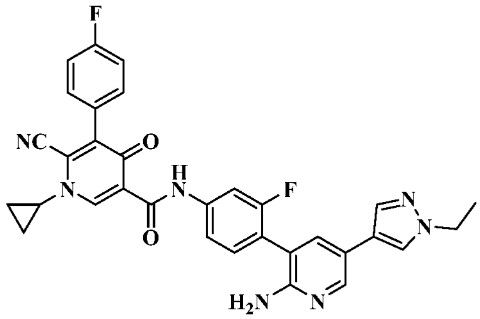 |
| | *N*-(4-(2-Amino-5-(1-ethyl-1*H*-pyrazol-4-yl)pyridin-3-yl)-3-fluorophenyl)-6-cyano-5-(4-fluorophenyl)-1-cyclopropyl-4-oxo-1,4-dihydropyridine-3-carboxamide |
| 11 | 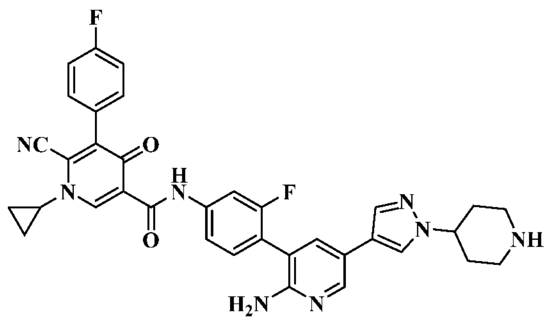 |
| | *N*-(4-(2-Amino-5-(1-(piperidin-4-yl)-1*H*-pyrazol-4-yl)pyridin-3-yl)-3-fluorophenyl)-6-cyano-5-(4-fluorophenyl)-1-cyclopropyl-4-oxo-1,4-dihydropyridine-3-carboxamide |

(continued)

| Example No. | Structure & Name |
|---|---|
| 12 | 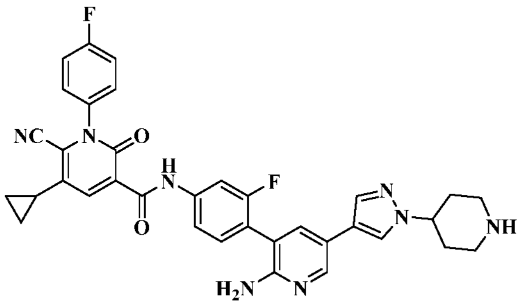 |
| | *N*-(4-(2-Amino-5-(1-(piperidin-4-yl)-1*H*-pyrazol-4-yl)pyridin-3-yl)-3-fluoro-phenyl)-6-cyano-5-cyclopropyl-1-(4-fluorophenyl)-2-oxo-1,2-dihydropyridine-3- carboxamide |
| 13 | 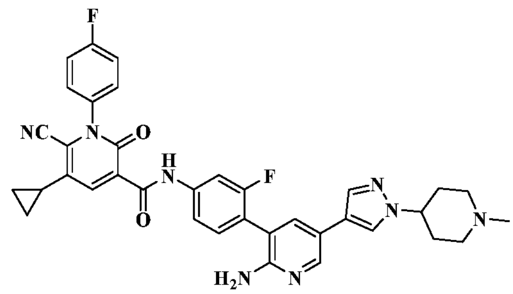 |
| | *N*-(4-(2-Amino-5-(1-(1-methylpiperidin-4-yl)-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide |
| 14 | 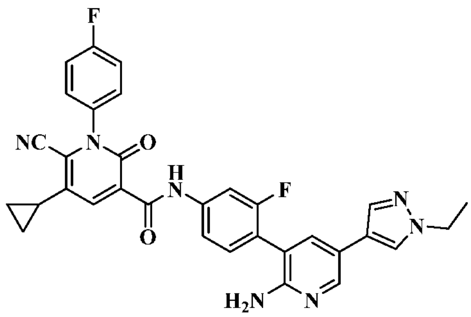 |
| | *N*-(4-(2-Amino-5-(1-ethyl-1*H*-pyrazol-4-yl)pyridin-3-yl)-3-fluorophenyl)-6-cyano-5-cyclopropyl-1-(4-fluorophenyl)-2-oxo-l,2-dihydropyridine-3-carboxamide |
| 15 | 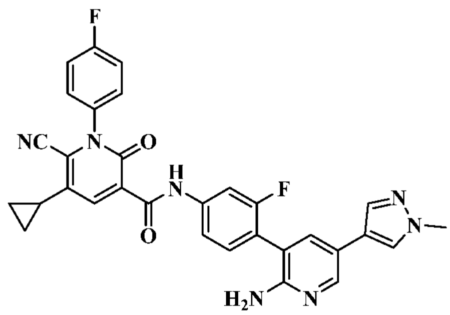 |
| | *N*-(4-(2-Amino-5-(1-methyl-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide |

(continued)

| Example No. | Structure & Name |
|---|---|
| 16 | 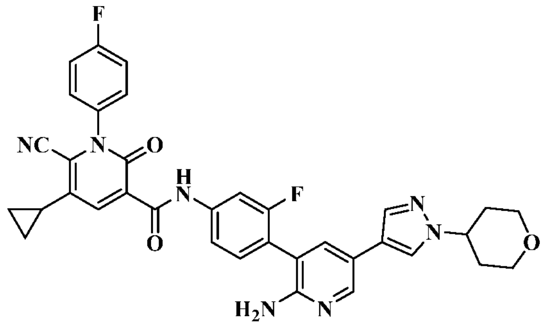 |
| | *N*-(4-(2-Amino-5-(1-(tetrahydro-2*H*-pyran-4-yl)-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide |
| 17 | 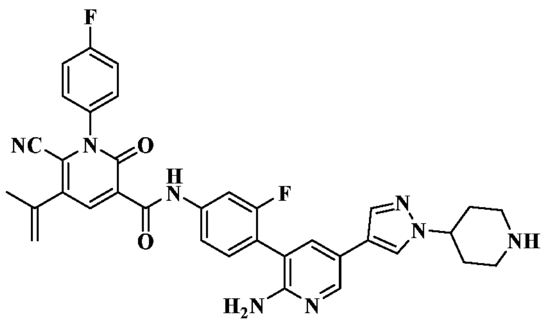 |
| | *N*-(4-(2-Amino-5-(1-(piperidin-4-yl)-1H-pyrazol-4-yl)pyridin-3-yl)-3-fluorophenyl)-6-cyano-1-(4-fluorophenyl)-2-oxo-5-(prop-1-en-2-yl)-1,2-dihydropyridine-3-carboxamide |
| 18 | 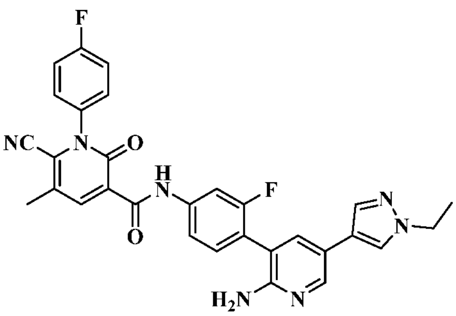 |
| | *N*-(4-(2-Amino-5-(1-ethyl-1*H*-pyrazol-4-yl)pyridin-3-yl)-3-fluorophenyl)-6-cyano-1-(4-fluorophenyl)-5-methyl-2-oxo-1,2-dihydropyridine-3-carboxamide |
| 19 | 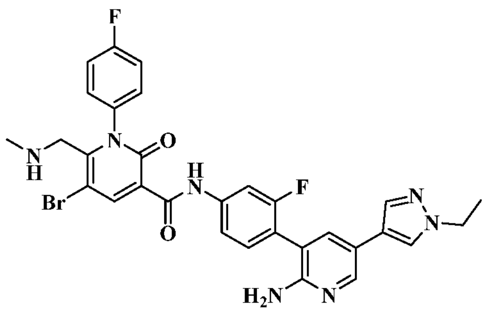 |
| | *N*-(4-(2-Amino-5-(1-ethyl-1*H*-pyrazol-4-yl)pyridin-3-yl)-3-fluorophenyl)-5-bromo-1-(4-fluorophenyl)-6-((methylamino)methyl)-2-oxo-1,2-dihydropyridine-3-carboxamide |

(continued)

| Example No. | Structure & Name |
|---|---|
| 20 | 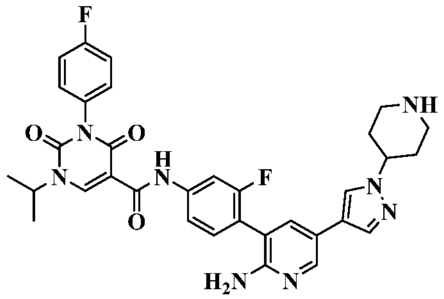 |
| | *N*-(4-(2-Amino-5-(1-(piperidin-4-yl)-1*H*-pyrazol-4-yl)pyridin-3-yl)-3-fluorophenyl)-3-(4-fluorophenyl)-1-isopropyl-2,4-dioxo-1,2,3,4-tetrahydropyrimidine-5-carboxamide |
| 21 | 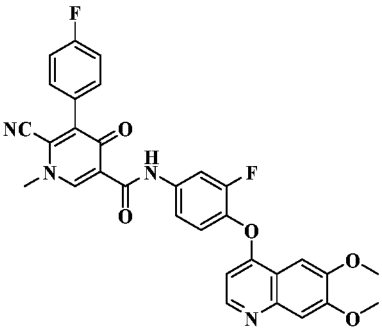 |
| | 6-Cyano-*N*-(4-((6,7-dimethoxyquinolin-4-yl)oxy)-3-fluorophenyl)-5-(4-fluorophenyl)-1-methyl-4-oxo-1,4-dihydropyridine-3-carboxamide |
| 22 | 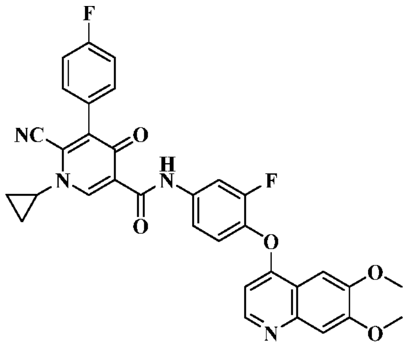 |
| | 6-Cyano-*N*-(4-((6,7-dimethoxyquinolin-4-yl)oxy)-3-fluorophenyl)-5-(4-fluorophenyl)-1-cyclopropyl-4-oxo-1,4-dihydropyridine-3-carboxamide |

(continued)

| Example No. | Structure & Name |
|---|---|
| 23 | 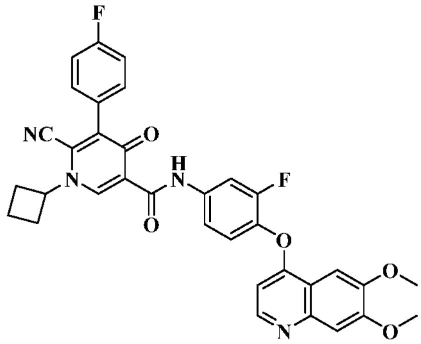 |
| | 6-Cyano-*N*-(4-((6,7-dimethoxyquinolin-4-yl)oxy)-3-fluorophenyl)-5-(4-fluorophenyl)-1-cyclobutyl-4-oxo-1,4-dihydropyridine-3-carboxamide |
| 24 | 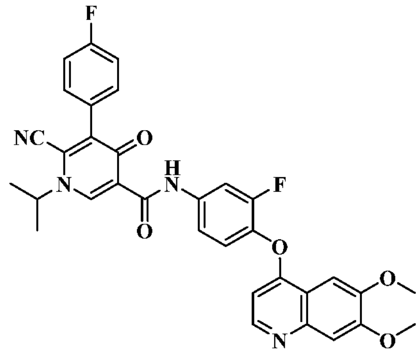 |
| | 6-Cyano-*N*-(4-((6,7-dimethoxyquinolin-4-yl)oxy)-3-fluorophenyl)-5-(4-fluorophenyl)-1-isopropyl-4-oxo-1, 4-dihydropyridine-3-carboxamide |
| 25 | 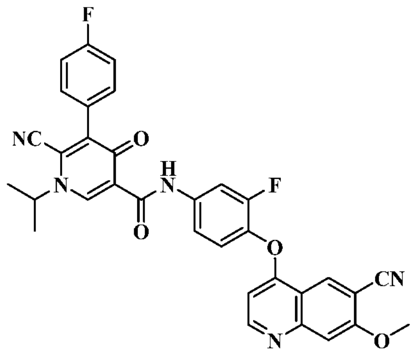 |
| | 6-Cyano-*N*-(4-((6-cyano-7-methoxyquinolin-4-yl)oxy)-3-fluorophenyl)-5-(4-fluorophenyl)-1-isopropyl-4-oxo-1,4-dihydropyridine-3-carboxamide |

(continued)

| Example No. | Structure & Name |
|---|---|
| 26 | 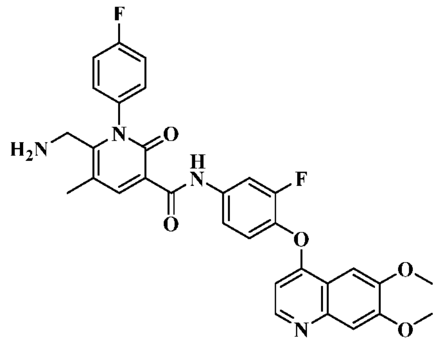 |
| | 6-(Aminomethyl)-*N*-(4-((6,7-dimethoxyquinolin-4-yl)oxy)-3-fluorophenyl)-1-(4-fluorophenyl)-5-methyl-2-oxo-1,2-dihydro-1,2-dihydropyridine-3-carboxamide |
| 27 | 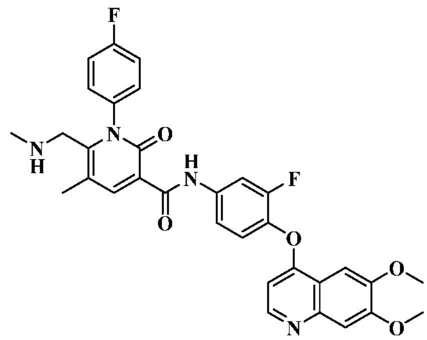 |
| | (4-((6,7-Dimethoxyquinolin-4-yl)oxy)-3-fluorophenyl)-1-(4-fluorophenyl)-5-methyl-6-((methylamino) methyl)-2-oxo-1,2-dihydropyridine-3-carboxamide |
| 28 | 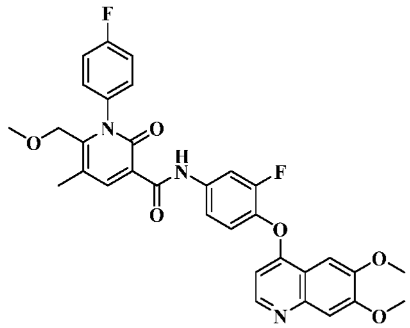 |
| | 5-Methyl-*N*-(4-((6,7-dimethoxyquinolin-4-yl)oxy)-3-fluorophenyl)-1-(4-fluorophenyl)-6-(methoxymethyl)-2-oxo-1,2-dihydropyridine-3-carboxamide |
| 29B | 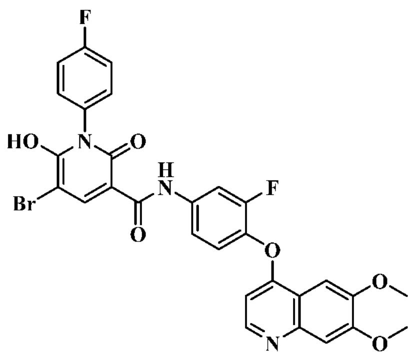 |

(continued)

| Example No. | Structure & Name |
|---|---|
| | 5-Bromo-*N*-(4-((6,7-dimethoxyquinolin-4-yl)oxy)-3-fluorophenyl)-1-(4-fluorophenyl)-6-hydroxy-2-oxo-1,2-dihydropyridine-3-carboxamide |
| 29A | 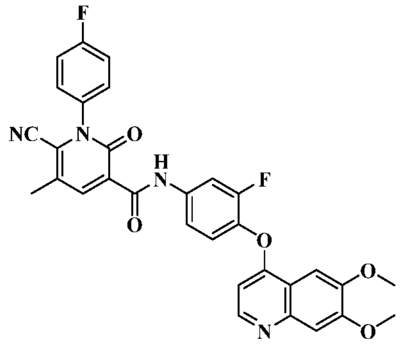 |
| | 6-Cyano-*N*-(4-((6,7-dimethoxyquinolin-4-yl)oxy)-3-fluorophenyl)-1-(4-fluorophenyl)-5-methyl-2-oxo-1,2-dihydropyridine-3-carboxamide |
| 30 | 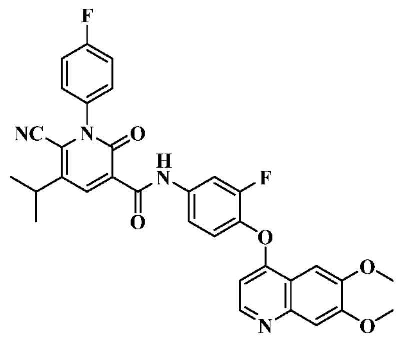 |
| | 6-Cyano-*N*-(4-((6,7-dimethoxyquinolin-4-yl)oxy)-3-fluorophenyl)-1-(4-fluorophenyl)-5-isopropyl-2-oxo-1,2-dihydropyridine-3-carboxamide |
| 31 | 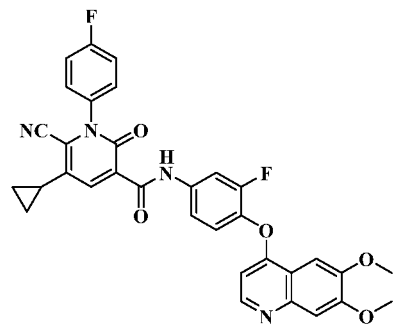 |
| | 6-Cyano-5-cyclopropyl-*N*-(4-((6,7-dimethoxyquinolin-4-yl)oxy)-3-fluorophenyl)-1-(4-fluorophenyl)-2-oxo-1,2-dihydropyridine-3-carboxamide |

(continued)

| Example No. | Structure & Name |
|---|---|
| 32 | 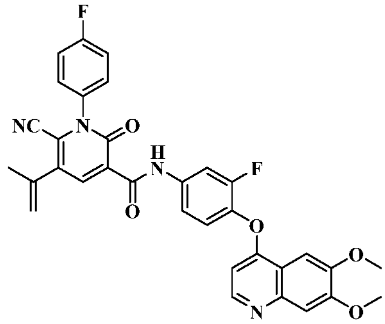 |
| | 6-Cyano-*N*-(4-((6,7-dimethoxyquinolin-4-yl)oxy)-3-fluorophenyl)-1-(4-fluorophen yl)-2-oxo-5-(prop-1-en-2-yl)-1,2-dihydropyridine-3-carboxamide |
| 33 | 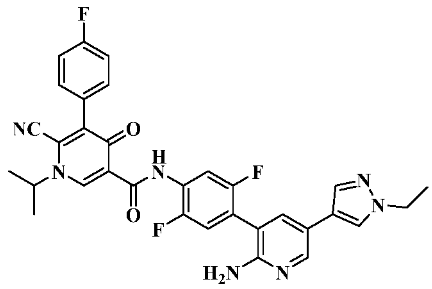 |
| | *N*-(4-(2-Amino-5-(1-ethyl-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide |
| 34 | 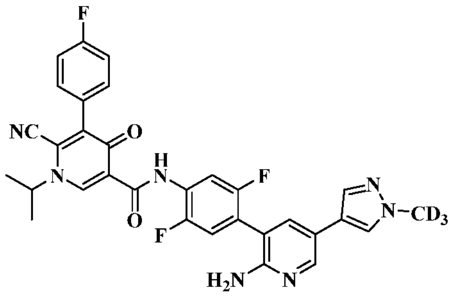 |
| | *N*-(4-(2-Amino-5-(1-methyl-$d_3$-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide |

or a mesomer, a racemate, an enantiomer, or a diastereomer thereof, or a mixture thereof, or a pharmaceutically acceptable salt thereof.

The present invention further provides a method for preparing the compound of formula (I), comprising the step of:

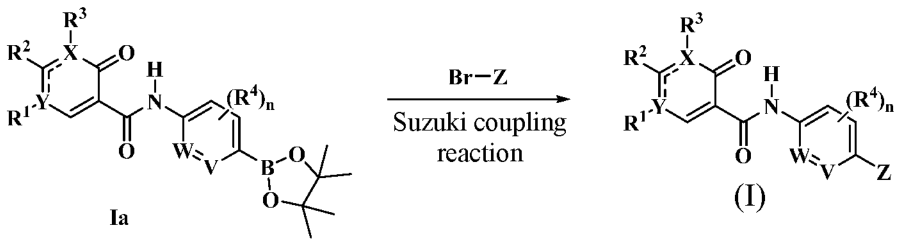

coupling pinacol borate Ia and aromatic bromide (Br-Z) *via* the Suzuki reaction in a solvent in the presence of a catalyst and a base to form the compound of formula (I), the catalyst being preferably $Pd(dppf)_2$, the base being preferably $K_2CO_3$, and the solvent being preferably dioxane and water;
wherein $R^1$, $R^2$, $R^3$, $R^4$, Z, X, Y, W, V, and n are each defined as in formula (I).

The present invention further provides another method for preparing the compound of formula (I), comprising the step of:

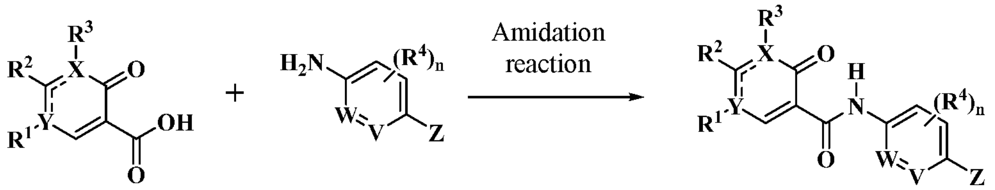


Ig Ic (I)

coupling carboxylic acid compound Ig and aromatic amine compound Ic in the presence of a coupling agent and a base to form the compound of formula (I), the coupling agent being preferably HATU, and the base being preferably triethylamine;
wherein $R^1$, $R^2$, $R^3$, $R^4$, Z, X, Y, W, V and n are each defined as in formula (I).

The present invention further provides another method for preparing the compound of formula (I), comprising the steps of:
when $R^2$ = CN,

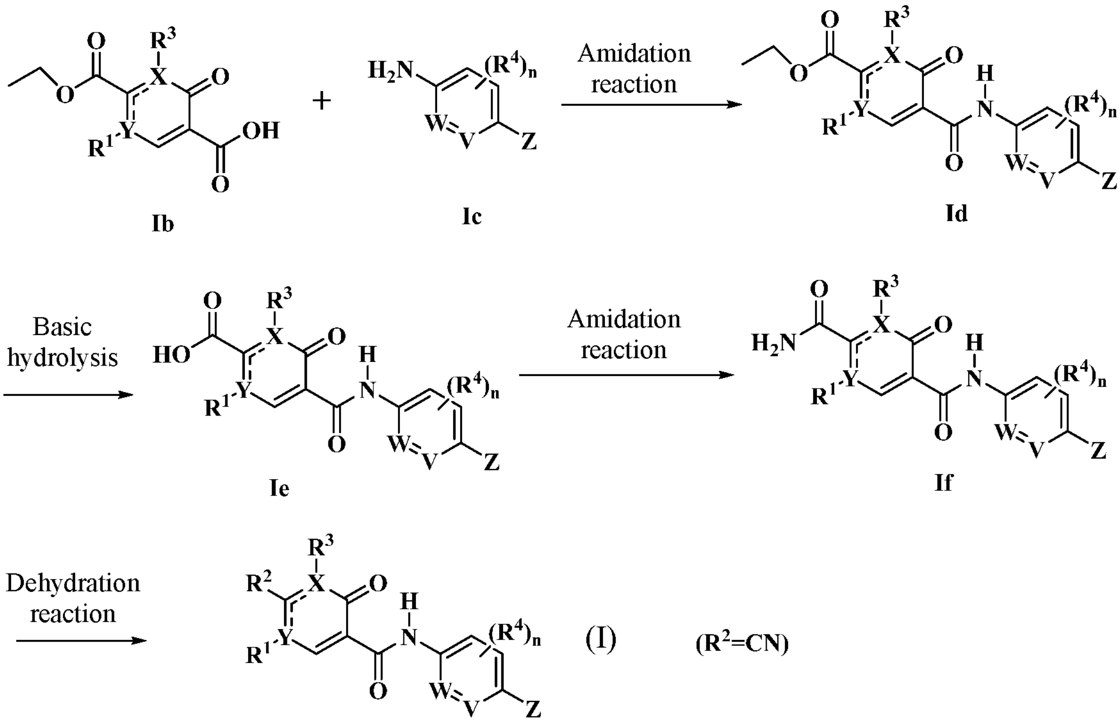


Ib Ic Id

Ie If

(I) ($R^2$=CN)

step 1: reacting carboxylic acid (Ib) and aromatic amine (Ic) in the presence of a coupling agent and a base to form arylamide intermediate (Id), the coupling agent being preferably HATU, and the base being preferably *N,N*-diisopropylethylamine;
step 2: hydrolyzing arylamide intermediate (Id) in a solvent in the presence of a base to form carboxylic acid intermediate (Ie), the base being preferably LiOH, and the solvent being preferably methanol-water solution;
step 3: reacting carboxylic acid intermediate (Ie) and ammonium chloride in the presence of a catalyst and a base to form dicarboxamide intermediate (If), the catalyst being preferably PyBrOP, and the base being preferably DIPEA; and
step 4: dehydrating dicarboxamide intermediate (If) in the presence of a dehydrating agent and a base to form

compound of formula (I), the dehydrating agent being preferably trifluoroacetic anhydride, and the base being preferably triethylamine;

wherein, $R^1$, $R^3$, $R^4$, Z, X, Y, W, V and n are each defined as in formula (I).

The present invention further provides a pharmaceutical composition comprising the compound of formula (I) according to the present invention and a pharmaceutically acceptable carrier or excipient.

In another aspect, the present invention provides use of the compound of formula (I) according to the present invention or the pharmaceutical composition comprising the same as a tyrosine kinase inhibitor, wherein the tyrosine kinase is preferably Axl, Mer, Tyro3, or c-MET.

The present invention further provides use of the compound of formula (I) according to the present invention or the pharmaceutical composition comprising the same in the manufacture of a medicament for treating a disease associated with tyrosine kinase activity, wherein the disease can be bladder cancer, breast cancer, cervical cancer, colorectal cancer, intestinal cancer, gastric cancer, head and neck cancer, kidney cancer, liver cancer, lung cancer, ovarian cancer, prostate cancer, testicular cancer, esophageal cancer, gallbladder cancer, pancreatic cancer, thyroid cancer, skin cancer, brain cancer, bone cancer, soft tissue cancer, leukemia, or lymph cancer, preferably leukemia, liver cancer, lung cancer, kidney cancer, breast cancer, or colorectal cancer, and more preferably leukemia, liver cancer, lung cancer, kidney cancer, breast cancer, gastric cancer, or colorectal cancer.

The present invention further provides use of the compound of formula (I) according to the present invention or the pharmaceutical composition comprising the same as a tyrosine kinase inhibitor, wherein the tyrosine kinase is preferably Axl, Mer, Tyro3, or c-MET.

The present invention further provides use of the compound of formula (I) according to the present invention or the pharmaceutical composition comprising the same as a medicament for treating a disease associated with tyrosine kinase activity, wherein the disease can be bladder cancer, breast cancer, cervical cancer, colorectal cancer, intestinal cancer, gastric cancer, head and neck cancer, kidney cancer, liver cancer, lung cancer, ovarian cancer, prostate cancer, testicular cancer, esophageal cancer, gallbladder cancer, pancreatic cancer, thyroid cancer, skin cancer, brain cancer, bone cancer, soft tissue cancer, leukemia, or lymph cancer, preferably leukemia, liver cancer, lung cancer, kidney cancer, breast cancer, or colorectal cancer, and more preferably leukemia, liver cancer, lung cancer, kidney cancer, breast cancer, gastric cancer, or colorectal cancer.

The present invention provides a method for treating a disease associated with tyrosine kinase activity, which comprises administering to a patient in need thereof a therapeutically effective amount of the compound of formula (I) according to the present invention or the pharmaceutical composition comprising the same, wherein the tyrosine kinase is preferably Axl, Mer, Tyro3, or c-MET, and the disease can be bladder cancer, breast cancer, cervical cancer, colorectal cancer, intestinal cancer, gastric cancer, head and neck cancer, kidney cancer, liver cancer, lung cancer, ovarian cancer, prostate cancer, testicular cancer, esophageal cancer, gallbladder cancer, pancreatic cancer, thyroid cancer, skin cancer, brain cancer, bone cancer, soft tissue cancer, leukemia, or lymph cancer, preferably leukemia, liver cancer, lung cancer, kidney cancer, breast cancer, or colorectal cancer, and more preferably leukemia, liver cancer, lung cancer, kidney cancer, breast cancer, gastric cancer, or colorectal cancer.

The compound of formula (I) of the present invention can form pharmaceutically acceptable acid addition salts with acids according to conventional methods in the art to which the present invention pertains. The acids include inorganic acids and organic acids, and particularly preferably hydrochloric acid, hydrobromic acid, sulfuric acid, phosphoric acid, methanesulfonic acid, ethanesulfonic acid, *p*-toluenesulfonic acid, benzenesulfonic acid, naphthalenedisulfonic acid, acetic acid, propionic acid, lactic acid, trifluoroacetic acid, maleic acid, citric acid, fumaric acid, oxalic acid, tartaric acid, benzoic acid, etc.

The compound of formula (I) of the present invention can form pharmaceutically acceptable base addition salts with bases according to conventional methods in the art to which the present invention pertains. The bases include inorganic bases and organic bases. Acceptable organic bases include diethanolamine, ethanolamine, *N*-methylglucamine, triethanolamine, tromethamine and the like, and acceptable inorganic bases include aluminum hydroxide, calcium hydroxide, potassium hydroxide, sodium carbonate, sodium hydroxide, etc.

The pharmaceutical composition containing the active ingredient can be in a form suitable for oral administration, for example, tablets, lozenges, pastilles, aqueous or oil suspensions, dispersible powders or granules, emulsions, hard or soft capsules, or syrups or elixirs. Oral compositions can be prepared according to any method known in the art for preparing pharmaceutical compositions, and such compositions can contain one or more ingredients selected from a sweetening agent, a flavoring agent, a coloring agent and a preservative to provide eye-pleasing and palatable pharmaceutical formulations. Tablets contain the active ingredient and non-toxic pharmaceutically acceptable excipients suitable for mixing to prepare the tablets. These excipients can be inert excipients (such as calcium carbonate, sodium carbonate, lactose, calcium phosphate or sodium phosphate), granulating and disintegrating agents (such as microcrystalline cellulose, croscarmellose sodium, corn starch or alginic acid), binding agents (such as starch, gelatin, polyvinylpyrrolidone or acacia) and lubricating agents (such as magnesium stearate, stearic acid or talc). These tablets may

be uncoated, or they may be coated by known techniques to mask the taste of the drug or delay disintegration and absorption in the gastrointestinal tract and thereby to provide a sustained action over a relatively long period of time. For example, water-soluble taste-masking substances (such as hydroxypropyl methylcellulose or hydroxypropyl cellulose) or time-delaying substances (such as ethyl cellulose or cellulose acetate butyrate) may be used.

Formulations for oral administration can also be made available in the form of hard gelatin capsules in which the active ingredient is mixed with an inert solid diluent, for example calcium carbonate, calcium phosphate or kaolin, or in the form of soft gelatin capsules in which the active ingredient is mixed with water-soluble carrier, such as polyethylene glycol or an oil medium, for example peanut oil, liquid paraffin or olive oil.

Aqueous suspensions contain active materials and excipients suitable for mixing to prepare the aqueous suspensions. Such excipients are suspending agents, such as sodium carboxymethylcellulose, methylcellulose, hydroxypropylmethylcellulose, sodium alginate, polyvinylpyrrolidone and acacia; or dispersing or wetting agents which may be: a naturally generated phosphatide (for example lecithin), condensation products of alkylene oxide with fatty acids (for example polyoxyethylene stearate), condensation products of ethylene oxide with long-chain fatty alcohol (for example heptadecaethyleneoxy cetanol), condensation products of ethylene oxide with partial esters derived from fatty acids and hexitol (for example polyoxyethylene sorbitol monooleate) or condensation products of ethylene oxide with partial esters derived from fatty acids and hexitol anhydride (for example polyoxyethylene sorbitan monooleate). The aqueous suspensions may also contain one or more preservatives, for example ethylparaben or *n*-propyl paraben, one or more coloring agents, one or more flavoring agents and one or more sweetening agents, such as sucrose, saccharin or aspartame.

Oil suspensions may be formulated by suspending the active ingredient in a vegetable oil such as peanut oil, olive oil, sesame oil or coconut oil, or in a mineral oil such as liquid paraffin. The oil suspensions may contain a thickening agent, for example beeswax, hard paraffin or cetanol. Sweetening agents and flavoring agents described above may be added to provide palatable formulations. These compositions can be preserved by the addition of antioxidants such as butylated hydroxyanisole or $\alpha$-tocopherol.

Dispersible powders and granules suitable for preparing an aqueous suspension, by the addition of water, may provide the active ingredient and a dispersing or wetting agent, a suspending agent or one or more preservatives for mixing. Suitable dispersing or wetting agents and suspending agents are described as above. Other excipients, such as a sweetening agent, a flavoring agent and a coloring agent, may also be added. These compositions are preserved by the addition of an antioxidant such as ascorbic acid.

The pharmaceutical compositions of the present invention may also be in the form of oil-in-water emulsions. The oil phase may be a vegetable oil, for example olive oil or peanut oil, or a mineral oil, for example liquid paraffin or a mixture thereof. Suitable emulsions may be naturally-occurring phosphatides (such as soya bean lecithin), esters or partial esters derived from fatty acids and hexitol anhydride (such as sorbitan monooleate) and condensation products of the partial esters with ethylene oxide (such as polyoxyethylene sorbitol monooleate). The emulsions may also contain a sweetening agent, a flavoring agent, a preservative and an antioxidant. Syrups and elixirs formulated by sweetening agents (such as glycerol, propylene glycol, sorbitol or sucrose) may be used. Such formulations may also contain a mitigant, a preservative, a coloring agent and an antioxidant.

The pharmaceutical composition of the present invention may be in the form of a sterile injectable aqueous solution. Acceptable vehicles and solvents that may be employed are water, Ringer's solution and isotonic sodium chloride solution. The sterile injection may be a sterile injectable oil-in-water microemulsion in which the active ingredient is dissolved in the oil phase. For example, the active ingredient is dissolved in a mixture of soybean oil and lecithin. The oil solution is then treated to form a microemulsion by adding it to a mixture of water and glycerol. The injection or microemulsion may be injected into the bloodstream of a patient by local bolus injection. Alternatively, it may be desirable to administer the solution and microemulsion in a way that maintain a constant circulating concentration of the compounds of the present invention. To maintain such a constant concentration, a device for continuous intravenous delivery may be used.

The pharmaceutical composition of the present invention may be in the form of a sterile injectable aqueous or oil suspension for intramuscular and subcutaneous administration. The suspension may be formulated according to the known art using those suitable dispersing or wetting agents and suspending agents described above. The sterile injectable formulation may also be a sterile injectable solution or suspension in a non-toxic parenterally-acceptable diluent or solvent, for example a solution prepared in 1,3-butanediol. In addition, sterile fixed oil is conventionally employed as a solvent or suspending medium. For this purpose, any blend fixed oil including synthetic monoglycerides or diglycerides may be used. In addition, fatty acids such as oleic acid may also be used in the preparation of injections.

The compounds of the present invention may be administered in the form of suppositories for rectal administration. These pharmaceutical compositions can be prepared by mixing the drug with a suitable non-irritating excipient which is solid at ordinary temperatures but liquid in the rectum and therefore will melt in the rectum to release the drug. Such materials include cocoa butter, glycerogelatin, hydrogenated vegetable oil, and mixtures of polyethylene glycols of various molecular weights and fatty acid esters of polyethylene glycol.

It is well known to those skilled in the art that the dosage of a drug administered depends on a variety of factors, including, but not limited to, the activity of the particular compound used, the patient's age, weight, health, gender and diet, the time of administration, the mode of administration, the rate of excretion, the combination of drugs, and the like. In addition, the optimal treatment regimen, such as mode of treatment, daily amount of the compound of the formula or type of pharmaceutically acceptable salt, can be verified according to conventional treatment protocols.

In the present invention, the compound of formula (I) of a compound or a pharmaceutically acceptable salt thereof, a hydrate thereof or a solvate thereof can be, as an active ingredient, mixed with a pharmaceutically acceptable carrier or excipient to prepare a composition and to be prepared into a clinically acceptable dosage form. The derivatives of the present invention may be used in combination with other active ingredients as long as they do not produce other adverse effects, such as allergic reactions. The compounds of the present invention may be used as the sole active ingredient or in combination with other drugs for the treatment of diseases associated with tyrosine kinase activity. Combination therapy is achieved by administering the individual therapeutic components simultaneously, separately or sequentially.

## DETAILED DESCRIPTION

Unless otherwise stated, the terms used in the specification and claims have the following meanings.

The term "alkyl" refers to linear or branched saturated aliphatic alkyl groups containing 1 to 20 carbon atoms, preferably 1 to 12 carbon atoms, and more preferably 1 to 6 carbon atoms. Non-limiting examples include methyl, ethyl, *n*-propyl, isopropyl, *n*-butyl, isobutyl, tert-butyl, sec-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl and various branched isomers thereof, and the like. Preferably, the alkyl is a lower alkyl containing 1-6 carbon atoms, and non-limiting examples include methyl, ethyl, *n*-propyl, isopropyl, *n*-butyl, isobutyl, *tert*-butyl, sec-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, the substituent may be substituted at any available connection point, and the substituent is preferably one or more groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "alkenyl" refers to an alkyl as defined above consisting of at least two carbon atoms and at least one carbon-carbon double bond, e.g., vinyl, 1-propenyl, 2-propenyl, 1-, 2- or 3-butenyl. The alkenyl may be substituted or unsubstituted. When substituted, the substituent is preferably one or more groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkoxy, heterocycloalkoxy, cycloalkylthio and heterocycloalkylthio.

The term "alkynyl" refers to an alkyl as defined above consisting of at least two carbon atoms and at least one carbon-carbon triple bond, e.g., ethynyl, propynyl or butynyl. The alkynyl may be substituted or unsubstituted. When substituted, the substituent is preferably one or more groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a monocyclic or polycyclic hydrocarbon substituent that is saturated or partially unsaturated, wherein the cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; and polycyclic cycloalkyl includes spirocycloalkyl, fused cycloalkyl and bridged cycloalkyl.

The term "spirocycloalkyl" refers to a 5-20 membered polycyclic group in which a carbon atom (called spiro-atom) is shared among monocyclic rings, wherein those rings may contain one or more double bonds, but none of them has a fully conjugated $\pi$-electron system. The spirocycloalkyl is preferably a 6-14 membered spirocycloalkyl, and more preferably a 7-10 membered spirocycloalkyl. According to the number of the spiro-atoms shared among the rings, the spirocycloalkyl may be monospirocycloalkyl, bispirocycloalkyl or polyspirocycloalkyl. Preferably, the spirocycloalkyl is monospirocycloalkyl and bispirocycloalkyl. More preferably, the spirocycloalkyl is a 4-membered/4-membered, 4-membered/5-membered, 4-membered/6-membered, 5-membered/5-membered or 5-membered/6-membered monospirocycloalkyl. Non-limiting examples of the spirocycloalkyl include:

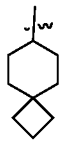 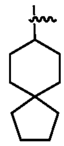 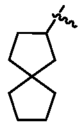 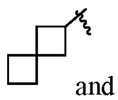 and 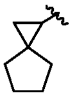.

The term "fused cycloalkyl" refers to a 5-20 membered all-carbon polycyclic group in which each ring in the system shares a pair of adjacent carbon atoms with another ring, wherein one or more of the rings may contain one or more double bonds, but none of them has a fully conjugated $\pi$-electron system. The fused cycloalkyl is preferably a 6-14 membered fused cycloalkyl, and more preferably a 7-10 membered fused cycloalkyl. According to the number of comprised rings, the fused cycloalkyl may be bicyclic, tricyclic, tetracyclic or polycyclic fused cycloalkyl. The fused cycloalkyl is preferably bicyclic or tricyclic fused cycloalkyl, and more preferably a 5-membered/5-membered or 5-membered/6-membered bicyclic fused cycloalkyl. Non-limiting examples of the fused cycloalkyl include:

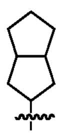 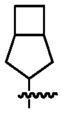 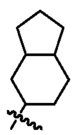 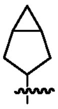 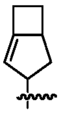 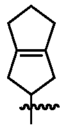 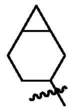 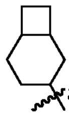 and 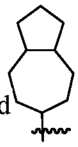.

The term "bridged cycloalkyl" refers to a 5-20 membered all-carbon polycyclic group in which any two rings share two carbon atoms that are not directly connected to each other, wherein these rings may contain one or more double bonds, but none of them has a fully conjugated $\pi$-electron system. The bridged cycloalkyl is preferably a 6-14 membered bridged cycloalkyl, and more preferably a 7-10 membered bridged cycloalkyl. According to the number of comprised rings, the bridged cycloalkyl may be bicyclic, tricyclic, tetracyclic or polycyclic bridged cycloalkyl. The bridged cycloalkyl is preferably bicyclic, tricyclic or tetracyclic bridged cycloalkyl, and more preferably bicyclic or tricyclic bridged cycloalkyl. Non-limiting examples of the bridged cycloalkyl include:

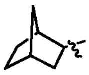 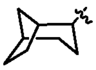 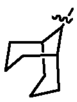 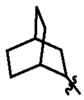 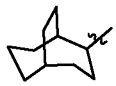

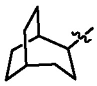 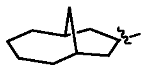 and 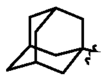.

The cycloalkyl ring can be fused to an aryl, heteroaryl or heterocycloalkyl ring, wherein the ring attached to the parent structure is cycloalkyl. Non-limiting examples include indanyl, tetrahydronaphthyl, benzocycloheptyl, etc. The cycloalkyl may be optionally substituted or unsubstituted. When substituted, the substituent is preferably one or more groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "heterocyclyl" refers to a monocyclic or polycyclic hydrocarbon substituent that is saturated or partially unsaturated and contains 3 to 20 ring atoms, wherein one or more of the ring atoms are heteroatoms selected from N, O and $S(O)_m$ (wherein m is an integer from 0 to 2), excluding ring portions of -O-O-, -O-S- or -S-S-, and the remaining ring atoms are carbon atoms. Preferably, the heterocyclyl contains 3 to 12 ring atoms, of which 1 to 4 are heteroatoms; more preferably, the heterocyclyl contains 3 to 8 ring atoms, of which 1 to 3 are heteroatoms; and most preferably, the heterocyclyl contains 5 to 7 ring atoms, of which 1 to 2 or 1 to 3 are heteroatoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, and the like, preferably 1,2,5-oxadiazolyl, pyranyl or morpholinyl. Polycyclic heterocyclyl includes spiroheterocyclyl, fused heterocyclyl, and bridged heterocyclyl.

The term "spiroheterocyclyl" refers to a 5-20 membered polycyclic heterocyclyl group in which an atom (called spiro-atom) is shared among monocyclic rings, wherein one or more ring atoms are heteroatoms selected from N, O or $S(O)_m$ (wherein m is an integer from 0 to 2), and the remaining ring atoms are carbon atoms. These rings may contain one or more double bonds, but none of them has a fully conjugated $\pi$-electron system. The spiroheterocyclyl is preferably a 6-14 membered spiroheterocyclyl, and more preferably a 7-10 membered spiroheterocyclyl. According to the number

of spiro-atoms shared among the rings, the spiroheterocyclyl may be monospiroheterocyclyl, bispiroheterocyclyl or polyspiroheterocyclyl. Preferably, the spiroheterocyclyl is monospiroheterocyclyl and bispiroheterocyclyl. More preferably, the spiroheterocyclyl is 4-membered/4-membered, 4-membered/5-membered, 4-membered/6-membered, 5-membered/5-membered or 5-membered/6-membered monospiroheterocyclyl. Non-limiting examples of spiroheterocyclyl include:

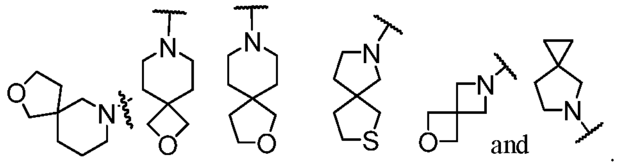
and .

The term "fused heterocyclyl" refers to a 5-20 membered polycyclic heterocyclyl group in which each ring in the system shares a pair of adjacent atoms with another ring, wherein one or more of the rings may contain one or more double bonds, but none of them has a fully conjugated $\pi$-electron system, wherein one or more of the ring atoms are heteroatoms selected from N, O and $S(O)_m$ (wherein m is an integer from 0 to 2), and the remaining ring atoms are carbon atoms. The fused heterocyclyl is preferably a 6-14 membered fused heterocyclyl, and more preferably a 7-10 membered fused heterocyclyl. According to the number of comprised rings, the fused heterocyclyl may be bicyclic, tricyclic, tetracyclic or polycyclic fused heterocyclyl. The fused heterocyclyl is preferably bicyclic or tricyclic fused heterocyclyl, and more preferably a 5-membered/5-membered or 5-membered/6-membered bicyclic fused heterocyclyl. Non-limiting examples of fused heterocyclyl include:

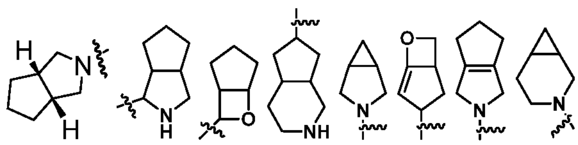

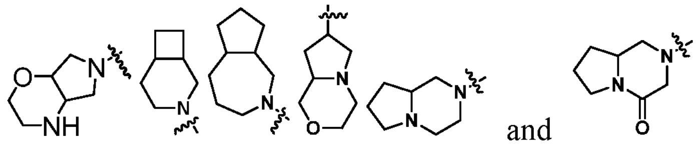
and .

The term "bridged heterocyclyl" refers to a 5-14 membered polycyclic heterocyclyl in which any two rings share two carbon atoms that are not directly attached to each other, wherein these rings may contain one or more double bonds, but none of them has a fully conjugated $\pi$-electron system, wherein one or more of the ring atoms are heteroatoms selected from N, O and $S(O)_m$ (wherein m is an integer from 0 to 2), and the remaining ring atoms are carbon atoms. The bridged heterocyclyl is preferably a 6-14 membered bridged heterocyclyl, and more preferably a 7-10 membered bridged heterocyclyl. According to the number of comprised rings, the bridged heterocyclyl may be bicyclic, tricyclic, tetracyclic or polycyclic bridged heterocyclyl. The bridged heterocyclyl is preferably bicyclic, tricyclic or tetracyclic bridged heterocyclyl, and more preferably bicyclic or tricyclic bridged heterocyclyl. Non-limiting examples of bridged heterocyclyl include:

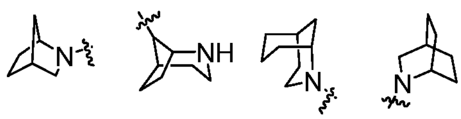

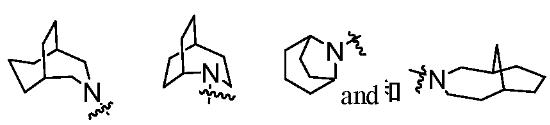
and .

The heterocyclyl ring may be fused to an aryl, heteroaryl or cycloalkyl ring, wherein the ring attached to the parent structure is heterocyclyl. Non-limiting examples of heterocyclyl include:

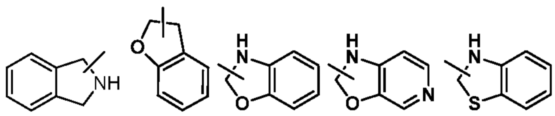,

etc.

The heterocyclyl may be optionally substituted or unsubstituted. When substituted, the substituent is preferably one or more groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "aryl" refers to a 6-14 membered all-carbon monocyclic or fused-polycyclic group (i.e., rings that share a pair of adjacent carbon atoms) having a conjugated $\pi$-electron system. The aryl is preferably a 6-10 membered aryl (such as phenyl and naphthyl), and more preferably phenyl. The aryl ring may be fused to a heteroaryl, heterocyclyl or cycloalkyl ring, wherein the ring attached to the parent structure is an aryl ring. Non-limiting examples of aryl include:

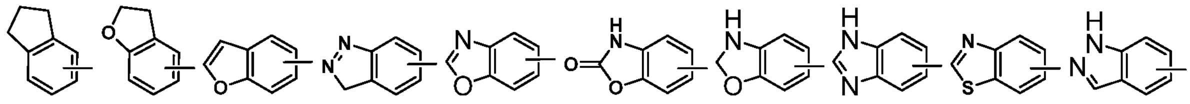

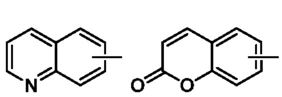 and 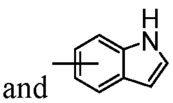.

The aryl may be substituted or unsubstituted. When substituted, the substituent is preferably one or more groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group.

The term "heteroaryl" refers to a heteroaromatic system containing 1 to 4 heteroatoms and 5 to 14 ring atoms, wherein the heteroatoms are selected from O, S and N. Preferably, the heteroaryl is a 5-10 membered heteroaryl containing 1 to 3 heteroatoms. More preferably, the heteroaryl is a 5-membered or 6-membered heteroaryl containing 1 to 2 heteroatoms. The heteroaryl is preferably, for example, imidazolyl, furanyl, thienyl, thiazolyl, pyrazolyl, oxazolyl, pyrrolyl, tetrazolyl, pyridinyl, pyrimidinyl, thiadiazolyl, pyrazinyl or the like, more preferably imidazolyl, thiazolyl, pyrazolyl, pyrimidinyl or thiazolyl, and more preferably pyrazolyl or thiazolyl. The heteroaryl ring may be fused to an aryl, heterocyclyl or cycloalkyl ring, wherein the ring attached to the parent structure is a heteroaryl ring. Non-limiting examples of heteroaryl include:

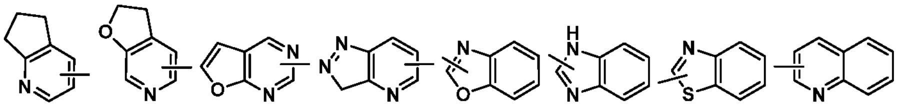 and 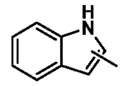.

The heteroaryl may be optionally substituted or unsubstituted. When substituted, the substituent is preferably one or more groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group.

The term "alkoxy" refers to -O-(alkyl) and -O-(unsubstituted cycloalkyl), wherein the alkyl is defined as above. Non-limiting examples of alkoxy include methoxy, ethoxy, propoxy, butoxy, cyclopropoxy, cyclobutoxy, cyclopentyloxy and cyclohexyloxy. The alkoxy may be optionally substituted or unsubstituted. When substituted, the substituent is preferably one or more groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group.

The term "haloalkyl" refers to an alkyl substituted with one or more halogens, wherein the alkyl is defined as above.

The term "haloalkoxy" refers to an alkoxy substituted with one or more halogens, wherein the alkoxy is defined as above.

The term "hydroxyl" refers to -OH group.

The term "halogen" refers to fluorine, chlorine, bromine or iodine.

The term "amino" refers to -NH2.

The term "cyano" refers to -CN.

The term "nitro" refers to $-NO_2$.

The term "oxo" refers to =O.

The term "carboxyl" refers to -C(O)OH.

The term "sulfydryl" refers to -SH.

The term "ester group" refers to -C(O)O(alkyl) or -C(O)O(cycloalkyl), wherein the alkyl and cycloalkyl are defined as above.

The term "acyl" refers to a compound containing the -C(O)R group, wherein R is alkyl, cycloalkyl, heterocyclyl, aryl or heteroaryl.

The term "optional" or "optionally" means that the event or circumstance subsequently described may, but not necessarily, occur, and that the description includes instances where the event or circumstance occurs or does not occur. For example, "heterocyclyl group optionally substituted with alkyl" means that alkyl may be, but not necessarily, present, and that the description includes instances where the heterocyclyl group is or is not substituted with alkyl.

The term "substituted" means that one or more hydrogen atoms, preferably 5 hydrogen atoms at most and more preferably 1-3 hydrogen atoms, in a group are each independently substituted with corresponding number of substituents. It goes without saying that a substituent is only in its possible chemical position, and those skilled in the art will be able to determine (by experiments or theories) possible or impossible substitution without undue efforts. For example, it may be unstable when an amino or hydroxyl having a free hydrogen is bound to a carbon atom having an unsaturated (such as olefinic) bond.

The term "pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities.

The term "pharmaceutically acceptable salt" refers to salts of the compounds disclosed herein which are safe and effective for use in a mammal *in vivo* and possess the required biological activity.

Synthesis Method of Compounds Disclosed Herein

To accomplish the objects of the present invention, the following synthetic schemes are employed to prepare the compound of formula (I) of the present invention.

When X is CH and Y is N, the compound of formula (IA) as the compound of formula (I) is prepared according to Scheme 1 below.

Step 1: ethyl acetoacetate and *N,N*-dimethylformamide dimethyl acetal are directly subjected to condensation reaction to give ethyl (Z)-2-((dimethylamino)methylene)-3-oxobutyrate (IA-1);

Step 2: ethyl(Z)-2-((dimethylamino)methylene)-3-oxobutyrate (IA-1) reacts with strong base to give an intermediate enol sodium salt, which is then subjected to cyclization reaction with diethyl oxalate to give diethyl 4-oxo-4*H*-pyran-2,5-dicarboxylate (IA-2), wherein the solvent is preferably anhydrous tetrahydrofuran, and the strong base is preferably sodium hydride;

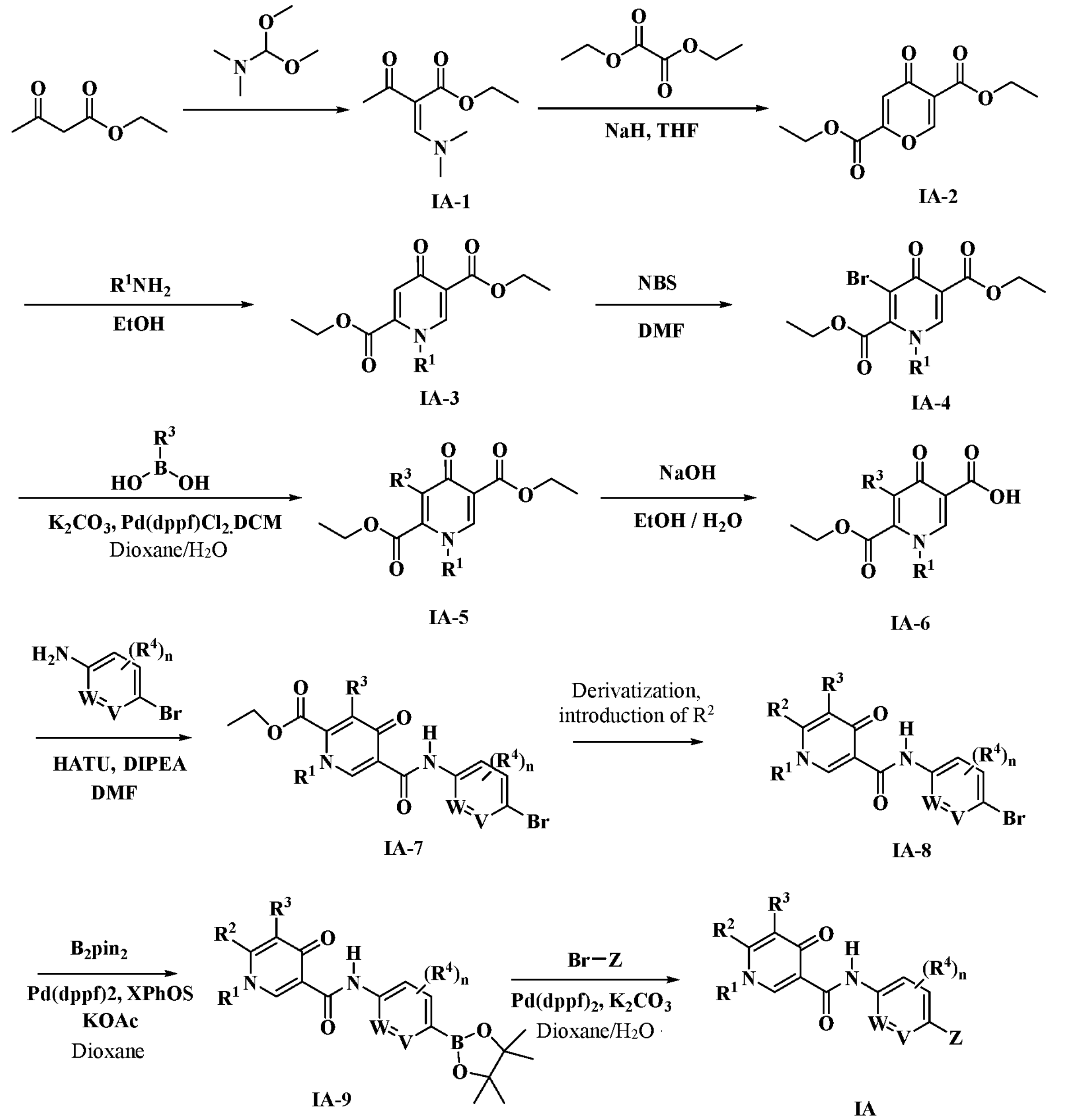


Scheme 1

Step 3: diethyl 4-oxo-4*H*-pyran-2,5-dicarboxylate (IA-2) and amine ($R^1NH_2$) are subjected to addition-condensation reaction to give diethyl *N*-$R^1$-4-oxo-1,4-dihydropyridine-2,5-dicarboxylate (IA-3);

Step 4: diethyl *N*-$R^1$-4-oxo-1,4-dihydropyridine-2,5-dicarboxylate (IA-3) is brominated by *N*-bromosuccinimide (NBS) to give diethyl *N*-$R^1$-3-bromo-4-oxo-1,4- dihydropyridine-2,5-dicarboxylate (IA-4);

Step 5: diethyl *N*-$R^1$-3-bromo-4-oxo-1,4-dihydropyridine-2,5-dicarboxylate (IA-4) and corresponding boric acid are subjected to Suzuki coupling reaction to give diethyl *N*-$R^1$-3-$R^3$-4-oxo-1,4-dihydropyridine-2,5-dicarboxylate (IA-5), wherein preferably, in the Suzuki reaction, potassium carbonate is used as a base, $Pd(dppf)Cl_2$ is used as a catalyst, dioxane/water is used as a mixed solvent, and the reaction temperature is 80 °C;

Step 6: diethyl *N*-$R^1$-3-$R^3$-4-oxo-1,4-dihydropyridine-2,5-dicarboxylate (IA-5) is subjected to selective hydrolysis in the presence of a base to give *N*-$R^1$-6-(ethoxycarbonyl)-5-$R^3$-4-oxo-1,4-dihydropyridine-3-carboxylic acid (IA-6), wherein the base is preferably sodium hydroxide;

Step 7: *N*-$R^1$-6-(ethoxycarbonyl)-5-$R^3$-4-oxo-1,4-dihydropyridine-3-carboxylic acid (IA-6) and bromo aromatic amine are subjected to amidation reaction in the presence of a coupling agent and a base to give *N*-$R^1$-6-(ethoxycarbonyl)-5-$R^3$-4-oxo-1,4-dihydropyridine-3-arylamide (IA-7), wherein the coupling agent is preferably HATU, and the base is preferably *N,N*-diisopropylethylamine (DIPEA);

Step 8: ethyl ester group of the intermediate *N*-$R^1$-6-(ethoxycarbonyl)-5-$R^3$-4-oxo-1,4-dihydropyridine-3-arylamide

(IA-7) is subjected to a three-step reaction (hydrolysis, amidation and dehydration), and then cyano ($R^2$) is introduced to give *N*-$R^1$-6-cyano-5-$R^3$-4-oxo-1,4-dihydropyridine-3-arylamide (IA-8);

Step 9: *N*-$R^1$-6-R-5-$R^3$-4-oxo-1,4-dihydropyridine-3-arylamide (IA-8) and bisboric acid ester are subjected to Suzuki reaction with the catalysis of palladium (preferably Pd(dppf)$_2$, XPhOS as a ligand and potassium acetate as a base) to form a borate intermediate (IA-9);

Step 10: the borate intermediate IA-9 and aromatic bromide are subjected to Suzuki coupling reaction to give a compound of formula (IA), wherein preferably, in the Suzuki reaction, the catalyst is Pd(dppf)$_2$, the base is $K_2CO_3$, and the solvent is dioxane and water.

Alternatively, the compound of formula (IA) is prepared according to Scheme 2 below.

Step 1: *N*-$R^1$-6-(ethoxycarbonyl)-5-$R^3$-4-oxo-1,4-dihydropyridine-3-carboxylic acid (IA-6) and aromatic amine are subjected to amidation reaction in the presence of a catalyst and a base to give *N*-$R^1$-6-(ethoxycarbonyl)-5-$R^3$-4-oxo-1,4-dihydropyridine-3-arylamide (IA-10), wherein the catalyst is preferably HATU, and the base is preferably *N,N*-diisopropylethylamine (DIPEA);

Step 2: ethyl ester of *N*-$R^1$-6-(ethoxycarbonyl)-5-$R^3$-4-oxo-1,4-dihydropyridine-3-arylamide (IA-10) is hydrolyzed in a solution in the presence of a base to give *N*-$R^1$-6-carboxyl-5-$R^3$-4-oxo-1,4-dihydropyridine-3-arylamide (IA-11), wherein the base is preferably LiOH, and the solution is preferably a methanol-water solution;

Step 3: *N*-$R^1$-6-carboxyl-5-$R^3$-4-oxo-1,4-dihydropyridine-3-arylamide (IA-11) and ammonium chloride are subjected to amidation reaction in the presence of a coupling agent and a base to give *N*-$R^1$-6-(aminocarbonyl)-5-$R^3$-4-oxo-1,4-dihydropyridine-3-arylamide (IA-12), wherein the coupling agent is preferably PyBrOP, and the base is preferably DIPEA;

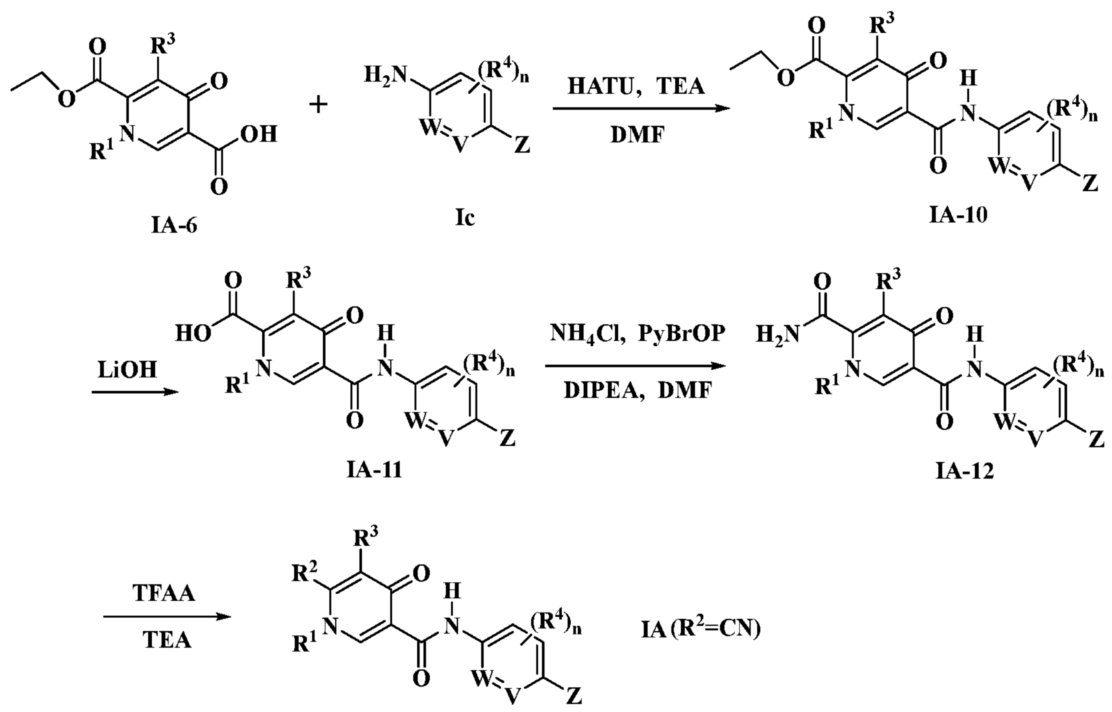


Scheme 2

Step 4: *N*-$R^1$-6-(aminocarbonyl)-5-$R^3$-4-oxo-1,4-dihydropyridine-3-arylamide (IA-12) is dehydrated in the presence of a dehydrating agent and a base to give a compound of formula (IA) ($R^2$ is cyano), wherein the dehydrating agent is preferably trifluoroacetic anhydride, and the base is triethylamine.

When X is N and Y is CH, the compound of formula (IB) as the compound of formula (I) is prepared according to Scheme 3 below.

Step 1: a starting material, namely ethyl amide malonate, and 2-carbonyl butyraldehyde methyl acetal are subjected to condensation-cyclization reaction in a basic medium to give 1-$R^3$-6-methyl-2-carbonyl-1,2-dihydropyridine-3-carboxylic acid (IB-1), wherein the basic medium is preferably EtONa/EtOH;

Step 2: 1-$R^3$-6-methyl-2-carbonyl-1,2-dihydropyridine-3-carboxylic acid (IB-1) is protected by iodoethane in the presence of a solvent and a base to give ethyl 1-$R^3$-6-methyl-2-carbonyl-1,2-dihydropyridine-3-carboxylate (IB-2), wherein the solvent is preferably NMP, and the base is preferably $Na_2CO_3$;

Step 3: *n*-bromosuccinimide (NBS) is simultaneously subjected to electrophilic substitution and free radical substitution initiated by perbenzoic acid (BPO) to form bisbrominated ethyl 1-$R^3$-6-bromomethyl-5-bromo-2-carbonyl-1,2-dihydropyridine-3- carboxylate (IB-3);

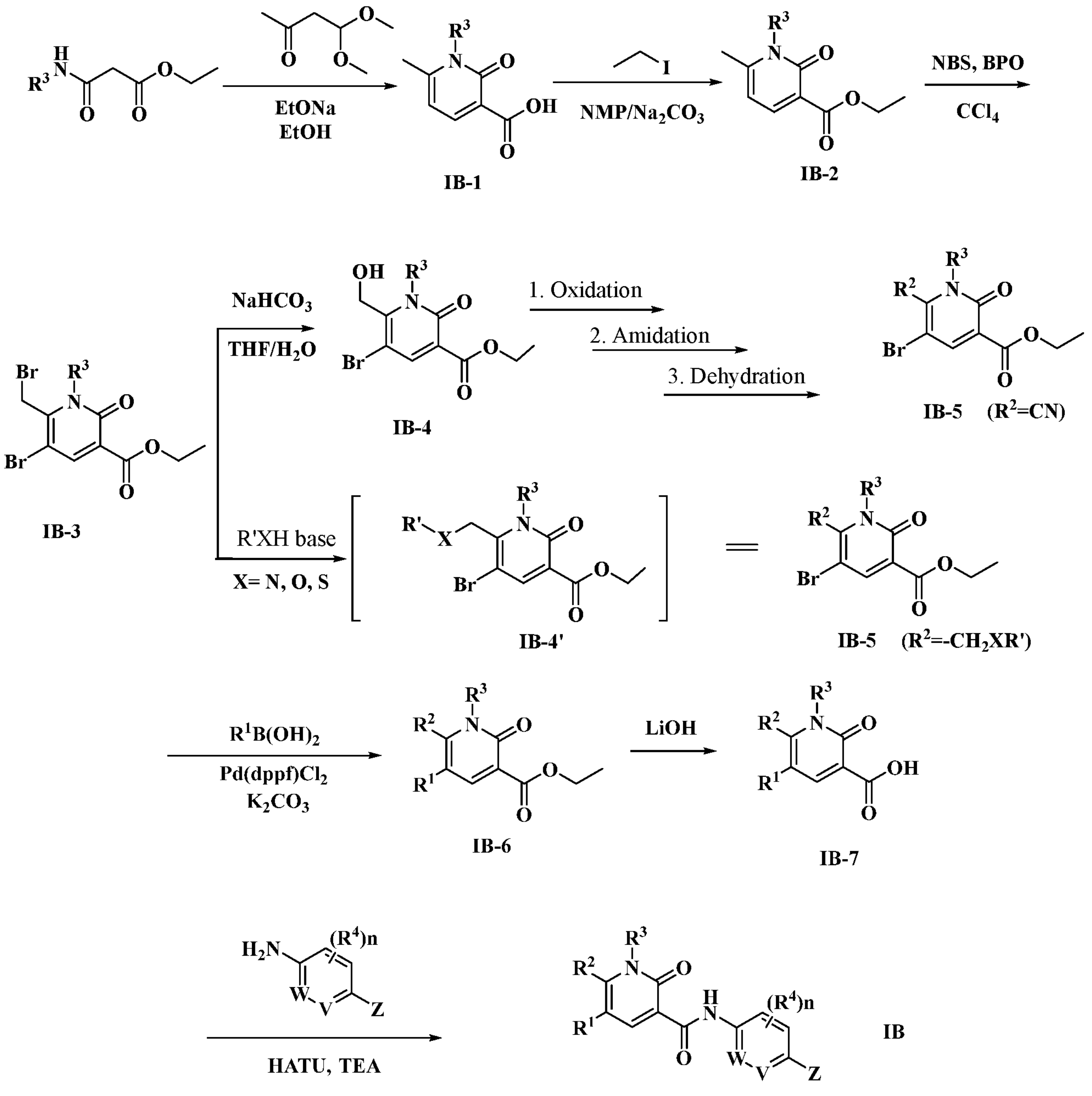


Scheme 3

Step 4: ethyl 1-$R^3$-6-bromomethyl-5-bromo-2-carbonyl-1,2-dihydropyridine-3-carboxylate (IB-3) is subjected to selective hydrolysis in the presence of a weak base to give ethyl 1-$R^3$-6-hydroxymethyl-5-bromo-2-carbonyl-1,2-dihydropyridine-3-carboxylate (IB-4), wherein the base is preferably $NaHCO_3$;

Step 5: the resulting hydroxymethyl intermediate (IB-4) is subjected to a three-step derivatization, namely oxidation reaction, amidation reaction and dehydration reaction, and then cyano (namely $R^2$ is cyano) to give ethyl 1-$R^3$-6-$R^2$-5-bromo-2-carbonyl-1,2- dihydropyridine-3-carboxylate (IB-5); or,

Step 4': various amines, alcohols and thiols (the presence of LiOH is needed for the alcohols and thiols) and ethyl

1-$R^3$-6-bromomethyl-5-bromo-2-carbonyl-1,2-dihydropyridine-3-carboxylate (IB-3) are subjected to nucleophilic substitution reaction, and then $R^2$ is introduced to give ethyl 1-$R^3$-6-$R^2$-5-bromo-2-carbonyl-1,2-dihydropyridine-3-carboxylate (IB-5) ($R^2$=$CH_2XR'$);

Step 6: ethyl 1-$R^3$-6-$R^2$-5-bromo-2-carbonyl-1,2-dihydropyridine-3-carboxylate (IB-5) and a boric acid compound are subjected to Suzuki coupling reaction to give ethyl 1-$R^3$-5-$R^1$-6-$R^2$-2-carbonyl-1,2-dihydropyridine-3-carboxylate (IB-6), wherein preferably, in the Suzuki coupling reaction, the catalyst is Pd(dppf)$Cl_2$, the base is $K_2CO_3$, the mixed solvent is dioxane/water, and the reaction temperature is 80 °C;

Step 7: ethyl 1-$R^3$-5-$R^1$-6-$R^2$-2-carbonyl-1,2-dihydropyridine-3-carboxylate (IB-6) is subjected to selective hydrolysis in a solvent in the presence of a base to give ethyl 1-$R^3$-5-$R^1$-6-$R^2$-2-carbonyl-1,2-dihydropyridine-3-carboxylate (IB-7), wherein the base is preferably LiOH, and the solvent is preferably methanol/water;

Step 8: ethyl 1-$R^3$-5-$R^1$-6-$R^2$-2-carbonyl-1,2-dihydropyridine-3-carboxylate (IB-7) and aromatic amine are subjected to amidation reaction to give the compound of formula (IB), wherein preferably, the amidation reagent is HATU, the base is TEA, and the reaction solvent is DMF

When X and Y are both N, and $R^2$ is oxo, the compound of formula (IC) as the compound of formula (I) is prepared according to Scheme 4 below.

Step 1: a starting material, namely diethyl 2-(aminomethylene)propanoate (IC-1) and isocyanate are subjected to condensation reaction to give a corresponding urea intermediate (IC-2);

Step 2: the urea intermediate (IC-2) is subjected to self-cyclization reaction in a basic medium to give ethyl 3-$R^3$-2,4-dioxo-1,2,3,4-tetrahydropyrimidine-5-carboxylate (IC-3), wherein the basic medium is preferably EtONa/EtOH;

Step 3: in the presence of a solvent and a base, $R^1$ is introduced into ethyl 3-$R^3$-2,4-dioxo-1,2,3,4-tetrahydropyrimidine-5-carboxylate (IC-3) by alkyl iodide ($R^1$-I) to give ethyl 1-$R^1$-3-$R^3$-2,4-dioxo-1,2,3,4-tetrahydropyrimidine-5-carboxylate (IC-4), wherein the solvent is preferably NMP, and the base is preferably $K_2CO_3$;

Step 4: ethyl 1-$R^1$-3-$R^3$-2,4-dioxo-1,2,3,4-tetrahydropyrimidine-5-carboxylate (IC-4) is subjected to acid hydrolysis in the presence of an acid to give 1-$R^1$-3-$R^3$-2,4-dioxo-1,2,3,4-tetrahydropyrimidine-5-carboxylic acid (IC-5), wherein the acid is preferably HCl;

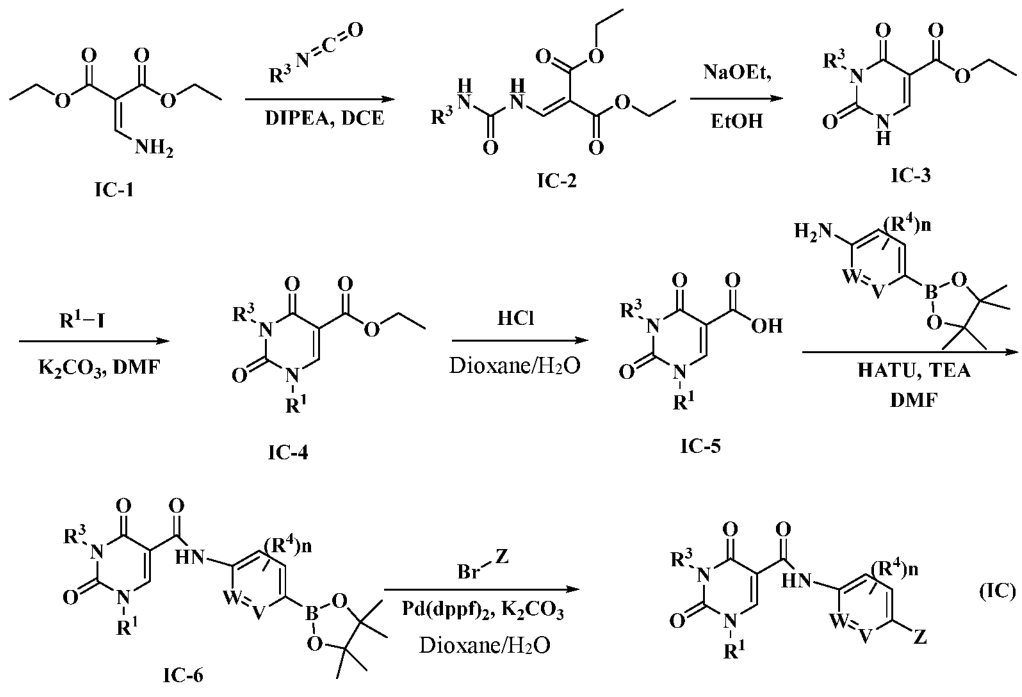

IC-1
IC-2
IC-3
IC-4
IC-5
IC-6
(IC)

Scheme 4

Step 5: 1-$R^1$-3-$R^3$-2,4-dioxo-1,2,3,4-tetrahydropyrimidine-5-carboxylic acid (IC-5) and an aromatic amine compound containing boronic acid are reacted with each other in the presence of an amide coupling agent and a base to form a corresponding amide intermediate containing the boronic acid substituent (IC-6), wherein the coupling agent is preferably HATU, and the base is preferably TEA;

Step 6: the amide intermediate containing the boronic acid substituent (IC-6) and a bromopyridine compound are

subjected to Suzuki coupling reaction to give the compound of formula (IC), wherein preferably, in the Suzuki reaction, the catalyst is $Pd(dppf)Cl_2$, the base is $K_2CO_3$, the mixed solvent is dioxane/water, and the reaction temperature is 80 °C.

In these compounds, $R^1$, $R^2$, $R^3$, $R^4$, W, V, Z, and n are each defined as in the formula (I).

**BRIEF DESCRIPTON OF THE DRAWINGS**

FIG. 1 shows the growth change in tumor volume of mice in the example compound group and the solvent control group in EBC-1 non-small cell lung cancer models.

FIG. 2 shows the body weight change of mice as a function of treatment time in the example compound group and the solvent control group in EBC-1 non-small cell lung cancer models.

**EXAMPLES**

The compounds of the present invention and preparation thereof are further understood by examples which illustrate some methods for preparing or using the compounds. However, it is to be understood that these examples do not limit the present invention. Variations of the present invention, either known already or further developed, are considered to fall within the scope of the present invention as described and claimed herein.

The compounds of the present invention are prepared using convenient starting materials and general preparative procedures. Typical or preferential reaction conditions such as reaction temperature, time, solvent, pressure, and molar ratio of reactants are provided in the present invention. However, unless otherwise specified, other reaction conditions can be adopted. The preferential conditions may vary with the particular reactants or solvents used. However, in general, preferential steps and conditions for reaction can be determined.

In addition, some protecting groups may be used in the present invention to protect certain functional groups from unwanted reactions. Protecting groups suitable for various functional groups and their protection or deprotection conditions are well known to those skilled in the art. For example, Protective Groups in Organic Synthesis (T. W. Greene and G. M. Wuts, 3rd edition, Wiley, New York, 1999 and references therein) describes in detail the protection or deprotection of a number of protective groups.

The isolation and purification of the compounds and intermediates may be carried out by any suitable method or procedure depending on the particular requirements, such as filtration, extraction, distillation, crystallization, column chromatography, preparative thin-layer plate chromatography, preparative high-performance liquid chromatography or a mixture thereof. The examples described herein may be referred to for specific method for isolation and purification. Of course, other similar means for separation and purification may be employed. They can be characterized using conventional methods, including physical constants and spectral data.

The structures of the compounds are determined by nuclear magnetic resonance (NMR) or/and mass spectrometry (MS). NMR shifts are given in $10^{-6}$ (ppm). NMR is determined using a Brukerdps 400 NMR equipment. The solvents for determination are deuterated-dimethyl sulfoxide (DMSO-$d_6$), deuterated-chloroform ($CDCl_3$) and deuterated-methanol ($CD_3OD$), and the internal standard is tetramethylsilane (TMS).

MS is determined using an ACQUITY H-Class UPLC mass spectrometer (QDa Detector) (manufacturer: Waters).

The liquid phase is prepared using Waters 2545 high performance liquid chromatography (Waters 2489 UV/Visual Detector 2767 sample MGR, single C18, 5 μm, 20 mm×250 mm) (manufacturer: Waters).

An initiator and an EU type microwave reactor (manufacturer: Biotage) are used in the microwave reaction.

In terms of the thin-layer chromatography silica gel plate, GF254 silica gel plate from Qingdao Ocean Chemical Co., Ltd. is employed. The specification of the silica gel plate used by thin-layer chromatography (TLC) is 0.15 mm-0.2 mm, and the specification of the silica gel plate used in the separation and purification of products by thin-layer chromatography is 0.4 mm-0.5 mm.

Generally, in the column chromatography, 100-200 mesh and 200-300 mesh silica gels from Qingdao Ocean Chemical Co., Ltd. are used as carriers.

Known starting materials of the present invention may be synthesized using or according to methods known in the art, or may be purchased from the companies such as WH Mall, Beijing Ouhe, Sigma, J&K Scientific, Yishiming (Beijing) Biomedical Technology, Shanghai Shuya, Shanghai Innochem, Energy Chemical and Shanghai Bidepharm.

Unless otherwise specified in the example, the reaction can be carried out under argon atmosphere or nitrogen atmosphere.

The argon atmosphere or nitrogen atmosphere means that the reaction flask is connected to an argon balloon or a nitrogen balloon with a volume of about 1 L.

The reaction solvent, organic solvent or inert solvent is each considered to be a solvent that, when used, does not participate in the reaction under the reaction conditions described and includes benzene, toluene, acetonitrile, tetrahydrofuran (THF), dimethylformamide (DMF), chloroform, dichloromethane, diethyl ether, methanol, nitrogen-methyl pyrroline (NMP), pyridine, and the like. Unless otherwise specified in the example, the solution refers to an aqueous solution.

The chemical reactions described herein are generally carried out under normal pressure. The reaction temperature is between -78 °C and 200 °C. The reaction time and conditions are, for example, between -78 °C and 200 °C at one atmosphere pressure, completion in about 1 to 24 hours. If the reaction is carried out overnight, the reaction time is generally 16 hours. Unless otherwise specified in the example, the reaction temperature is room temperature (20 °C to 30 °C).

The progress of reaction in the examples is monitored by thin layer chromatography (TLC), and the developing agent system used in the reaction includes: A: dichloromethane and methanol system, B: *n*-hexane and ethyl acetate system, C: petroleum ether and ethyl acetate system, and D: acetone, wherein the volume ratio of solvents is adjusted according to the polarity of the compound.

The eluent system for column chromatography and the developing agent system for thin-layer chromatography used for purifying the compounds include: A: dichloromethane and methanol system, B: *n*-hexane and ethyl acetate system, C: petroleum ether and ethyl acetate system, wherein the volume ratio of the solvents is adjusted according to the polarity of the compound, and a small amount of basic or acidic reagents such as triethylamine and acetic acid may also be added for adjustment.

Unless otherwise defined, all the professional and scientific terms used herein have the same meanings as those familiar to those skilled in the art. In addition, any method and material similar or equivalent to the described contents can be applied in the method of the present invention.

Abbreviations

μL = microliter
μM = micromole
NMR = nuclear magnetic resonance
Boc = *tert*-butoxycarbonyl
br = broad peak
d = doublet
δ = chemical shift
°C= Celsius degree
dd = double doublet
DIPEA = diisopropylethylamine
DMF = *N,N*-dimethylformamide
DMSO = dimethyl sulfoxide
DCM = dichloromethane
EA = ethyl acetate
HATU = 2-(7-azabenzotriazol-1-yl)-*N,N,N',N'*-tetramethyluronium hexafluorophosphate
HPLC = high performance liquid chromatography
Hz = Hertz
$IC_{50}$ = concentration required for inhibiting activity by 50%
J = coupling constant (Hz)
LC-MS = liquid chromatography-mass spectrometry
m = multiplet
$M+H^+$ = parent compound mass + one proton
mg = milligram
mL = milliliter
mmol = millimole
MS = mass spectrometry
*m*/*z* = mass-to-charge ratio
nM = nanomole
NBS = *N*-bromosuccinimide

PE = petroleum ether
ppm = parts per million
PyBrOP = bromo-tris-pyrrolidino-phosphonium hexafluorophosphate
s = singlet
t = triplet
TEA= triethylamine
TBDPS= tert-butyl diphenyl silicon
TFA = trifluoroacetic acid
THF = tetrahydrofuran

Preparation Example 1: preparation of 6-(ethoxycarbonyl)-5-(4-fluorophenyl)-1-isopropyl-4-oxo-1,4-dihydropyridine-3-carboxylic acid (intermediate **a)**

Step 1: preparation of ethyl (Z)-2-((dimethylamino)methylene)-3-oxobutanoate (**a1**)

*N,N*-dimethylformamide dimethyl acetal (1.72 kg, 14.4 mol) was added dropwise to a reaction flask (with an internal temperature of less than 10 °C) containing ethyl acetoacetate (1.79 kg, 13.75 mol) at 0 °C. The reaction mixture was warmed to room temperature and stirred overnight. After the reaction was completed, the reaction mixture was concentrated under reduced pressure to give ethyl (Z)-2-((dimethylamino)methylene)-3-oxobutanoate (2.54 kg, red oil),which was directly used in the next step.
LC-MS (ESI): *m/z* 186.2[M+H$^+$].
$^1$H NMR (400 MHz, Chloroform-*d*) $\delta$ 7.66 (s, 1H), 4.22 (q, *J*= 7.1 Hz, 2H), 3.24 - 2.84 (m, 6H), 2.31 (s, 3H), 1.31 (t, *J* = 7.1 Hz, 3H).

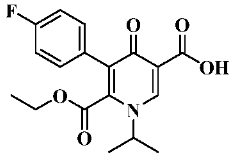

**a**

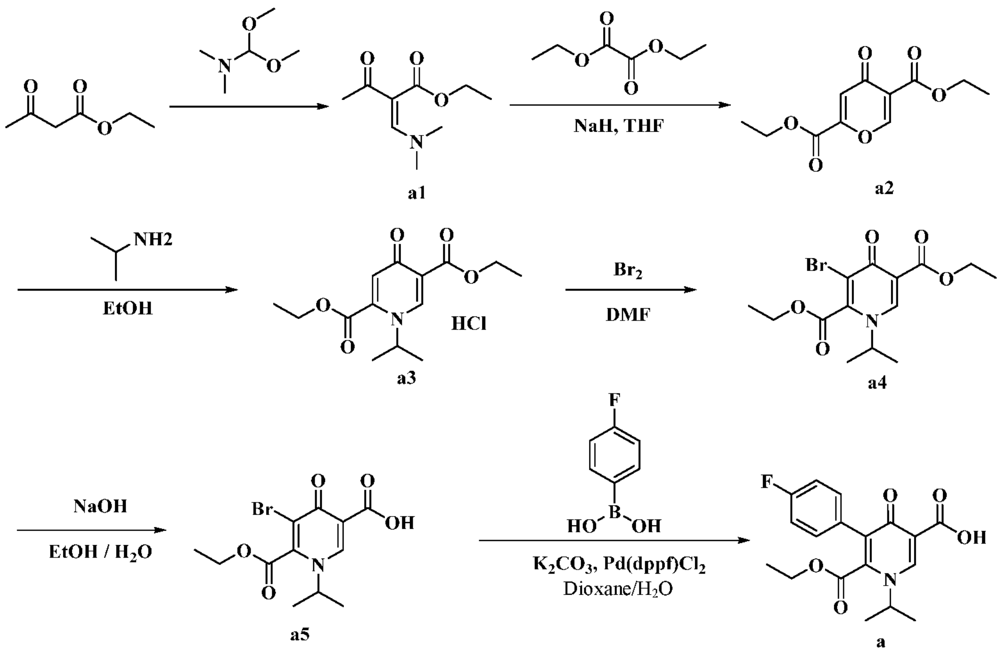


Step 2: preparation of diethyl 4-oxo-4*H*-pyran-2,5-dicarboxylate **(a2)**

Ethyl (Z)-2-((dimethylamino)methylene)-3-oxobutanoate (150 g, 0.81 mol) and diethyl oxalate (130 g, 0.89 mol) were added to a reaction flask containing anhydrous tetrahydrofuran (700 mL). Sodium hydride (38.8 g, 0.97 mol, 60%) was then slowly added to the reaction mixture at 80 °C, and then the resulting reaction mixture was stirred for 30 min

after the addition was completed. After the reaction was completed, the reaction mixture was cooled to room temperature, added with 1 N diluted hydrochloric acid (1800 mL) at 0 °C to quench the reaction, and then extracted with ethyl acetate (600 mL × 3). The organic phases were combined, washed with saturated brine, dried over anhydrous sodium sulfate and filtered, and the filtrate was concentrated under reduced pressure. The crude product was slurried with ethanol and petroleum ether (1:10) to give diethyl 4-oxo-4*H*-pyran-2,5-dicarboxylate (140 g, yellow solid, yield: 72%).

LC-MS (ESI): *m*/*z* 241.1[M+H$^+$].

$^1$H NMR (400 MHz, Chloroform-*d*) $\delta$8.53 (s, 1H), 7.18 (s, 1H), 4.45 - 4.34 (m, 4H), 1.39 (dd, *J* = 12.8, 7.1 Hz, 6H).

Step 3: preparation of diethyl 1-isopropyl-4-oxo-1,4-dihydropyridine-2,5-dicarboxylate **(a3)**

Diethyl 4-oxo-4*H*-pyran-2,5-dicarboxylate (300 g, 1.25 mol) was added to a reaction flask containing ethanol (1875 mL), and then the reaction mixture was cooled to 0 °C and added dropwise with isopropylamine (73.8 g, 1.25 mol) while stirring. After the addition was completed, the reaction mixture was warmed to room temperature, stirred for 30 min, and then heated to reflux overnight. After the reaction was completed, the reaction mixture was concentrated under reduced pressure. The residue was dissolved in ethyl acetate (200 mL), cooled to 0 °C and then added dropwise with concentrated hydrochloric acid (104 mL). The resulting reaction mixture was stirred for about 30 min and filtered, and the filter cake was washed with ethyl acetate (600 mL). A solid was collected to give diethyl 1-isopropyl-4-oxo-1,4-dihydropyridine-2,5-dicarboxylate hydrochloride (307.99 g, white solid, yield: 70%).

LC-MS (ESI): *m*/*z* 282.12[M+H$^+$].

$^1$H NMR (400 MHz, DMSO-$d_6$) $\delta$ 8.46 (s, 1H), 6.77 (s, 1H), 4.73 - 4.62 (m, 1H), 4.37 (d, *J* = 7.1 Hz, 2H), 4.24 (q, *J*= 7.1 Hz, 2H), 1.44 (d, *J*= 6.6 Hz, 7H), 1.35 - 1.21 (m, 6H).

Step 4: preparation of diethyl 3-bromo-1-isopropyl-4-oxo-1,4-dihydropyridine-2,5-dicarboxylate **(a4)**

Diethyl 1-isopropyl-4-oxo-1,4-dihydropyridine-2,5-dicarboxylate hydrochloride (307 g, 1.09 mol) was added to a reaction flask containing DMF (1500 mL). The reaction mixture was cooled to 0 °C and then added dropwise with liquid bromine (167.5 mL, 3.27 mol) slowly. After the addition was completed, the reaction mixture was stirred for 30 min. After the reaction was completed, the reaction mixture was added dropwise to water containing sodium hydrogen sulfite (7500 mL). The resulting mixture was stirred for 1 h and filtered, and the filter cake was washed with water (2 L). The filter cake was collected and dried to give diethyl 3-bromo-1-isopropyl-4-oxo-1,4-dihydropyridine-2,5-dicarboxylate (236.69 g, white solid, yield: 67.8%).

LC-MS (ESI): *m*/*z* 360.07/362.07[M+H$^+$].

$^1$H NMR (400 MHz, Chloroform-*d*) $\delta$ 8.25 (s, 1H), 4.50 (q, *J* = 7.2 Hz, 2H), 4.37 (q, *J* = 7.1 Hz, 2H), 4.13 (p, *J*= 6.6 Hz, 1H), 1.52 (d, *J*= 6.6 Hz, 6H), 1.41 (dt, *J*= 22.2, 7.1 Hz, 6H).

Step 5: preparation of 5-bromo-6-(ethoxycarbonyl)-1-isopropyl-4-oxo-1,4-dihydropyridine-3-carboxylic acid **(a5)**

Diethyl 3-bromo-1-isopropyl-4-oxo-1,4-dihydropyridine-2,5-dicarboxylate (323.11 g, 0.9 mol) was added to a reaction flask containing ethanol (1600 mL). The reaction mixture was cooled to 0 ° C, added dropwise with aqueous sodium hydroxide (160 mL of water, 1.06 mol sodium hydroxide), and then stirred for 1 h after the addition was completed. After the reaction was completed, the reaction mixture was added with 1.5 N diluted hydrochloric acid (700 mL) to adjust the pH to 7. After a white solid was precipitated out, the reaction mixture was stirred for 30 min. The reaction mixture was filtered, and the solid was collection and dried to give 5-bromo-6-(ethoxycarbonyl)-1-isopropyl-4-oxo-1,4-dihydropyridine-3-carboxylic acid (271.2 g, white solid, yield: 90.8%).

LC-MS (ESI): *m*/*z* 331.95/333.91[M+H$^+$].

$^1$H NMR (400 MHz, Chloroform-*d*) $\delta$ 14.71 (s, 1H), 8.59 (s, 1H), 4.56 (q, *J*= 7.1 Hz, 2H), 4.28 (hept, *J*= 6.7 Hz, 1H), 1.58 (d, *J*= 6.6 Hz, 6H), 1.47 (t, *J*= 7.2 Hz, 3H).

Step 6: preparation of 6-(ethoxycarbonyl)-5-(4-fluorophenyl)-1-isopropyl-4-oxo-1,4-dihydropyridine-3-carboxylic acid **(a)**

5-bromo-6-(ethoxycarbonyl)-1-isopropyl-4-oxo-1,4-dihydropyridine-3-carboxylic acid (43.5 g, 0.13 mol), *p*-fluorophenylboronic acid (25.18 g, 0.18 mol), potassium carbonate (71.8 g, 0.52 mol) and Pd(dppf)$Cl_2$(475 mg, 0.065 mmol) were added to a reaction flask containing 1,4-dioxane (400 mL) and water (100 mL) at room temperature. The reaction mixture was sealed in the flask, purged with nitrogen three times, and stirred at 85 °C overnight. After being cooled to room temperature, the reaction mixture was diluted with water (600 mL) and ethyl acetate (200 mL), filtered, and left to stand for liquid separation. The aqueous phase was adjusted to pH 6 with concentrated hydrochloric acid and then filtered after a white precipitate was formed. The filter cake was washed with water and dried to give 6-(ethoxycarbonyl)-5-(4-fluorophenyl)-1-isopropyl-4-oxo-1,4-dihydropyridine-3-carboxylic acid **(a)** (31 g, white solid, yield: 68%).

LC-MS (ESI): *m/z* 348.10[M+H+].

$^1$H NMR (400 MHz, DMSO-$d_6$) $\delta$ 15.73 (s, 1H), 8.81 (s, 1H), 7.37 - 7.22 (m, 4H), 4.39 (p, *J* = 6.5 Hz, 1H), 4.15 (q, *J*= 7.1 Hz, 2H), 1.54 (d, *J*= 6.5 Hz, 6H), 0.93 (t, *J* = 7.1 Hz, 3H).

Preparation Example 2: preparation of 6-(ethoxycarbonyl)-5-(4-fluorophenyl)-1-methyl-4-oxo-1,4-dihydropyridine-3-carboxylic acid (intermediate **b)**

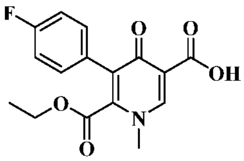

b

The same procedures as in Preparation Example 1 were performed, except that aqueous methylamine solution was used in place of isopropylamine, to give 6-(ethoxycarbonyl)-5-(4-fluorophenyl)-l-methyl-4-oxo-1,4-dihydropyridine-3-carboxylic acid (grey solid, four-step yield: 38.5%).

LC-MS (ESI): *m/z* 320.1[M+H+].

Preparation Example 3: preparation of 1-cyclobutyl-6-(ethoxycarbonyl)-5-(4-fluorophenyl)-4-oxo-1,4-dihydropyridine-3-carboxylic acid (intermediate **c)**

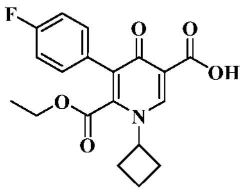

c

The same procedures as in Preparation Example 1 were performed, except that cyclobutylamine was used in place of isopropylamine, to give 1-cyclobutyl-6-(ethoxy-carbonyl)-5-(4-fluorophenyl)-4-oxo-1,4-dihydropyridine-3-carboxylic acid (white solid, four-step yield: 80.9%).

LC-MS (ESI): *m/z* 360.1[M+H+].

Preparation Example 4: preparation of 1-cyclopropyl-6-(ethoxycarbonyl)-5-(4-fluorophenyl)-4-oxo-1,4-dihydropyridine-3-carboxylic acid (intermediate d)

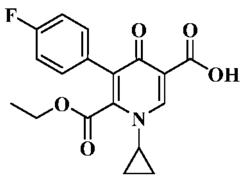

d

The same procedures as in Preparation Example 1 were performed, except that cyclopropylamine was used in place of isopropylamine, to give 1-cyclopropyl-6-(ethoxycarbonyl)-5-(4-fluorophenyl)-4-oxo-1,4-dihydropyridine-3-carboxylic acid (grey solid, four-step yield: 70.8%).

LC-MS (ESI): *m/z* 346.3 [M+H+].

Preparation Example 5: preparation of 6-cyano-1-cyclopropyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide (intermediate **e)**

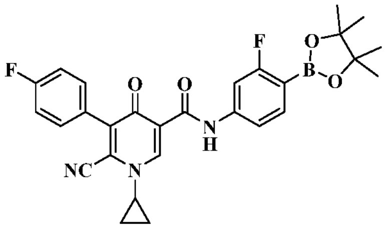

e

Step 1: preparation of ethyl 5-((4-bromo-3-fluorophenyl) carbamoyl)-1-cyclopropyl-3-(4-fluorophenyl)-4-oxo-1,4-dihydropyridine-2-carboxylate (**e1**)

1-cyclopropyl-6-(ethoxycarbonyl)-5-(4-fluorophenyl)-4-oxo-1,4-dihydropyridine-3-carboxylic acid **(d)** (8.0 g, 23.2 mmol), 4-bromo-3-fluoroaniline (4.0 g, 21.1 mmol), HATU (12 g, 31.6 mmol) and DIPEA (5.4 g, 42.1 mmol) were added to a reaction flask containing DMF (100 mL).

The reaction mixture was stirred at room temperature for 30 min. After the reaction was completed, the reaction mixture was added with saturated sodium bicarbonate solution to quench the reaction, and then extracted with ethyl acetate (100 mL × 3). The organic phases were combined, washed with saturated brine, dried over anhydrous sodium sulfate and filtered, and the filtrate was concentrated under reduced pressure. The residue was purified by silica gel column chromatography (eluent: PE:EA= 5:1) to give ethyl 5-((4-bromo-3-fluorophenyl)carbamoyl)-1-cyclopropyl-3-(4-fluorophenyl)-4-oxo-1,4-dihydropyridine-2-carboxylate (**e1**) (8.5 g, yellow solid, yield: 78%).

LC-MS (ESI): *m/z* 517.05/519.06 [M+H$^+$].

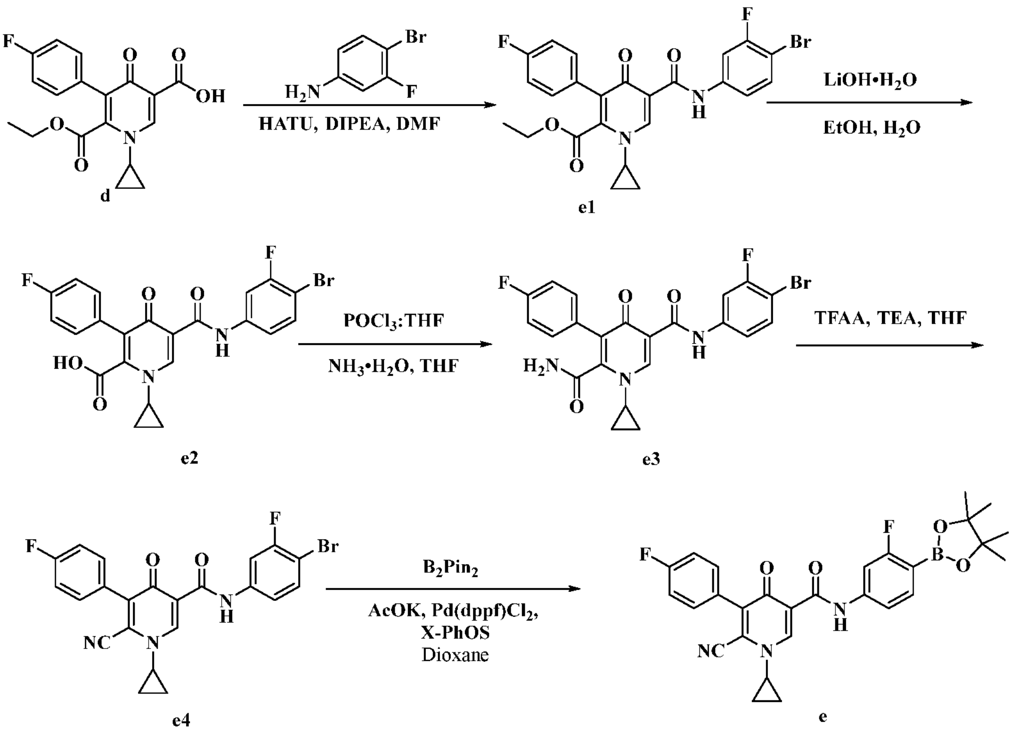


d e1 e2 e3 e4 e

Step 2: preparation of 5-((4-bromo-3-fluorophenyl)carbamoyl)-1-cyclopropyl-3-(4-fluorophenyl)-4-oxo-1,4-dihydropyridine-2-carboxylic acid **(e2)**

Ethyl 5-((4-bromo-3-fluorophenyl)carbamoyl)-1-cyclopropyl-3-(4-fluorophenyl)-4-oxo-1,4-dihydropyridine-2-carboxylate (**e1**) (8.5 g, 16.4 mmol), lithium hydroxide monohydrate (1.03 g, 24.6 mmol) and water (25 mL) were added to a reaction flask containing ethanol (100 mL) at room temperature. The reaction mixture was warmed to 70 °C and stirred overnight. After the reaction was completed, the reaction mixture was concentrated under reduced pressure to give crude 5-((4-bromo-3-fluorophenyl)carbamoyl)-1-cyclopropyl-3-(4-fluorophenyl)-4-oxo-1,4-dihydropyridine-2-carboxylic acid **(e2)** (yellow solid), which was directly used in the next step without purification.

Step 3: preparation of $N^5$-(4-bromo-3-fluorophenyl)-1-cyclopropyl-3-(4-fluorophenyl)-4-oxo-1,4-dihydropyridine-2,5-dicarboxamide **(e3)**

5-((4-bromo-3-fluorophenyl)carbamoyl)-1-cyclopropyl-3-(4-fluorophenyl)-4-oxo-1,4-dihydropyridine-2-carboxylic acid **(e2)** (2.2 g, 4.5 mmol) was added to a reaction flask containing tetrahydrofuran (15 mL) and phosphorus oxychloride (15 mL) at room temperature. The reaction mixture was heated to reflux and stirred for 30 min. After the reaction was completed, the reaction mixture was concentrated under reduced pressure to give acyl chloride intermediate, which was directly used in the next step without purification.

The acyl chloride intermediate was added to a reaction flask containing tetrahydrofuran (20 mL) and aqueous ammonia (20 mL) at 0 °C. After the addition was completed, the reaction mixture was stirred for 20 min. After the reaction was completed, ethyl acetate (30 mL) was added to dilute the reaction mixture. The organic phase was washed successively with water and saturated brine, dried over anhydrous sodium sulfate, and filtered, and the filtrate was concentrated under reduced pressure. The residue was purified by silica gel column chromatography (eluent: MeOH:DCM = 1:20) to give $N^5$-(4-bromo-3-fluorophenyl)-1-cyclopropyl-3-(4-fluorophenyl)-4-oxo-1,4-dihydropyridine-2,5-dicarboxamide **(e3)** (1.5 g, yellow solid, yield: 68%).

Step 4: preparation of *N*-(4-bromo-3-fluorophenyl)-6-cyano-1-cyclopropyl-5-(4-fluorophenyl)-4-oxo-1,4-dihydropyridine-3-carboxamide **(e4)**

$N^5$-(4-bromo-3-fluorophenyl)-1-cyclopropyl-3-(4-fluorophenyl)-4-oxo-1,4-dihydropyridine-2,5-dicarboxamide **(e3)** (1.5 g, 3.1 mmol) was added to a reaction flask containing anhydrous tetrahydrofuran (20 mL). The reaction mixture was cooled to 0 °C and then added successively with triethylamine (2.5 g, 24.6 mmol) and trifluoromethanesulfonic anhydride (2.6 g, 12.2 mmol). After the addition was completed, the reaction mixture was stirred for 30 min. After the reaction was completed, ethyl acetate (100 mL) was added to dilute the reaction mixture. The organic phase was washed successively with water and saturated brine, dried over anhydrous sodium sulfate, and filtered, and the filtrate was concentrated under reduced pressure. The residue was purified by silica gel column chromatography (eluent: MeOH:DCM = 1:20) to give *N*-(4-bromo-3-fluorophenyl)-6-cyano-1-cyclopropyl-5-(4-fluorophenyl)-4-oxo-1,4-dihydropyridine-3-carboxamide **(e4)** (1.3 g, yellow solid, yield: 90.3%).

Step 5: preparation of 6-cyano-1-cyclopropyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide **(e)**

*N*-(4-bromo-3-fluorophenyl)-6-cyano-1-cyclopropyl-5-(4-fluorophenyl)-4-oxo-1,4-dihydropyridine-3-carboxamide **(e4)** (1.3 g, 2.7 mmol), bis(pinacolato)diboron (1.04 g, 4.1 mmol), potassium acetate (795 mg, 8.1 mmol), $Pd_2(dba)_3$ (275 mg, 0.3 mmol) and X-PHOS (257 mg, 0.54 mmol) were added to a reaction flask containing 1,4-dioxane (16 mL) at room temperature. The reaction mixture was sealed in the flask, purged with nitrogen three times, and subjected to microwave reaction for 30 min (90 °C). After being cooled to room temperature, the reaction mixture was diluted with water (100 mL) and extracted with ethyl acetate (100 mL × 3). The organic phases were combined, washed with saturated brine, dried over anhydrous sodium sulfate and filtered, and the filtrate was concentrated under reduced pressure. The residue was purified by silica gel column chromatography (eluent: MeOH:DCM = 1:20) to give 6-cyano-1-cyclopropyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide **(e)** (800 mg, grey solid, yield: 55.9%).

LC-MS (ESI): *m/z* 518.14 [M+H$^+$].

Preparation Example 6: preparation of 6-cyano-*N*-(3-fluoro-4-(4,4,5,5-tetramethyl-1,3,2-dioxa-borolan-2-yl)phenyl)-5-(4-fluorophenyl)-1-isopropyl-4-oxo-1,4-dihydropyridine-3-carboxamide (intermediate **f)**

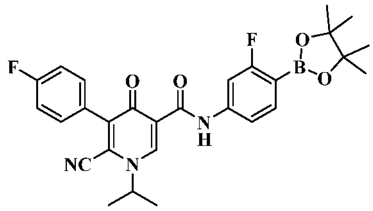

f

The same procedures as in Preparation Example 5 were performed, except that 6-(ethoxycarbonyl)-5-(4-fluorophenyl)-1-isopropyl-4-oxo-1,4-dihydropyridine-3-carboxylic acid **(a)** was used in place of 1-cyclopropyl-6-(ethoxycarbonyl)-5-(4-fluorophenyl)-4-oxo-1,4-dihydropyridine-3-carboxylic acid **(d),** so as to give 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (grey solid, five-step yield: 25.3%).

LC-MS (ESI): *m*/*z* 520.21[M+H$^+$].

Preparation Example 7: preparation of 6-cyano-1-cyclobutyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide (intermediate **g)**

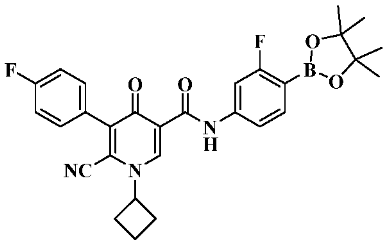

g

The same procedures as in Preparation Example 5 were performed, except that 1-cyclobutyl-6-(ethoxycarbonyl)-5-(4-fluorophenyl)-4-oxo-1,4-dihydropyridine-3-carboxylic acid **(c)** was used in place of 1-cyclopropyl-6-(ethoxycarbonyl)-5-(4-fluorophenyl)-4-oxo-1,4-dihydropyridine-3-carboxylic acid **(d),** so as to give 6-cyano-1-cyclobutyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide (grey solid, five-step yield: 29.6%).

LC-MS (ESI): *m*/*z* 532.20[M+H$^+$].

Preparation Example 8: preparation of 5-bromo-6-(ethoxycarbonyl)-1-(4-fluorophenyl)-2-oxo-1,2-dihydropyridine-3-carboxylic acid (intermediate **h)**

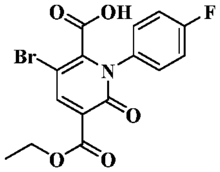

h

Step 1: preparation of ethyl 3-((4-fluorophenyl)amino)-3-oxopropanoate **(hi)**

4-fluoroaniline (30 g, 0.27 mol) and triethylamine (28.7 g, 0.28 mol) were added to a reaction flask containing acetone (300 mL). The reaction mixture was cooled to 0 °C, and then ethyl 3-chloro-3-oxopropanoate (42.9 g, 0.28 mol)

was added dropwise while stirring. After the addition was completed, the reaction mixture was warmed to room temperature and then stirred overnight. After the reaction was completed, the reaction solution was concentrated under reduced pressure. The residue was added with water (500 mL), stirred for 2 h, and then filtered under reduced pressure. The solid was collected to give ethyl 3-((4-fluorophenyl)amino)-3-oxopropanoate **(hi)** (60 g, yellow solid, yield: 98.8%).

LC-MS (ESI): *m/z* 226.07[M+H$^+$].

Step 2: preparation of 1-(4-fluorophenyl)-6-methyl-2-oxo-1,2-dihydropyridine-3-carboxylic acid **(h2)**

Ethyl 3-(4-fluorophenyl)amino)-3-oxopropanoate **(hi)** (30 g, 0.13 mol), 4,4-dimethoxy-butan-2-one (21.1 g, 0.16 mol) and a solution of 20% sodium ethoxide in ethanol (166 mL, 0.43 mol) were added to a reaction flask containing ethanol (200 mL). The reaction mixture was heated to reflux overnight at 80 °C, and then concentrated under reduced pressure after the reaction was completed. The residue was added with 5 M hydrochloric acid to adjust the pH to 4-5, and then dichloromethane (100 mL × 3) was added for extraction. The organic phases were combined, washed with saturated brine, dried over anhydrous sodium sulfate, filtered and concentrated under reduced pressure. The residue was slurried with a mixed solution of petroleum ether and ethyl acetate (5:1, 100 mL),and then filtered. The filter cake was washed with petroleum ether, and the solid was collected to give the product 1-(4-fluorophenyl)-6-methyl-2-oxo-1,2-dihydropyridine-3-carboxylic acid (**h2**) (24 g, yellow solid, yield: 74.7%).

LC-MS (ESI): *m/z* 248.12[M+H$^+$].

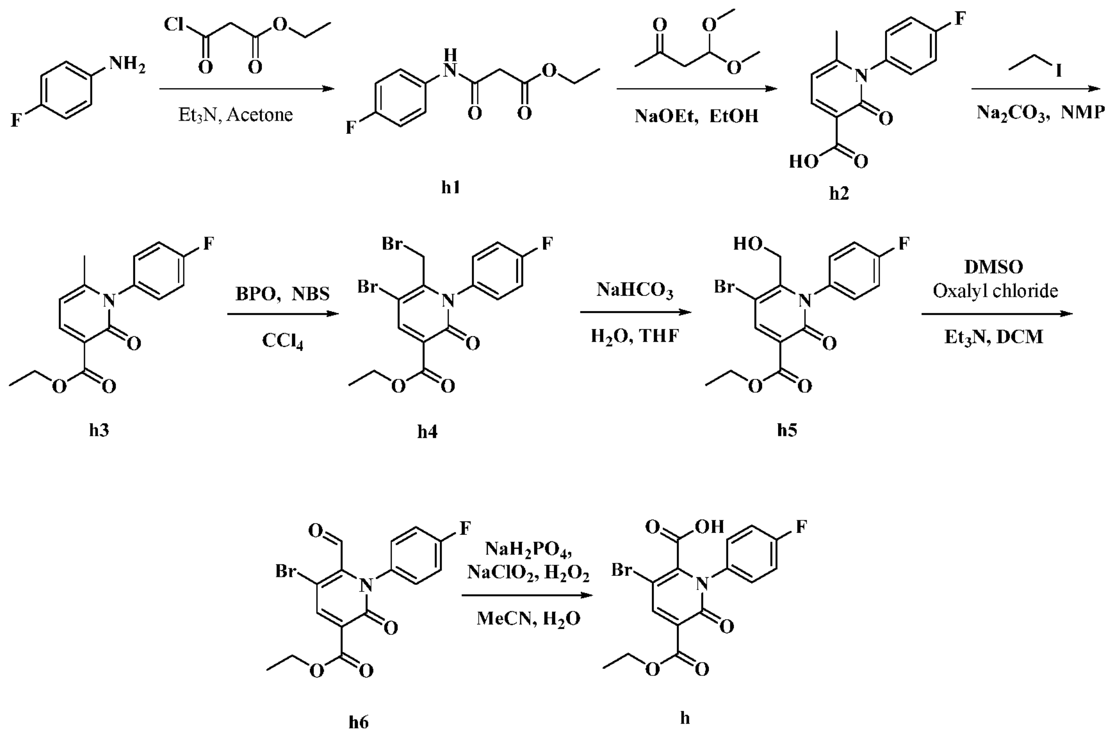


Step 3: preparation of ethyl 1-(4-fluorophenyl)-6-methyl-2-oxo-1,2-dihydropyridine-3-carboxylate (**h3**)

1-(4-fluorophenyl)-6-methyl-2-oxo-1,2-dihydropyridine-3-carboxylic acid (**h2**) (16 g, 64.8 mmol), sodium carbonate (7.6 g, 71.3 mmol) and iodoethane (11.1 g, 71.3 mmol) were added to a reaction flask containing NMP (150 mL), and the reaction mixture was stirred at room temperature for 4 h. After the reaction was completed, the reaction mixture was added with water (300 mL) and then extracted with ethyl acetate (150 mL × 3). The combined organic phases were washed with saturated brine, dried over anhydrous sodium sulfate and filtered, and the filtrate was concentrated under reduced pressure. The residue was purified by slurrying with diethyl ether (150 mL) to give ethyl 1-(4-fluorophenyl)-6-methyl-2-oxo-1,2-dihydropyridine-3-carboxylate (**h3**) (15 g, yellow solid, yield: 84.2%).

LC-MS (ESI): *m/z* 276.10[M+H$^+$],

Step 4: preparation of ethyl 5-bromo-6-(bromomethyl)-1-(4-fluorophenyl)-2-oxo-1,2-dihydropyridine-3-carboxylate (**h4**)

Ethyl 1-(4-fluorophenyl)-6-methyl-2-oxo-1,2-dihydropyridine-3-carboxylate (**h3**) (15 g, 54.5 mmol), NBS (21.4

g, 120 mmol) and BPO (660 mg, 2.72 mmol) were added to a reaction flask containing carbon tetrachloride (150 mL). The reaction mixture was heated to 70 °C and stirred for 24 h. After the reaction was completed, the reaction mixture was added with saturated aqueous sodium thiosulfate solution (200 mL) to quench the reaction, and extracted with dichloromethane (100 mL × 3). The combined organic phases were washed with saturated brine, dried over anhydrous sodium sulfate and filtered, and the filtrate was concentrated under reduced pressure. The residue was purified by silica gel column chromatography (eluent: PE:EA = 2:1) to give ethyl 5-bromo-6-(bromomethyl)-1-(4-fluorophenyl)-2-oxo-1,2-dihydropyridine-3-carboxylate (**h4**) (16.8 g, yellow solid, yield: 71.2%).

LC-MS (ESI): *m/z* 431.94/433.93[M+H$^+$].

Step 5: preparation of ethyl 5-bromo-1-(4-fluorophenyl)-6-(hydroxymethyl)-2-oxo-1,2-dihydropyridine-3-carboxylate (**h5**)

Ethyl 5-bromo-6-(bromomethyl)-1-(4-fluorophenyl)-2-oxo-1,2-dihydropyridine-3-carboxylate (**h4**) (16.8 g, 38.8 mmol) and saturated aqueous sodium bicarbonate solution (150 mL) were added to a reaction flask containing tetrahydrofuran (150 mL). The reaction mixture was heated to 60 °C and stirred overnight. After the reaction was complete, the reaction mixture was extracted with ethyl acetate (100 mL × 3). The combined organic phases were washed with saturated brine, dried over anhydrous sodium sulfate and filtered, and the filtrate was concentrated under reduced pressure. The residue was purified by silica gel column chromatography (eluent: PE:EA = 1:3) to give ethyl 5-bromo-1-(4-fluorophenyl)-6-(hydroxymethyl)-2-oxo-1,2-dihydropyridine-3-carboxylate (**h5**) (7.2 g, yellow solid, yield: 50.2%).

LC-MS (ESI): *m/z* 369.95/371.95[M+H$^+$].

Step 6: preparation of ethyl 5-bromo-1-(4-fluorophenyl)-6-formyl-2-oxo-1,2-dihydropyridine-3-carboxylate (**h6**)

Oxalyl chloride (208 mg, 1.64 mmol) was added to a reaction flask containing anhydrous dichloromethane (5 mL). The reaction mixture was sealed in the flask and purged with nitrogen three times. After being cooled to -78 ° C, the reaction mixture was added dropwise with dimethyl sulfoxide (176 mg, 2.26 mmol) slowly and then stirred at this temperature for 20 min after the addition was completed. Then the resulting mixture was added dropwise with a solution of ethyl 5-bromo-1-(4-fluorophenyl)-6-(hydroxymethyl)-2-oxo-1,2-dihydropyridine-3-carboxylate (**h5**) (380 mg, 1.03 mmol) in dichloromethane (5 mL) slowly and stirred at this temperature for 30 min after the addition was completed. A solution of triethylamine (520 mg, 5.15 mmol) in dichloromethane was then slowly added dropwise, and then the resulting reaction mixture was stirred at this temperature for 30 min after the addition was completed. After the reaction was completed, the reaction mixture was added dropwise with water to quench the reaction, and then extracted with dichloromethane (10 mL × 3). The combined organic phases were washed with saturated brine, dried over anhydrous sodium sulfate and filtered, and the filtrate was concentrated under reduced pressure to give crude ethyl 5-bromo-1-(4-fluorophenyl)-6-formyl-2-oxo-1,2-dihydropyridine-3-carboxylate (**h6**) (380 mg, yellow solid, yield: 100%).

LC-MS (ESI): *m/z* 367.94/369.95[M+H$^+$].

Step 7: preparation of 5-bromo-6-(ethoxycarbonyl)-1-(4-fluorophenyl)-2-oxo-1,2-dihydro pyridine-3-carboxylic acid (**h**)

The crude ethyl 5-bromo-1-(4-fluorophenyl)-6-formyl-2-oxo-1,2-dihydropyridine-3-carboxylate (**h6**) (7.2 g, 19.6 mmol), sodium dihydrogen phosphate (1.17 g, 9.8 mmol), hydrogen peroxide (20 mL) and sodium chlorite (3.5 g, 39.2 mmol) were added to a reaction flask containing acetonitrile (50 mL). The reaction mixture was stirred at room temperature overnight. After the reaction was completed, the reaction mixture was diluted with water (100 mL) and extracted with ethyl acetate (50 mL × 3). The combined organic phases were washed with saturated brine, dried over anhydrous sodium sulfate and filtered, and the filtrate was concentrated under reduced pressure. The residue was purified by slurrying with a mixed solution of petroleum ether and ethyl acetate (PE:EA =10:1, 50 mL) to give 5-bromo-6-(ethoxycarbonyl)-1-(4-fluorophenyl)-2-oxo-1,2-dihydropyridine-3-carboxylic acid (**h**) (5.6 g, yellow solid, yield: 74.4%).

LC-MS (ESI): *m/z* 383.99/385.97[M+H$^+$].

Preparation Example 9: preparation of 6-cyano-1-(4-fluorophenyl)-5-methyl-2-oxo-1,2-dihydropyridine-3-carboxylic acid (intermediate **i**)

Step 1: preparation of ethyl 5-bromo-6-carbamoyl-1-(4-fluorophenyl)-2-oxo-1,2-dihydropyridine-3-carboxylate **(i1)**

5-bromo-6-(ethoxycarbonyl)-1-(4-fluorophenyl)-2-oxo-1,2-dihydropyridine-3-carboxylic acid (**h**) (500 mg, 1.3 mmol), ammonium chloride (348 mg, 6.5 mmol), PyBrOP (760 mg, 1.6 mmol) and DIPEA (503 mg, 3.9 mmol) were added to a reaction flask containing DMF (8 mL). The reaction mixture was stirred at room temperature for 1 h. After the reaction was completed, the reaction mixture was added with saturated aqueous sodium bicarbonate solution (20 mL) to quench the reaction, and then extracted with ethyl acetate (10 mL × 3). The combined organic phases were

washed with saturated brine, dried over anhydrous sodium sulfate and filtered, and the filtrate was concentrated under reduced pressure. The residue was purified by silica gel column chromatography (eluent: PE:EA = 1:3) to give ethyl 5-bromo-6-carbamoyl-1-(4-fluorophenyl)-2-oxo-1,2-dihydropyridine-3-carboxylate **(i1)** (400 mg, white solid, yield: 80.3%).

LC-MS (ESI): *m/z* 383.01/385.03[M+H+].

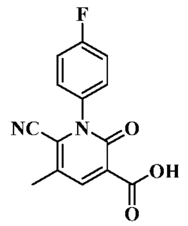

i

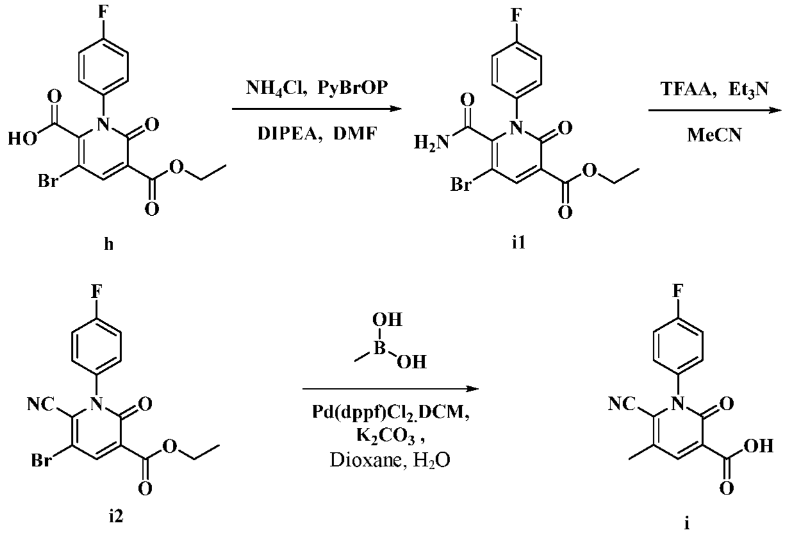


h i1 i2 i

Step 2: preparation of ethyl 5-bromo-6-cyano-1-(4-fluorophenyl)-2-oxo-1,2-dihydropyridine-3-carboxylate (**i2**)

Ethyl 5-bromo-6-carbamoyl-1-(4-fluorophenyl)-2-oxo-1,2-dihydropyridine-3-carboxylate **(i1)** (100 mg, 0.26 mol) was added to a reaction flask containing acetonitrile (5 mL). After being cooled to 0 °C, the reaction mixture was added dropwise with trifluoroacetic anhydride (103 mg, 0.52 mol) and triethylamine (80 mg, 0.78 mol) slowly and successively. The reaction mixture was stirred at 0 °C for 1 h. After the reaction was completed, the reaction mixture was diluted with water (10 mL) and extracted with ethyl acetate (10 mL × 3). The combined organic phases were washed with saturated brine, dried over anhydrous sodium sulfate and filtered, and the filtrate was concentrated under reduced pressure to give crude ethyl 5-bromo-6-cyano-1-(4-fluorophenyl)-2-oxo-1,2-dihydropyridine-3-carboxylate (**i2**) (96 mg, yellow solid, yield: 100%).

LC-MS (ESI): *m/z* 364.95/366.98[M+H+].

Step 3: preparation of 6-cyano-1-(4-fluorophenyl)-5-methyl-2-oxo-1,2-dihydropyridine-3-carboxylic acid (intermediate **i**)

Ethyl 5-bromo-6-cyano-1-(4-fluorophenyl)-2-oxo-1,2-dihydropyridine-3-carboxylate (**i2**) (100 mg, 0.27 mmol), methylboronic acid (164 mg, 2.7 mmol), potassium carbonate (113 mg, 0.82 mmol) and $Pd(dppf)Cl_2$.DCM (44 mg, 0.055 mmol) were added to a reaction flask containing 1,4-dioxane (9 mL) and water (3 mL) at room temperature. The reaction mixture was sealed in the flask, purged with nitrogen three times, heated to 100 °C in a microwave reactor and then stirred for 30 min. After being cooled to room temperature, the reaction mixture was diluted with water (10 mL) and extracted with ethyl acetate (10 mL × 3), and the organic phase was discarded. The aqueous phase was acidified to pH 3-4 with 1 N aqueous hydrochloric acid solution and then extracted with a mixed solvent of isopropyl alcohol and dichloromethane (15% isopropyl alcohol) (10 mL × 3). The combined organic phases were washed with saturated brine, dried over anhydrous sodium sulfate and filtered, and the filtrate was concentrated under reduced pressure to give crude 6-cyano-1-(4-fluorophenyl)-5-methyl-2-oxo-1,2-dihydropyridine-3-carboxylic acid (intermediate *i*) (30 mg, yellow oil, yield: 40.3%).

LC-MS (ESI): *m/z* 273.08[M+H+].

Preparation Example 10: preparation of 6-cyano-5-cyclopropyl-1-(4-fluorophenyl)-2-oxo-1,2-dihydropyridine-3-carboxylic acid (intermediate **j**)

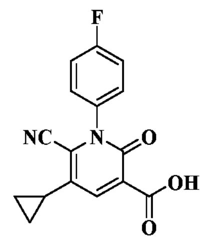

j

The same procedures as in Preparation Example 9 were performed, except that cyclopropylboronic acid was used in place of methylboronic acid, to give 6-cyano-5-cyclopropyl-1-(4-fluorophenyl)-2-oxo-1,2-dihydropyridine-3-carboxylic acid (intermediate **j**) (yellow oil, one-step yield: 65.3%).
LC-MS (ESI): *m/z* 299.10 [M+H+].

Preparation Example 11: preparation of 6-cyano-1-(4-fluorophenyl)-2-oxo-5-(prop-1-en-2-yl)-1,2-dihydropyridine-3-carboxylic acid (intermediate **k**)

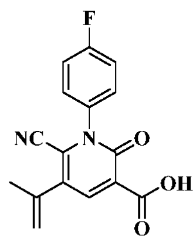

k

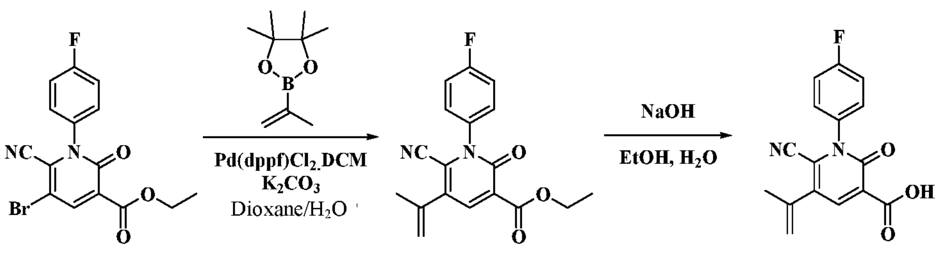


i2 k1 k

Step 1: preparation of ethyl 6-cyano-1-(4-fluorophenyl)-2-oxo-5-(prop-1-en-2-yl)-1,2-dihydropyridine-3-carboxylate (intermediate **k1**)

Ethyl 5-bromo-6-cyano-1-(4-fluorophenyl)-2-oxo-1,2-dihydropyridine-3-carboxylate (**i2**) (100 mg, 0.27 mmol), pinacol isopropenylborate (230 mg, 1.37 mmol), potassium carbonate (113 mg, 0.82 mmol) and Pd(dppf)Cl·DCM (44 mg, 0.055 mmol) were added to a reaction flask containing 1,4-dioxane (9 mL) and water (3 mL) at room temperature. The reaction mixture was sealed in the flask, purged with nitrogen three times, heated to 100 °C in a microwave reactor and then stirred for 30 min. After being cooled to room temperature, the reaction mixture was diluted with water (10 mL) and then extracted with ethyl acetate (10 mL × 3). The combined organic phases were washed with saturated brine, dried over anhydrous sodium sulfate and filtered, and the filtrate was concentrated under reduced pressure. The residue

was purified by silica gel column chromatography (eluent: PE:EA = 1:1) to give ethyl 6-cyano-1-(4-fluorophenyl)-2-oxo-5-(prop-1-en-2-yl)-1,2-dihydropyridine-3-carboxylate (intermediate **k1**) (60 mg, yellow oil, yield: 66.7%).

LC-MS (ESI): *m/z* 327.14[M+$H^+$].

Step 2: preparation of 6-cyano-1-(4-fluorophenyl)-2-oxo-5-(prop-1-en-2-yl)-1,2-dihydropyridine-3-carboxylic acid (intermediate **k**)

Ethyl 6-cyano-1-(4-fluorophenyl)-2-oxo-5-(prop-1-en-2-yl)-1,2-dihydropyridine-3-carboxylate (intermediate **k1**) (60 mg, 0.18 mmol) was added to a reaction flask containing ethanol (3 mL). After being cooled to 0 °C, the reaction mixture was added dropwise with aqueous sodium hydroxide solution (1 mL, 10 mg, 0.24 mmol) slowly, and after the addition was completed, the reaction mixture was warmed to room temperature and stirred for 30 min. After the reaction was completed, the reaction mixture was concentrated under reduced pressure to give crude 6-cyano-1-(4-fluorophenyl)-2-oxo-5-(prop-1-en-2-yl)-1,2-dihydropyridine-3-carboxylic acid (intermediate **k**) (60 mg, yellow solid, yield: 100%).

LC-MS (ESI): *m/z* 300.12[M+$H^+$].

Preparation Example 12: preparation of 5-bromo-6-(((*tert*-butoxycarbonyl)(methyl)amino)-methyl)-1-(4-fluorophenyl)-2-oxo-1,2-dihydropyridine-3-carboxylic acid (intermediate **l**)

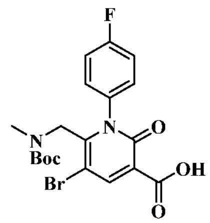

l

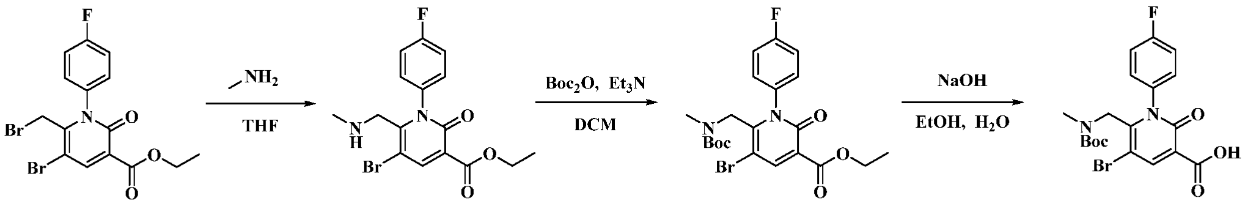


h4 l1 l2 l

Step 1: preparation of ethyl 5-bromo-1-(4-fluorophenyl)-6-((methylamino)methyl)-2-oxo-1,2-dihydropyridine-3-carboxylate (**l1**)

Ethyl 5-bromo-6-(bromomethyl)-1-(4-fluorophenyl)-2-oxo-1,2-dihydropyridine-3-carboxylate (**h4**) (300 mg, 0.75 mmol) was added to a reaction flask containing tetrahydrofuran (4 mL). After being cooled to 0 °C, the reaction mixture was added dropwise with aqueous methylamine (0.5 mL) slowly, and after the addition was completed, the reaction mixture was warmed to room temperature and stirred for 1 h. After the reaction was completed, the reaction mixture was diluted with water and extracted with ethyl acetate (10 mL × 3). The combined organic phases were washed with saturated brine, dried over anhydrous sodium sulfate and filtered, and the filtrate was concentrated under reduced pressure. The residue was purified by silica gel column chromatography (eluent: PE:EA = 2:1) to give ethyl 5-bromo-1-(4-fluoro-phenyl)-6-((methylamino)methyl)-2-oxo-1,2-dihydropyridine-3-carboxylate (**l1**) (250 mg, yellow solid, yield: 86.7%).

LC-MS (ESI): *m/z* 383.13/385.10[M+$H^+$].

Step 2: preparation of ethyl 6-(((*tert*-butoxycarbonyl)methyl)amino)(methyl)-5-bromo-1-(4-fluorophenyl)-2-oxo-1,2-dihydropyridine-3-carboxylate (**l2**)

Ethyl 5-bromo-1-(4-fluorophenyl)-6-((methylamino)methyl)-2-oxo-1,2-dihydropyridine-3-carboxylate (**l1**) (250 mg, 0.65 mmol), $Boc_2O$ (202 mg, 0.94 mmol) and triethylamine (94 mg, 0.94 mmol) were added to a reaction flask

containing dichloromethane (4 mL). The reaction mixture was stirred at room temperature for 3 h. After the reaction was completed, the reaction mixture was concentrated under reduced pressure, and the residue was purified by silica gel column chromatography (eluent: PE:EA = 5:1) to give ethyl 6-(((*tert*-butoxycarbonyl)(methyl)amino) methyl)-5-bromo-1-(4-fluorophenyl)-2-oxo-1,2-dihydropyridine-3-carboxylate (**l2**) (230 mg, yellow solid, yield: 73.3%).

LC-MS (ESI): *m*/*z* 483.15/485.13[M+H$^+$].

Step 3: preparation of 5-bromo-6-(((*tert*-butoxycarbonyl)(methyl)amino)methyl)-1-(4-fluorophenyl)-2-oxo-1,2-dihydropyridine-3-carboxylic acid (intermediate **l**)

Ethyl 6-(((*tert*-butoxycarbonyl)(methyl)amino)methyl)-5-bromo-1-(4-fluorophenyl)-2-oxo-1,2-dihydropyridine-3-carboxylate (**l2**) (230 mg, 0.48 mmol) was added to a reaction flask containing ethanol (4.5 mL). After being cooled to 0 °C, the reaction mixture was added dropwise with aqueous sodium hydroxide solution (1.5 mL, 25 mg, 0.62 mmol) slowly, and after the addition was completed, the reaction mixture was warmed to room temperature and stirred for 30 min. After the reaction was completed, the reaction mixture was concentrated under reduced pressure to give crude 5-bromo-6-(((*tert*-butoxycarbonyl)(methyl)amino)methyl)-1-(4-fluoro-phenyl)-2-oxo-1,2-dihydropyridine-3-carboxylic acid (intermediate **l**) (230 mg, yellow solid, yield: 100%).

LC-MS (ESI): *m*/*z* 455.12/457.09[M+H$^+$].

Preparation Example 13: preparation of 6-((*tert*-butoxycarbonyl)amino)methyl)-5-bromo-1-(4-fluorophenyl)-2-oxo-1,2-dihydropyridine-3-carboxylic acid (intermediate **m**)

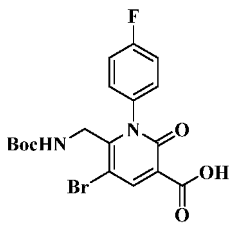

**m**

The same procedures as in Preparation Example 12 were performed, except that aqueous ammonia was used in place of aqueous methylamine solution, to give 6-((*tert*-butoxy-carbonyl)-amino)methyl)-5-bromo-1-(4-fluorophenyl)-2-oxo-1,2-dihydropyridine-3-carboxylic acid (intermediate **m**) (yellow solid, three-step yield: 45.3%).

LC-MS (ESI): *m*/*z* 441.15/443.11[M+H$^+$].

Preparation Example 14: preparation of 5-bromo-1-(4-fluorophenyl)-6-(methoxymethyl)-2-oxo-1,2-dihydropyridine-3-carboxylic acid (intermediate **n**)

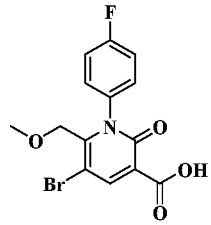

n

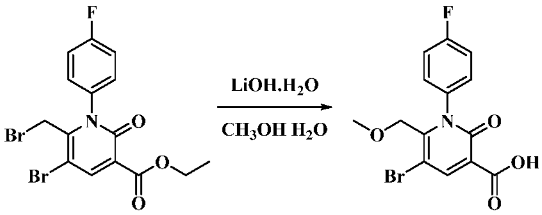


h4 n

Step 1: preparation of 5-bromo-1-(4-fluorophenyl)-6-(methoxymethyl)-2-oxo-1,2-dihydropyridine-3-carboxylic acid (intermediate **n**)

Ethyl 5-bromo-6-(bromomethyl)-1-(4-fluorophenyl)-2-oxo-1,2-dihydropyridine-3-carboxylate (**h4**) (500 mg, 1.15 mmol) was added to a reaction flask containing methanol (8 mL). After being cooled to 0 °C, the reaction mixture was added dropwise with aqueous lithium hydroxide solution (1.4 mL, 241 mg, 5.75 mmol) slowly, and after the addition was completed, the reaction mixture was warmed to room temperature and stirred for 30 min. After the reaction was completed, the reaction mixture was concentrated under reduced pressure to give crude 5-bromo-1-(4-fluorophenyl)-6-(methoxymethyl)-2-oxo-1,2-dihydropyridine-3-carboxylic acid (intermediate **n**) (500 mg, yellow solid, yield: 100%).

LC-MS (ESI): *m*/*z* 355.95/357.95[M+H$^+$].

Preparation Example 15: preparation of *tert*-butyl 4-(4-(6-amino-5-(4-amino-2-fluorophenyl)-pyridin-3-yl)-1*H*-pyrazol-1-yl)piperidine-1-carboxylate (intermediate **p**)

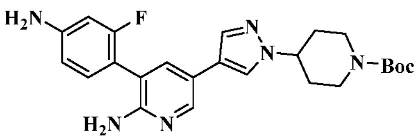

p

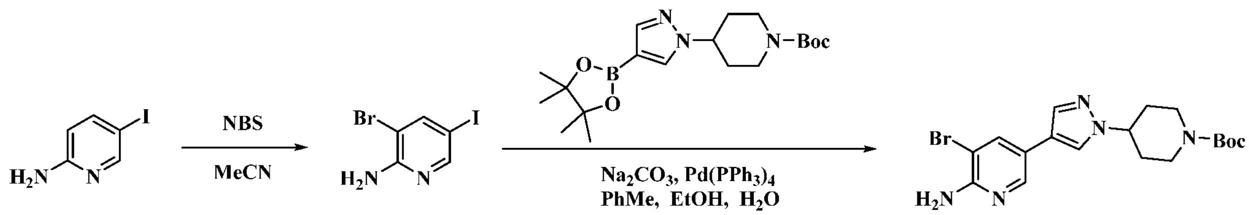


p1

p2

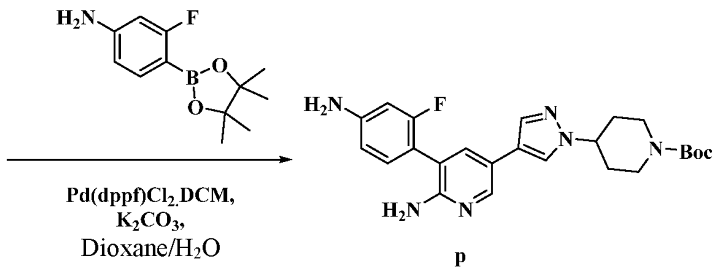


p

Step 1: preparation of 3-bromo-5-iodopyridin-2-amine (intermediate **p1**)

5-iodopyridin-2-amine (5.0 g, 22.73 mmol) and NBS (4.0 g, 22.73 mmol) were added to a reaction flask containing acetonitrile (100 mL). The reaction mixture was stirred at 50 °C for 12 h, then cooled to room temperature, filtered and concentrated under reduced pressure. The residue was purified by silica gel column chromatography (eluent: PE:EA = 20:1) to give 3-bromo-5-iodopyridin-2-amine (intermediate **p1**) (2.5 g, yellow solid, yield: 36.8%).
LC-MS (ESI): *m/z* 299.35/301.31[M+H$^+$].

Step 2: preparation of *tert*-butyl 4-(4-(6-amino-5-bromopyridin-3-yl)-1*H*-pyrazol-1-yl)-piperidine-1-carboxylate (intermediate *p2*)

3-bromo-5-iodopyridin-2-amine (intermediate **p1**) (2.5 g, 8.36 mmol), *tert*-butyl 4-(4-(4,4,5,5-tetramethyl-1,3,2-dioxaborolan-2-yl)-1*H*-pyrazol-1-yl)piperidine-1-carboxylate (Beijing Ouhe Technology, 3.78 g, 10.03 mmol), sodium carbonate (2.66 g, 25.08 mmol) and $Pd(PPh_3)_4$ (483 mg, 0.42 mmol) were added to a reaction flask containing a mixed solution of toluene, ethanol and water (50 mL, 2:2:1) at room temperature. The reaction mixture was sealed in the flask, purged with nitrogen three times, heated to 80 °C and then stirred for 12 h. After the reaction was completed, the reaction mixture was filtered. The filtrate was diluted with water (50 mL) and then extracted with ethyl acetate (50 mL × 3). The organic phases were combined, washed with saturated brine, dried over anhydrous sodium sulfate and filtered, and the filtrate was concentrated under reduced pressure. The residue was purified by silica gel column chromatography (eluent: PE:EA = 1:1) to give *tert*-butyl 4-(4-(6-amino-5-bromopyridin-3-yl)-1*H*-pyrazol-1-yl)piperidine-1-carboxylate (intermediate **p2**) (2.8 g, yellow solid, yield: 79.4%).
LC-MS (ESI): *m/z* 422.24/424.21[M+H$^+$].

Step 3: preparation of *tert*-butyl 4-(4-(6-amino-5-(4-amino-2-fluorophenyl)pyridin-3-yl)-1*H*-pyrazol-1-yl)piperidine-1-carboxylate (intermediate **p**)

*Tert*-butyl 4-(4-(6-amino-5-bromopyridin-3-yl)-1*H*-pyrazol-1-yl)piperidine-1-carboxylate (intermediate **p2**) (2.8 g, 6.64 mmol), 4-amino-2-fluorophenylboronic acid pinacol ester (Shanghai Bidepharm, 1.88 g, 7.96 mmol), potassium carbonate (2.75 g, 19.92 mmol) and Pd(dppf)$Cl_2$.DCM (538 mg, 0.66 mmol) were added to a reaction flask containing a mixed solution of 1,4-dioxane and water (40 mL, 4:1) at room temperature. The reaction mixture was sealed in the flask, purged with nitrogen three times, heated to 90 °C and then stirred overnight. After the reaction was completed, the reaction mixture was filtered. The filtrate was diluted with water (50 mL) and then extracted with ethyl acetate (50 mL × 3). The organic phase was washed with saturated brine, dried over anhydrous sodium sulfate, filtered and concentrated under reduced pressure. The residue was purified by silica gel column chromatography (eluent: PE:EA = 1:2) to give *tert*-butyl 4-(4-(6-amino-5-(4-amino-2-fluorophenyl)-pyridin-3-yl)-1*H*-pyrazol-1-yl)piperidine-1-carboxylate (intermediate **p**) (2.3 g, yellow solid, yield: 77.1%).
LC-MS (ESI): *m/z* 453.43[M+H$^+$].

Preparation Example 16: preparation of 3-(4-amino-2-fluorophenyl)-5-(1-ethyl-1*H*-pyrazol-4-yl)pyridin-2-amine (intermediate **q**)

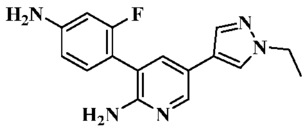

q

The same procedures as in Preparation Example 15 were performed, except that 1-ethyl-4-(4,4,5,5-tetramethyl-1,3,2-dioxaborolan-2-yl)-1*H*-pyrazole (Beijing Ouhe) was used in place of *tert-butyl* 4-(4-(4,4,5,5-tetramethyl-1,3,2-dioxaborolan-2-yl)-1*H*-pyrazol-1-yl)piperidine-1-carboxylate, so as to give 3-(4-amino-2-fluorophenyl)-5-(1-ethyl-1*H*-pyrazol-4-yl)pyridin-2-amine (intermediate **q**) (yellow solid, two-step yield: 68.8%).
LC-MS (ESI): *m/z* 298.17[M+H$^+$].

Preparation Example 17: preparation of 3-(4-amino-2-fluorophenyl)-5-(1-(tetrahydro-2*H*-pyran-4-yl)-1*H*-pyrazol-4-yl)pyridin-2-amine (intermediate **r**)

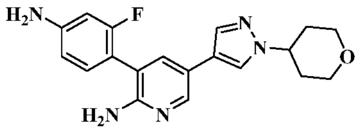

r

The same procedures as in Preparation Example 15 were performed, except that 1-(tetrahydro-2*H*-pyran-4-yl)-4-(4,4,5,5-tetramethyl-1,3,2-dioxaborolan-2-yl)-1*H*-pyrazole (Beijing Ouhe) was used in place of *tert*-butyl 4-(4-(4,4,5,5-tetramethyl-1,3,2-dioxaborolan-2-yl)-1*H*-pyrazol-1-yl)piperidine-1-carboxylate, so as to give 3-(4-amino-2-fluorophenyl)-5-(1-(tetrahydro-2*H*-pyran-4-yl)-1*H*-pyrazol-4-yl)pyridin-2-amine (intermediate r) (yellow solid, two-step yield: 72.3%).
LC-MS (ESI): *m/z* 354.31[M+H$^+$].

Preparation Example 18: preparation of 3-(4-amino-2-fluorophenyl)-5-(3,4-dimethoxyphenyl)pyridin-2-amine (intermediate **s**)

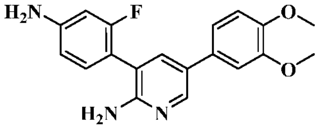

s

The same procedures as in Preparation Example 15 were performed, except that (3,4-dimethoxyphenyl)boronic acid (Beijing Ouhe) was used in place of *tert*-butyl 4-(4-(4,4,5,5-tetramethyl-1,3,2-dioxaborolan-2-yl)-1*H*-pyrazol-1-yl)piperidine-1-carboxylate, to give 3-(4-amino-2-fluorophenyl)-5-(3,4-dimethoxyphenyl)pyridin-2-amine (intermediate **s**) (yellow solid, two-step yield: 79.1%).
LC-MS (ESI): *m/z* 340.22[M+H$^+$].

Preparation Example 19: preparation of 3-(4-amino-2-fluorophenyl)-5-(1-methyl-1*H*-pyrazol-4-yl)pyridin-2-amine (intermediate **t**)

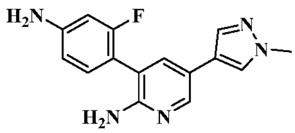

t

The same procedures as in Preparation Example 15 were performed, except that 1-methyl-4-(4,4,5,5-tetramethyl-1,3,2-dioxaborolan-2-yl)-1*H*-pyrazole (Beijing Ouhe) was used in place of *tert*-butyl 4-(4-(4,4,5,5-tetramethyl-1,3,2-dioxaborolan-2-yl)-1*H*-pyrazol-1-yl)-piperidine-1-carboxylate, so as to give 3-(4-amino-2-fluorophenyl)-5- (1-methyl-1*H*-pyrazol-4-yl)pyridin-2-amine (intermediate **t**) (yellow solid, two-step yield: 75.8%).
LC-MS (ESI): *m*/*z* 284.15[M+$H^+$].

Preparation Example 20: preparation of 3-(4-amino-2-fluorophenyl)-5-(1-(1-methylpiperidin-4-yl)-1*H*-pyrazol-4-yl)pyridin-2-amine (intermediate **u**)

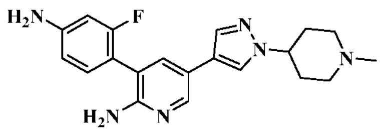

u

The same procedures as in Preparation Example 15 were performed, except that 1-methyl-4-(4-(4,4,5,5-tetramethyl-1,3,2-dioxaborolan-2-yl)-1*H*-pyrazol-1-yl)piperidine (Shanghai Bidepharm) was used in place of *tert*-butyl 4-(4-(4,4,5,5-tetramethyl-1,3,2-dioxaborolan-2-yl)-1*H*-pyrazol-1-yl)piperidine-1-carboxylate, so as to prepare 3-(4-amino-2-fluorophenyl)-5-(1-(1-methylpiperidin-4-yl)-1*H*-pyrazol-4-yl)pyridin-2-amine (intermediate **u**) (yellow solid, two-step yield: 32.4%).
LC-MS (ESI): *m*/*z* 367.33 [M+$H^+$].

Preparation Example 21: preparation of 3-bromo-5-(1-ethyl-1*H*-pyrazol-4-yl)pyridin-2-amine (**v**)

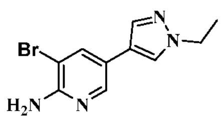

v

The same procedures as steps 1 and 2 in Preparation Examples 15 were performed, except that 1-ethyl-1*H*-pyrazole-4-boronic acid pinacol ester was used in place of *tert*-butyl 4-[4-(4,4,5,5-tetramethyl-1,3,2-dioxaborolan-2-yl)-1*H*-pyrazol-1-yl]piperidine-1-carboxylate, so as to give 3-bromo-5-(1-ethyl-1*H*-pyrazol-4-yl)pyridin-2-amine (**v**) (yellow solid, two-step yield: 43%).
LC-MS (ESI): *m*/*z* 267.01,269.08[M+$H^+$].

Preparation Example 22: preparation of 3-bromo-5-(1-methyl-1*H*-pyrazol-4-yl)pyridin-2-amine (**w**)

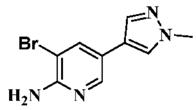

w

The same procedures as steps 1 and 2 in Preparation Examples 15 were performed, except that 1-Methyl-1*H*-pyrazole-4-boronic acid pinacol ester was used in place of *tert-butyl* 4-[4-(4,4,5,5-tetramethyl-1,3,2-dioxaborolan-2-yl)-1*H*-pyrazol-1-yl]piperidine-1-carboxylate, so as to give 3-bromo-5-(1-methyl-1*H*-pyrazol-4-yl)pyridin-2-amine (**w**) (yellow solid, two-step yield: 36%).

LC-MS (ESI): *m*/*z* 253.01,255.08[M+H$^+$].

Preparation Example 23: preparation of 3-bromo-5-(1-(tetrahydro-2*H*-pyran-4-yl)-1*H*-pyrazol-4-yl)-pyridin-2-amine (**x**)

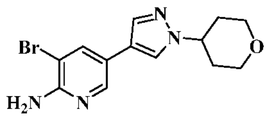

x

The same procedures as steps 1 and 2 in Preparation Examples 15 were performed, except that 1-(tetrahydropyran-4-yl)-1*H*-pyrazole-4-boronic acid pinacol ester was used in place of *tert-butyl* 4-[4-(4,4,5,5-tetramethyl-1,3,2-dioxaborolan-2-yl)-1*H*-pyrazol-1-yl]piperidine-1-carboxylate, so as to give 3-bromo-5-(1-(tetrahydro-2*H*-pyran-4-yl)-1*H*-pyrazol-4-yl)pyridin-2-amine (**x**) (yellow solid, two-step yield: 49%).

LC-MS (ESI): *m*/*z* 323.18/325.14[M+H$^+$].

Preparation Example 24: preparation of 6-cyano-*N*-(2,5-difluoro-4-(4,4,5,5-tetramethyl-1,3,2-dioxaborolan-2-yl)-phenyl)-5-(4-fluorophenyl)-1-isopropyl-4-oxo-1,4-dihydropyridine-3-carboxamide (**y**)

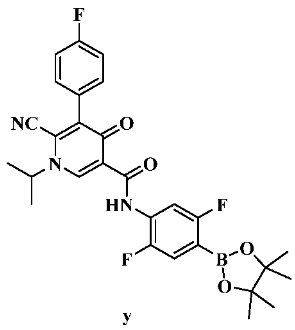

y

The same procedures as in Preparation Example 5 were performed, except that 6-(ethoxycarbonyl)-5-(4-fluorophenyl)-1-isopropyl-4-oxo-1,4-dihydropyridine-3-carboxylic acid (**a**) was used in place of 1-cyclopropyl-6-(ethoxycarbonyl)-5-(4-fluorophenyl)-4-oxo-1,4-dihydropyridine-3-carboxylic acid (**d**), and 4-bromo-2,5-difluoroaniline was used in place of 4-bromo-3-fluoroaniline, so as to give 6-cyano-*N*-(2,5-difluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (**y**) (yellow solid, five-step yield: 18%).

LC-MS (ESI): *m*/*z* 538.25[M+H$^+$].

Preparation Example 25: preparation of 3-bromo-5-(1-(1-methylpiperidin-4-yl)-1*H*-pyrazol-4-yl)pyridin-2-amine (**z**)

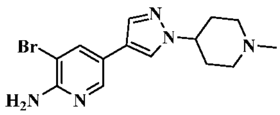

z

The same procedures as steps 1 and 2 in Preparation Example 15 were performed, except that 1-methyl-4-(4-(4,4,5,5-tetramethyl-1,3,2-dioxaborolan-2-yl)-1*H*-pyrazol-1-yl)piperidine (Shanghai Bidepharm) was used in place of *tert*-butyl 4-(4-(4,4,5,5-tetramethyl-1,3,2-dioxaborolan-2-yl)-1*H*-pyrazol-1-yl)piperidine-1-carboxylate, so as to give 3-bromo-5-(1-(1-methylpiperidin-4-yl)-1*H*-pyrazol-4-yl)pyridin-2-amine (**z**) (yellow solid, two-step yield: 23%).
LC-MS (ESI): *m*/*z* 336.22/338.18[M+H$^+$].

Preparation Example 26: preparation of 4-((6,7-dimethoxyquinolin-4-yl)oxy)-3-fluoroaniline hydrochloride (intermediate **aa**)

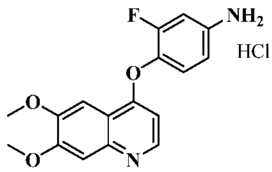

aa

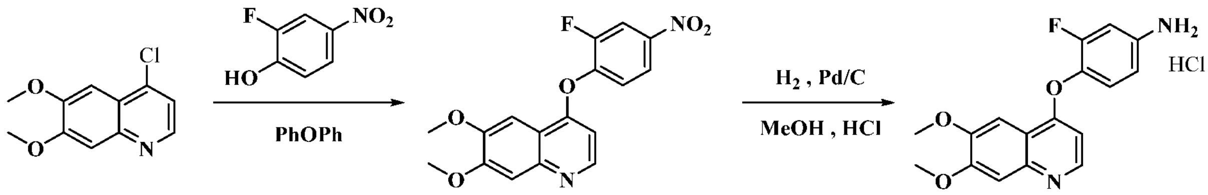


aa1

aa

Step 1: preparation of 4-(2-fluoro-4-nitrophenoxy)-6,7-dimethoxyquinoline (intermediate **aa1**)

4-chloro-6,7-dimethoxyquinoline (300 g, 1.341 mol) and 2-fluoro-4-nitrophenol (273 g, 1.743 mol) were added to a reaction flask containing diphenyl ether (2000 mL) at room temperature. The reaction mixture was heated to 140 °C and stirred for 24 h, and after the reaction mixture was cooled to room temperature, an off-white solid was precipitated out. The reaction mixture was added with petroleum ether (2000 mL), stirred for 2 h, and then filtered. The filter cake was washed with petroleum ether (1000 mL), and the solid was collected to give 4-(2-fluoro-4-nitrophenoxy)-6,7-dimethoxyquinoline (intermediate **aa1**) (440 g, off-white solid, yield: 95.38%).
LC-MS (ESI): *m*/*z* 345.23[M+H$^+$]

Step 2: preparation of 4-((6,7-dimethoxyquinolin-4-yl)oxy)-3-fluoroaniline hydrochloride (intermediate **aa**)

4-(2-fluoro-4-nitrophenoxy)-6,7-dimethoxyquinoline (300 g, 0.872 mol), palladium on carbon (30 g, 10%) and concentrated hydrochloric acid (72 mL, 0.872 mol) were added to a reaction flask containing methanol (1500 mL) at room temperature. After being sealed in the flask and purged with nitrogen three times, the reaction mixture was purged with hydrogen three times and then stirred for 36 h. The reaction mixture was added with methanol (1500 mL) and heated to 60 °C until the solid was completely dissolved. The reaction mixture was then filtered, the filtrate was cooled to 0 °C slowly, and a lot of solids were precipitated out. The reaction mixture was filtered, and the filter cake was washed with ethyl acetate (1500 mL). The solids were collected to give 4-((6,7-dimethoxyquinolin-4-yl)oxy)-3-fluoroaniline hydrochloride (intermediate **aa**) (290 g, light yellow solid, yield: 94.9%).
LC-MS (ESI): *m*/*z* 315.27[M+H$^+$].

Preparation Example 27: preparation of *N*-(3-fluoro-4-(4,4,5,5-tetramethyl-1,3,2-dioxa-borolan-2-yl)phenyl)-3-(4-fluorophenyl)-1-isopropyl-2,4-dioxo-1,2,3,4-tetrahydropyrimidine-5-carboxamide (**bb**)

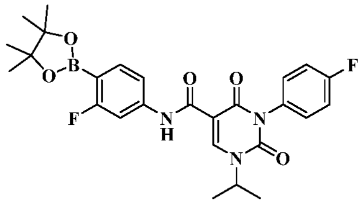

bb

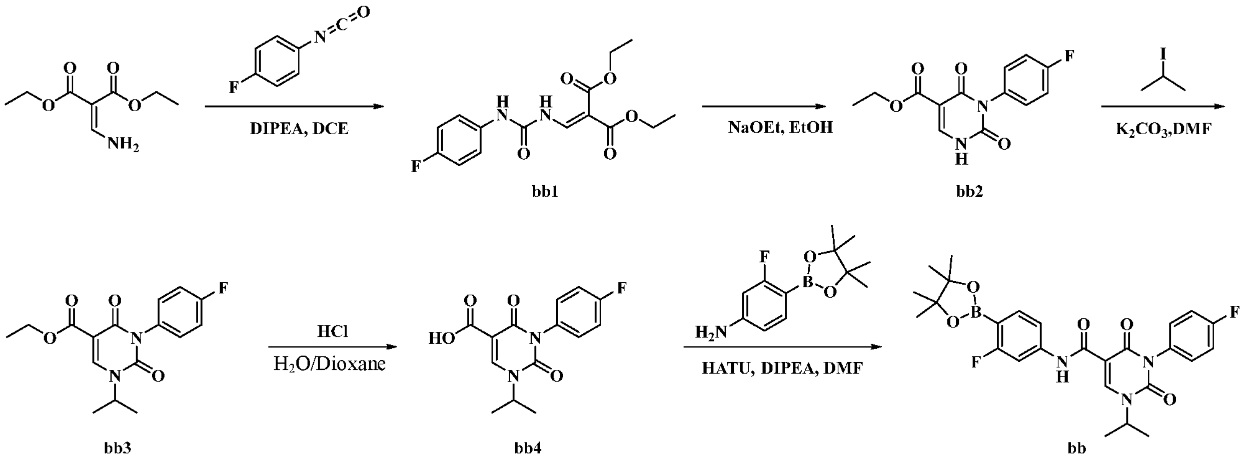


bb1 bb2 bb3 bb4 bb

Step 1: preparation of diethyl 2-((3-(4-fluorophenyl)ureido)methylene)malonate (**bb1**)

Diethyl aminomethylenemalonate (1.7 g, 9.1 mmol), fluorophenyl isocyanate (1.37 g, 10 mmol) and DIPEA (2.35 g, 18.2 mmol) were added to a reaction flask containing 1,2-dichloroethane (30 mL) at room temperature. The reaction mixture was sealed in the flask, purged with nitrogen three times, and heated to reflux for 6 h. After the reaction was completed, the reaction mixture was cooled to 0 °C, and a solid was precipitated out. The reaction mixture was filtered, and the filter cake was washed with diethyl ether. The solid was collected and dried to give diethyl 2-((3-(4-fluorophenyl)ureido)methylene)malonate (**bb1**) (980 mg, white solid, yield: 33.7%).
LC-MS (ESI): *m/z* 325.1[M+H$^+$].

Step 2: preparation of ethyl 3-(4-fluorophenyl)-2,4-dioxo-1,2,3,4-tetrahydropyrimidine-5-carboxylate (**bb2**)

Diethyl 2-((3-(4-fluorophenyl)ureido)methylene)malonate (**bb1**) (980 mg, 3 mmol) and sodium ethoxide (306 mg, 4.5 mmol) were added to a reaction flask containing ethanol (20 mL) at room temperature. The reaction mixture was stirred for 1 h and then concentrated under reduced pressure. The residue was diluted with ethyl acetate (100 mL). The organic phase was washed successively with citric acid (200 mL, 1 M) and saturated brine, dried over anhydrous sodium sulfate and filtered, and the filtrate was concentrated under reduced pressure. The residue was purified by silica gel column chromatography (eluent: DCM:MeOH = 10:1) to give ethyl 3-(4-fluorophenyl)-2,4-dioxo-1,2,3,4-tetrahydropyrimidine-5-carboxylate (**bb2**) (780 mg, white solid, yield: 92.7%).
LC-MS (ESI): *m/z* 279.1[M+H$^+$].

Step 3: preparation of ethyl 3-(4-fluorophenyl)-1-isopropyl-2,4-dioxo-1,2,3,4-tetrahydropyrimidine-5-carboxylate (**bb3**)

Ethyl 3-(4-fluorophenyl)-2,4-dioxo-1,2,3,4-tetrahydropyrimidine-5-carboxylate (**bb2**) (350 mg, 1.3 mmol), potassium carbonate (538 mg, 4.5 mmol) and 2-iodopropane (660 mg, 3.9 mmol) were added to a reaction flask containing DMF (10 mL) at room temperature. The reaction mixture was warmed to 75 °C and stirred overnight. After being cooled to room temperature, the reaction mixture was diluted with ethyl acetate (50 mL). The organic phase was washed successively with water (100 mL) and saturated brine, dried over anhydrous sodium sulfate and filtered, and the filtrate

was concentrated under reduced pressure. The residue was purified by silica gel column chromatography (eluent: PE:EA = 1:1) to give ethyl 3-(4-fluorophenyl)-1-isopropyl-2,4-dioxo-1,2,3,4-tetrahydropyrimidine-5-carboxylate (**bb3**) (380 mg, white solid, yield: 94.3%).

LC-MS (ESI): *m*/*z* 321.1[M+H$^+$].

Step 4: preparation of 3-(4-fluorophenyl)-1-isopropyl-2,4-dioxo-1,2,3,4-tetrahydropyrimidine-5-carboxylic acid (**bb4**)

Ethyl 3-(4-fluorophenyl)-1-isopropyl-2,4-dioxo-1,2,3,4-tetrahydropyrimidine-5-carboxylate (**bb3**) (380 mg, 1.2 mmol) and water (0.5 mL) were added to a reaction flask containing a solution of hydrochloric acid in 1,4-dioxane (10 mL, 4 M) at room temperature. The reaction mixture was heated to 70 °C and stirred for 5 h. After being cooled to room temperature, the reaction mixture was concentrated under reduced pressure to give 3-(4-fluorophenyl)-1-isopropyl-2,4-dioxo-1,2,3,4-tetrahydropyrimidine-5-carboxylic acid (**bb4**), which was directly used in the next step without purification.

LC-MS (ESI): *m*/*z* 293.1[M+H$^+$].

Step 5: preparation of *N*-(3-fluoro-4-(4,4,5,5-tetramethyl-1,3,2-dioxaborolan-2-yl)phenyl)-3-(4-fluorophenyl)-1-isopropyl-2,4-dioxo-1,2,3,4-tetrahydropyrimidine-5-carboxamide (**bb**)

3-(4-fluorophenyl)-1-isopropyl-2,4-dioxo-1,2,3,4-tetrahydropyrimidine-5-carboxylic acid (**bb4**) (300 mg, 1 mmol), 4-amino-2-fluorophenylboronic acid pinacol ester (244 mg, 1 mmol), HATU (608 mg, 1.6 mmol) and DIPEA (387 mg, 3 mmol) were added to a reaction flask containing DMF (6 mL). The reaction mixture was stirred at room temperature for 30 min. After the reaction was completed, the reaction mixture was added with saturated sodium bicarbonate solution to quench the reaction, and then extracted with ethyl acetate (50 mL × 3). The organic phase was washed with saturated brine, dried over anhydrous sodium sulfate and filtered, and the filtrate was concentrated under reduced pressure. The residue was purified by silica gel column chromatography (eluent: PE:EA = 5:1) to give *N*-(3-fluoro-4-(4,4,5,5-tetramethyl-1,3,2-dioxaborolan-2-yl)phenyl)-3-(4-fluorophenyl)-1-isopropyl-2,4-dioxo-1,2,3,4-tetrahydropyrimidine-5-carboxamide (**bb**) (280 mg, yellow solid, yield: 53.3%).

LC-MS (ESI): *m*/*z* 512.3 [M+H$^+$].

Preparation Example 28: preparation of 4-(4-amino-2-fluorophenoxy)-7-methoxyquinoline-6-carboxamide (intermediate **cc**)

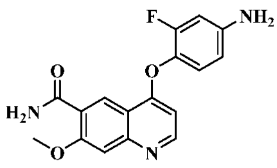

cc

Step 1: preparation of methyl 4-(((2,2-dimethyl-4,6-dioxo-1,3-dioxan-5-ylidene)methyl)-amino)-2-methoxybenzoate (intermediate **cc1**)

Methyl 4-amino-2-methoxybenzoate (Shanghai Bidepharm, 5 g, 27.6 mmol) was added to a reaction flask containing ethanol (100 mL) at room temperature. The reaction mixture was warmed to 50 °C, stirred for 10 min, and then added with 5-(methoxymethylene)-2,2-dimethyl-1,3-dioxane-4,6-dione (Shanghai Bidepharm, 5.13 g, 27.6 mmol). The reaction mixture was warmed to 80 °C and stirred for 1 h. After being cooled to room temperature, the reaction mixture was filtered, and the solid was collected to give methyl 4-(((2,2-dimethyl-4,6-dioxo-1,3-dioxan-5-ylidene)methyl)-amino)-2-methoxybenzoate (intermediate **cc1**) (8 g, brown solid, yield: 86.5%).

LC-MS (ESI): *m*/*z* 336.3[M+H$^+$].

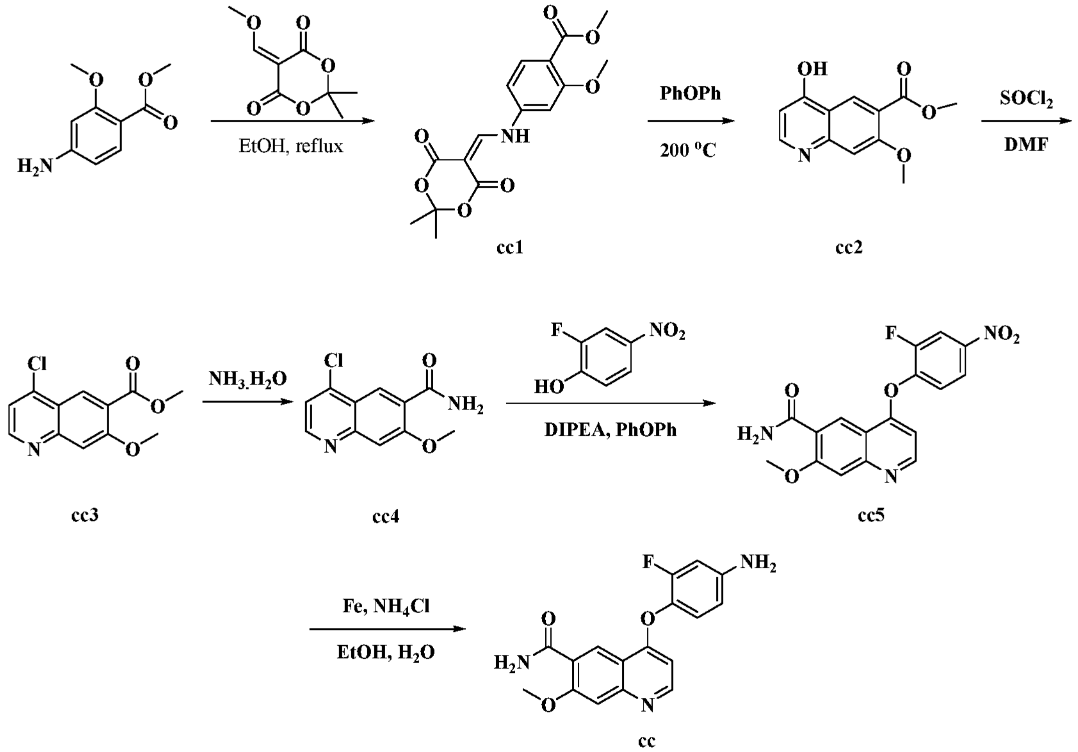

cc1 cc2 cc3 cc4 cc5 cc

Step 2: preparation of methyl 4-hydroxy-7-methoxyquinoline-6-carboxylate (intermediate **cc2**)

Methyl 4-(((2,2-dimethyl-4,6-dioxo-1,3-dioxan-5-ylidene)methyl)amino)-2-methoxy-benzoate (intermediate **cc1**) (8 g, 23.9 mmol) was added to a reaction flask containing diphenyl ether (100 mL) at room temperature. The reaction mixture was warmed to 200 °C and stirred for 8 h. After being cooled to room temperature, the reaction mixture was filtered, and a solid was collected to give methyl 4-hydroxy-7-methoxyquinoline-6-carboxylate (intermediate **cc2**) (5.2 g, brown solid, yield: 93.4%).

LC-MS (ESI): *m/z* 234.1[M+H$^+$].

Step 3: preparation of methyl 4-chloro-7-methoxyquinoline-6-carboxylate (intermediate **cc3**)

Methyl 4-hydroxy-7-methoxyquinoline-6-carboxylate (intermediate **cc2**) (1 g, 4.29 mmol) and DMF (0.1 mL) were added to a reaction flask containing thionyl chloride (20 mL) at room temperature. The reaction mixture was heated to reflux and stirred for 3 h. After being cooled to room temperature, the reaction mixture was concentrated under reduced pressure. The residue was diluted with dichloromethane (30 mL). The organic phase was washed successively with saturated aqueous sodium bicarbonate solution and saturated brine, dried over anhydrous sodium sulfate and filtered, and the filtrate was concentrated under reduced pressure to give crude methyl 4-chloro-7-methoxyquinoline-6-carboxylate (intermediate **cc3**) (1.0 g, brown solid, yield: 92.8%).

LC-MS (ESI): *m/z* 237.1/239.1[M+H$^+$].

Step 4: preparation of 4-chloro-7-methoxyquinoline-6-carboxamide (intermediate **cc4**)

Methyl 4-chloro-7-methoxyquinoline-6-carboxylate (intermediate **cc3**) (1 g, 3.98 mmol) and aqueous ammonia (33.0%, 5 mL) were added to a reaction flask containing THF (10 mL) at room temperature. The reaction mixture was sealed in the flask, heated to 80 °C and then stirred overnight. After being cooled to room temperature, the reaction mixture was filtered, and the solid was collected to give 4-chloro-7-methoxyquinoline-6-carboxamide (intermediate **cc4**) (600 mg, brown solid, yield: 63.9%).

LC-MS (ESI): *m/z* 236.2/238.2[M+H$^+$].

Step 5: preparation of 4-(2-fluoro-4-nitrophenoxy)-7-methoxyquinoline-6-carboxamide (intermediate **cc5**)

4-chloro-7-methoxyquinoline-6-carboxamide (intermediate **cc4**) (600 mg, 2.39 mmol) and 2-fluoro-4-nitrophenol (Beijing Ouhe, 750 mg, 4.78 mmol) were added to a reaction flask containing diphenylether (10 mL) at room temperature. The reaction mixture was heated to 140 °C and stirred for 10 h, and after the reaction mixture was cooled to room temperature, an off-white solid was precipitated out. The reaction mixture was filtered, and a solid was collected to give 4-(2-fluoro-4-nitrophenoxy)-7-methoxyquinoline-6-carboxamide (intermediate **cc5**) (800 mg, off-white solid, yield: 93.7%).

LC-MS (ESI): *m/z* 358.2[M+H$^+$]

Step 6: preparation of 4-(4-amino-2-fluorophenoxy)-7-methoxyquinoline-6-carboxamide (intermediate **m**)

4-(2-fluoro-4-nitrophenoxy)-7-methoxyquinoline-6-carboxamide (intermediate **m5**) (800 mg, 2.24 mmol), iron powder (627 mg, 11.2 mmol) and ammonium chloride (1.3 g, 22.7 mmol) were added to a reaction flask containing ethanol (15 mL) and water (5 mL) at room temperature. The reaction mixture was warmed to 80 °C and stirred for 1 h. After being cooled to room temperature, the reaction mixture was filtered, and the filtrate was concentrated under reduced pressure. The residue was purified by silica gel column chromatography (eluent: DCM:MeOH = 5:1) to give 4-(4-amino-2-fluorophenoxy)-7-methoxyquinoline-6-carboxamide (intermediate **cc**) (600 mg, grey solid, yield: 81.7%).

LC-MS (ESI): *m/z* 328.3[M+H$^+$]

Preparation Example 29: preparation of 1-(methyl-$d_3$)-4-(4,4,5,5-tetramethyl-1,3,2-dioxa-borolan-2-yl)-1*H*-pyrazole (intermediate **dd**)

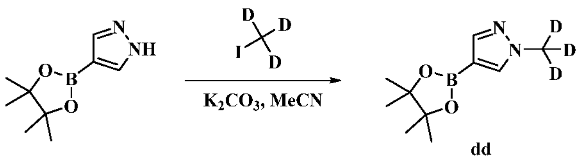


dd

4-pyrazoleboronic acid pinacol ester (Shanghai Bidepharm) (1.2 g, 0.5 mmol), deuterated iodomethane (Energy Chemical) (1.7 g, 1 mmol) and potassium carbonate (1.7 g, 1 mmol) were added to a reaction flask containing acetonitrile (15 mL) at room temperature. The reaction mixture was stirred at room temperature for 48 h. After the reaction was completed, the reaction mixture was filtered, and the filtrate was concentrated under reduced pressure. The residue was purified by silica gel column chromatography (eluent: PE:EA = 5:1) to give 1-(methyl-$d_3$)-4-(4,4,5,5-tetramethyl-1,3,2-dioxaborolan-2-yl)-1H-pyrazole (intermediate **dd**) (650 mg, white oil, yield: 50%).

LC-MS (ESI): *m/z* 212.23[M+H$^+$]

Preparation Example 30: preparation of 3-bromo-5-(1-(methyl-$d_3$)-1*H*-pyrazol-4-yl)pyridin-2-amine (intermediate **ee**)

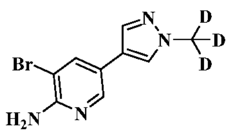

ee

The same procedure as steps 1 and 2 in Preparation Example 15 were performed, except that 1-(methyl-$d_3$)-4-(4,4,5,5-tetramethyl-1,3,2-dioxaborolan-2-yl)-1*H*-pyrazole was used in place of *tert*-butyl 4-(4-(4,4,5,5-tetramethyl-1,3,2-dioxaborolan-2-yl)-1*H*-pyrazol-1-yl)piperidine-1-carboxylate, so as to prepare 3-bromo-5-(1-(methyl-$d_3$)-1*H*-pyrazol-4-yl)pyridin-2-amine (intermediate **ee**) (yellow solid, two-step yield: 35%).

LC-MS (ESI): *m/z* 256.07/258.05[M+H$^+$].

Example 1: preparation of *N*-(4-(2-amino-5-(3,4-dimethoxyphenyl)pyridin-3-yl)-3-fluorophenyl)-6-cyano-5-(4-fluorophenyl)-1-isopropyl-4-oxo-1,4-dihydropyridine-3-carboxamide (**1**)

Step 1: preparation of methyl 5-((4-(2-amino-5-(3,4-dimethoxyphenyl)pyridin-3-yl)-3-fluorophenyl)carbamoyl)-3-(4-fluorophenyl)-1-isopropyl-4-oxoethyl ester-1,4-dihydropyridine-2-carboxylate (**1a**)

6-(ethoxycarbonyl)-5-(4-fluorophenyl)-1-isopropyl-4-oxo-1,4-dihydropyridine-3-carboxylic acid (intermediate (**a**) (625 mg, 1.8 mmol), 3-(4-amino-2-fluorophenyl)-5-(3,4-dimethoxyphenyl)-pyridin-2-amine (**s**) (500 mg, 1.5 mmol), HATU (875 mg, 2.3 mmol) and DIPEA (387 mg, 3.0 mmol) were added to a reaction flask containing DMF (20 mL). The reaction mixture was stirred at room temperature for 30 min. After the reaction was completed, the reaction mixture was added with saturated sodium bicarbonate solution to quench the reaction, and then extracted with ethyl acetate (60 mL × 3). The organic phases were combined, washed with saturated brine, dried over anhydrous sodium sulfate and filtered, and the filtrate was concentrated under reduced pressure. The residue was purified by silica gel column chromatography (eluent: MeOH:DCM = 1:20) to give ethyl 5-((4-(2-amino-5-(3,4-dimethoxyphenyl)pyridin-3-yl)-3-fluorophenyl)carbamoyl)-3-(4-fluorophenyl)1-isopropyl-4-oxo-1,4-dihydropyridine-2-carboxylate (**1a**) (800 mg, yellow solid, yield: 81.2%).

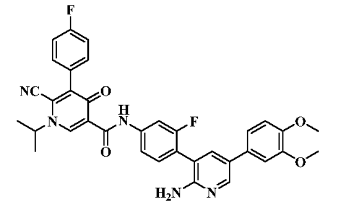

1

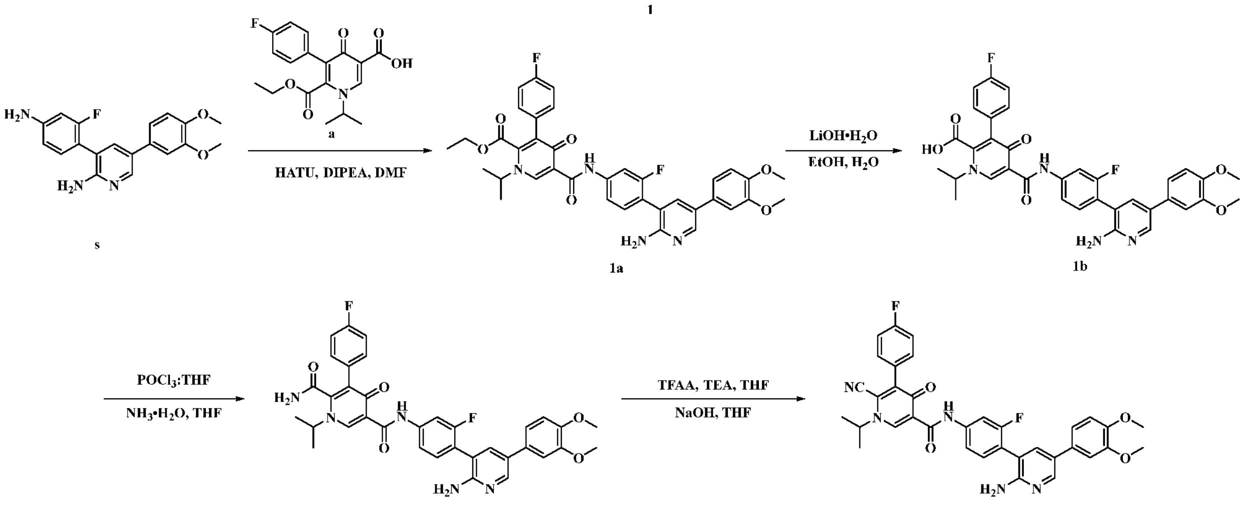


s a 1a 1b 1c 1

LC-MS (ESI): *m/z* 669.28[M+H$^+$].

Step 2: preparation of 5-((4-(2-amino-5-(3,4-dimethoxyphenyl)pyridin-3-yl)-3-fluorophenyl)carbamoyl)-3-(4-fluorophenyl)-1-isopropyl-4-oxo-1,4-dihydropyridine-2-carboxylic acid (**1b**)

Ethyl 5-((4-(2-amino-5-(3,4-dimethoxyphenyl)pyridin-3-yl)-3-fluorophenyl)carbamoyl)-3-(4-fluorophenyl)-1-isopropyl-4-oxo-1,4-dihydropyridine-2-carboxylate (**1a**) (800 mg, 1.2 mmol), lithium hydroxide monohydrate (67 mg, 1.6 mmol), and water (5 mL) were added to a reaction flask containing ethanol (20 mL) at room temperature. The reaction mixture was stirred at 70 °C overnight. After the reaction was completed, the reaction mixture was concentrated under reduced pressure to give crude 5-((4-(2-amino-5-(3,4-dimethoxyphenyl)pyridin-3-yl)-3-fluorophenyl)carbamoyl)-3-(4-fluorophenyl)-1-isopropyl-4-oxo-1,4-dihydropyridine-2-carboxylic acid (**1b**) (yellow solid), which was directly used in the next step without purification.

LC-MS (ESI): *m/z* 641.3[M+H$^+$].

Step 3: preparation of $N^5$-(4-(2-amino-5-(3,4-dimethoxyphenyl)pyridin-3-yl)-3-fluorophenyl)-3-(4-fluorophenyl)-1-isopropyl-4-oxo-1,4-dihydropyridine-2,5-dicarboxamide (**1c**)

5-((4-(2-amino-5-(3,4-dimethoxyphenyl)pyridin-3-yl)-3-fluorophenyl)carbamoyl)-3-(4-fluoroph enyl)-1-isopropyl-4-oxo-1,4-dihydropyridine-2-carboxylic acid (**1b**) (400 mg, 0.6 mmol) was added to a reaction flask containing tetrahydrofuran (8 mL) and phosphorus oxychloride (4 mL) at room temperature. The reaction mixture was heated to reflux for 30 min, and after the reaction was completed, the reaction mixture was concentrated under reduced pressure to give the acyl chloride intermediate, which was directly used in the next step without purification. The acyl chloride intermediate was added dropwise to a reaction flask containing tetrahydrofuran (10 mL) and aqueous ammonia (10 mL) at 0 °C. After the addition was completed, the reaction mixture was stirred for 20 min. After the reaction was completed, ethyl acetate (30 mL) was added to dilute the reaction mixture. The organic phase was washed successively with water and saturated brine, dried over anhydrous sodium sulfate, and filtered, and the filtrate was concentrated under reduced pressure. The residue was purified by silica gel column chromatography (eluent MeOH:DCM = 1:10) to give $N^5$-(4-(2-amino-5-(3,4-dimethoxyphenyl)pyridin-3-yl)-3-fluorophenyl)-3-(4-fluorophenyl)-1-isopropyl-4-oxo-1,4-dihydropyridine-2,5-dicarboxamide (**1c**) (280 mg, yellow solid, yield: 70.2%).
LC-MS (ESI): *m/z* 640.7[M+H$^+$].

Step 4: preparation of *N*-(4-(2-amino-5-(3,4-dimethoxyphenyl)pyridin-3-yl)-3-fluorophenyl)-6-cyano-5-(4-fluorophenyl)-1-isopropyl-4-oxo-1,4-dihydropyridine-3-carboxamide (**1**)

$N^5$-(4-(2-amino-5-(3,4-dimethoxyphenyl)pyridin-3-yl)-3-fluorophenyl)-3-(4-fluorophenyl)-1-iso propyl-4-oxo-1,4-dihydropyridine-2,5-dicarboxamide (**6c**) (100 mg, 0.16 mol) was added to a reaction flask containing anhydrous tetrahydrofuran (4 mL). The reaction mixture was cooled to 0 °C and then added successively with triethylamine (130 mg, 1.3 mmol) and trifluoromethanesulfonic anhydride (126 mg, 0.6 mol). After the addition was completed, the reaction mixture was stirred for 30 min. After the reaction was completed, the reaction mixture was added with aqueous potassium hydroxide solution (5 M, 0.2 mL), stirred for 1 h, and then diluted with ethyl acetate (50 mL). The organic phase was washed successively with water and saturated brine, dried over anhydrous sodium sulfate and filtered, and the filtrate was concentrated under reduced pressure. The residue was purified by preparative HPLC (C18, acetonitrile/water (0.1% formic acid): 10%-100%) to give *N*-(4-(2-amino-5-(3,4-dimethoxyphenyl)pyridin-3-yl)-3-fluorophenyl)-6-cyano-5-(4-fluorophenyl)-1-isopropyl-4-oxo-1,4-dihydropyridine-3-carboxamide (**1**) (30 mg, yellow solid, yield: 31%).
LC-MS (ESI): *m/z* 622.2[M+H$^+$].
$^1$H NMR (400 MHz, DMSO-$d_6$) $\delta$ 12.55 (s, 1H), 8.85 (s, 1H), 8.29 (d, $J$ = 2.5 Hz, 1H), 7.90 (dd, $J$ = 12.2, 2.0 Hz, 1H), 7.64 - 7.58 (m, 3H), 7.48 - 7.36 (m, 4H), 7.18 - 7.10 (m, 2H), 6.97 (d, $J$ = 8.4 Hz, 1H), 5.67 (s, 2H), 4.86 (p, $J$ = 6.6 Hz, 1H), 3.81 (s, 3H), 3.76 (s, 3H), 1.63 (d, $J$ = 6.6 Hz, 6H).

Example 2: preparation of *N*-(4-(2-amino-5-(1-(piperidin-4-yl)-1*H*-pyrazol-4-yl)pyridin-3-yl)-3-fluorophenyl)-6-cyano-5-(4-fluorophenyl)-1-isopropyl-4-oxo-1,4-dihydropyridine-3 - carboxamide (**2**)

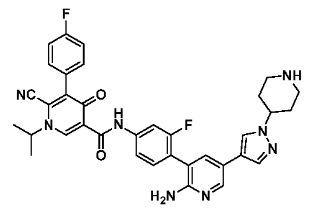

2

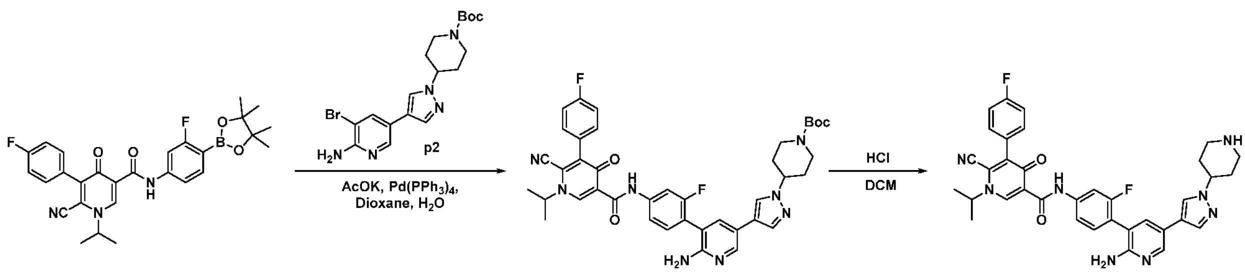


f 2a 2

Step 1: preparation of *tert*-butyl 4-(4-(6-amino-5-(4-(6-cyano-5-(4-fluorophenyl)-1-isopropyl-4-oxo-1,4-dihydropyridine-3-carboxamido)-2-fluorophenyl)pyridin-3-yl)-1*H*-pyrazol-1-yl)piperidine 1-carboxylate (**2a**)

*Tert*-butyl 4-(4-(6-amino-5-bromopyridin-3-yl)-1*H*-pyrazol-1-yl)piperidine-1-carboxylate (**p2**) (260 mg, 0.62 mmol), 6-cyano-*N*-(3-fluoro-4-(4,4,5,5-tetramethyl-1,3,2-dioxaborolan-2-yl)-phenyl)-5-(4-fluorophenyl)-1-isopropyl-4-oxo-1,4-dihydropyridine-3-carboxamide (**f**) (300 mg, 0.62 mmol), potassium carbonate (256 mg, 1.85 mmol) and $Pd(PPh_3)_4$ (285 mg, 0.25 mmol) were added to a reaction flask containing 1,4-dioxane (8 mL) and water (2 mL) at room temperature. The reaction mixture was sealed in the flask, purged with nitrogen three times, heated to 90 °C in a microwave reactor and then stirred for 30 min. After the reaction was completed, the reaction mixture was diluted with water (50 mL) and extracted with ethyl acetate (30 mL × 3). The organic phases were combined, washed with saturated brine, dried over anhydrous sodium sulfate and filtered, and the filtrate was concentrated under reduced pressure. The residue was purified by silica gel column chromatography (eluent: PE:EA = 1:2) to give *tert*-butyl 4-(4-(6-amino-5-(4-(6-cyano-5-(4-fluorophenyl)-1-isopropyl-4-oxo-1,4-dihydropyridine-3-carboxamido)-2-fluorophenyl)pyridin-3-yl)-1*H*-pyrazol-1-yl)piperidine-1-carboxylate (**2a**) (290 mg, yellow solid, yield: 68.3%).

LC-MS (ESI): *m*/*z* 735.4[$M+H^+$].

Step 2: preparation of N-(4-(2-amino-5-(1-(piperidin-4-yl)-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (**2**)

*Tert*-butyl 4-(4-(6-amino-5-(4-(6-cyano-5-(4-fluorophenyl)-1-isopropyl-4-oxo-1,4-dihydropyridine-3-carboxamido)-2-fluorophenyl)pyridin-3-yl)-1*H*-pyrazol-1-yl)piperidine-1-carboxylate (**2a**) (100 mg, 0.14 mmol) and hydrochloric acid (1 mL, 4 M) were added to a reaction flask containing DCM (4 mL) at room temperature. The reaction mixture was stirred for 30 min, and after the reaction was completed, the reaction mixture was concentrated under reduced pressure. The residue was purified by preparative HPLC (C18, acetonitrile/water (0.1% formic acid): 10%-100%) to give *N*-(4-(2-amino-5-(1-(piperidin-4-yl)-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (**2**) (25 mg, yellow solid, yield: 29%).

LC-MS (ESI): *m*/*z* 635.3[$M+H^+$].

$^1H$ NMR (400 MHz, DMSO-$d_6$) $\delta$ 12.55 (s, 1H), 8.84 (s, 1H), 8.26 (d, *J* = 2.3 Hz, 1H), 8.14 (s, 1H), 7.90 (dd, *J* = 12.3, 2.1 Hz, 1H), 7.84 (s, 1H), 7.64 - 7.58 (m, 2H), 7.56 (d, *J* = 2.4 Hz, 1H), 7.46 (dd, *J* = 8.4, 2.1 Hz, 1H), 7.38 (td, *J* = 8.7, 3.5 Hz, 3H), 5.56 (s, 2H), 4.86 (p, *J* = 6.6 Hz, 1H), 4.35 (tt, *J* = 11.0, 4.1 Hz, 1H), 3.24 (s, 2H), 2.92 (td, *J* = 12.5, 3.0 Hz, 2H), 2.16 - 2.09 (m, 2H), 2.01 (qd, *J*= 12.1, 4.2 Hz, 3H), 1.63 (d, *J* = 6.6 Hz, 6H).

Example 3: preparation of *N*-(4-(2-amino-5-(1-ethyl-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (3)

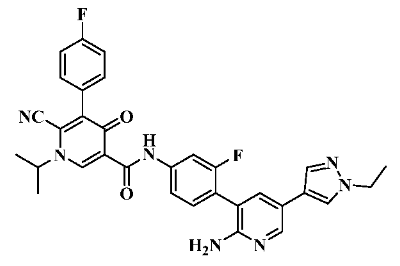

3

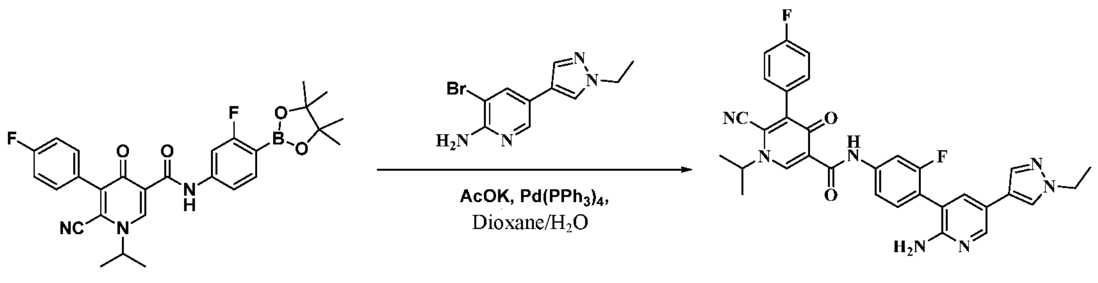


f

3

Step 1: preparation of *N*-(4-(2-amino-5-(1-ethyl-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (**3**)

3-bromo-5-(1-ethyl-1*H*-pyrazol-4-yl)pyridin-2-amine (**v**) (80 mg, 0.3 mmol), 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (**f**) (186 mg, 0.36 mmol), potassium acetate (88 mg, 0.9 mmol), and $Pd(PPh_3)_4$ (69 mg, 0.06 mmol) were added to a reaction flask containing 1,4-dioxane (8 mL) and water (2 mL) at room temperature. The reaction mixture was sealed in the flask, purged with nitrogen three times, heated to 90 °C in a microwave reactor and then stirred for 30 min. After the reaction was completed, the reaction mixture was diluted with water (50 mL) and extracted with ethyl acetate (30 mL × 3). The organic phases were combined, washed with saturated brine, dried over anhydrous sodium sulfate and filtered, and the filtrate was concentrated under reduced pressure. The residue was purified by preparative HPLC (C18, acetonitrile/water (0.1% formic acid): 10%-100%) to give *N*-(4-(2-amino-5-(1-ethyl-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (**3**) (35 mg, yellow solid, yield: 20%).

LC-MS (ESI): *m/z* 580.2[M+H$^+$].

$^1$H NMR (400 MHz, DMSO-$d_6$) $\delta$ 12.55 (s, 1H), 8.84 (s, 1H), 8.24 (d, *J* = 2.4 Hz, 1H), 8.09 (s, 1H), 7.90 (dd, *J* = 12.3, 2.1 Hz, 1H), 7.77 (d, *J* = 0.8 Hz, 1H), 7.63 - 7.58 (m, 2H), 7.52 (d, *J* = 2.4 Hz, 1H), 7.46 (dd, *J*= 8.4, 2.0 Hz, 1H), 7.38 (tt, *J*= 8.5, 2.3 Hz, 3H), 5.54 (s, 2H), 4.86 (p, *J*= 6.6 Hz, 1H), 4.10 (q, *J* = 7.3 Hz, 2H), 1.63 (d, *J* = 6.6 Hz, 6H), 1.38 (t, *J* = 7.3 Hz, 3H).

Example 4: preparation of *N*-(4-(2-amino-5-(1-methyl-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (**4**)

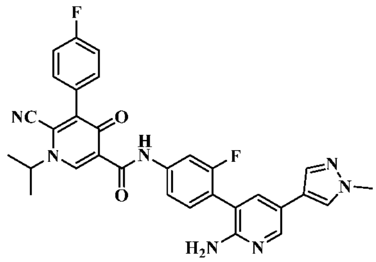

**4**

The same procedures as in Example 3 were performed, except that 3-bromo-5-(1-methyl-1*H*-pyrazol-4-yl)pyridin-2-amine (**w**) was used in place of 3-bromo-5-(1-ethyl-1*H*-pyrazol-4-yl)-pyridin-2-amine (**v**), so as to give *N*-(4-(2-amino-5-(1-methyl-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (**4**) (15 mg, yellow solid, yield: 56%).

LC-MS (ESI): *m/z* 566.23 [M+H$^+$].

$^1$H NMR (400 MHz, DMSO-d6) $\delta$ 12.54(s, 1H),8.84 (s, 1H), 8.23 (d, *J* = 2.4 Hz, 1H), 8.02 (d, *J* = 0.8 Hz, 1H), 7.90 (dd, *J* = 12.3, 2.0 Hz, 1H), 7.77 (d, *J* = 0.8 Hz, 1H), 7.63 - 7.58 (m, 2H), 7.51 (d, *J* = 2.3 Hz, 1H), 7.46 (dd, *J* = 8.4, 2.1 Hz, 1H), 7.38 (td, *J* = 8.7, 2.0 Hz, 3H), 5.55 (s, 2H), 4.88 - 4.83 (m, 1H), 3.82 (s, 3H), 1.63 (d, *J* = 6.6 Hz, 6H).

Example 5: preparation of *N*-(4-(2-amino-5-(1-(tetrahydro-2*H*-pyran-4-yl)-1*H*-pyrazol-4-yl)-pyridin-3-yl)-3-fluorophenyl)-6-cyano-5-(4-fluorophenyl)-1-isopropyl-4-oxo-1,4-dihydropyridine-3-carboxamide (**5**)

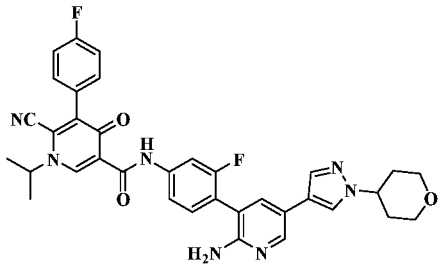

5

The same procedures as in Example 3 were used, except that 3-bromo-5-(1-(tetrahydro-2*H*-pyran-4-yl)-1*H*-pyrazol-4-yl)pyridin-2-amine (**x**) was used in place of 3-bromo-5-(1-ethyl-1*H*-pyrazol-4-yl)pyridin-2-amine (**v**), so as to give *N*-(4-(2-amino-5-(1-(tetrahydro-2*H*-pyran-4-yl)-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (**5**) (22 mg, yellow solid, yield: 42%).

LC-MS (ESI): *m*/*z* 636.29[M+H$^+$].

$^1$H NMR (400 MHz, DMSO-$d_6$) $\delta$ 12.55 (s, 1H), 8.84 (s, 1H), 8.26 (d, *J* = 2.3 Hz, 1H), 8.17 (s, 1H), 7.90 (dd, *J* = 12.2, 2.0 Hz, 1H), 7.81 (s, 1H), 7.64 - 7.58 (m, 2H), 7.55 (d, *J* = 2.3 Hz, 1H), 7.46 (dd, *J* = 8.4, 2.1 Hz, 1H), 7.38 (td, *J* = 8.7, 2.4 Hz, 3H), 5.55 (s, 2H), 4.85 (q, *J* = 6.6 Hz, 1H), 4.39 - 4.32 (m, 1H), 3.99 - 3.92 (m, 2H), 3.48 (dd, *J* = 11.6, 2.6 Hz, 2H), 2.02 - 1.90 (m, 4H), 1.63 (d, *J* = 6.6 Hz, 6H).

Example 6: preparation of *N*-(4-(2-amino-5-(1-methyl-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide (**6**)

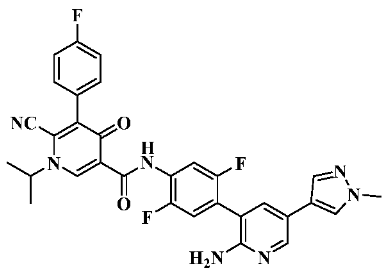

6

The same procedures as in Example 3 were performed, except that 6-cyano-*N*-(2,5-difluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (**y**) was used in place of 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridi ne-3-carboxamide (**f**), and 3-bromo-5-(1-methyl-1*H*-pyrazol-4-yl)pyridin-2-amine (**w**) was used in place of 3-bromo-5-(1-ethyl-1*H*-pyrazol-4-yl)pyridin-2-amine (**v**), so as to give *N*-(4-(2-amino-5-(1-methyl-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide (**6**) (14 mg, yellow solid, yield: 39%).

LC-MS (ESI): *m*/*z* 584.23 [M+H$^+$].

$^1$H NMR (400 MHz, DMSO-$d_6$) $\delta$ 12.86 (s, 1H), 8.86 (s, 1H), 8.42 (dd, *J* = 11.6, 6.6 Hz, 1H), 8.24 (d, *J* = 2.3 Hz, 1H), 8.02 (s, 1H), 7.77 (d, *J* = 0.8 Hz, 1H), 7.66 - 7.58 (m, 2H), 7.54 (d, *J* = 2.4 Hz, 1H), 7.45 - 7.35 (m, 3H), 5.69 (s, 2H), 4.86 (q, *J* = 6.6 Hz, 1H), 3.82 (s, 3H), 1.63 (d, *J* = 6.6 Hz, 6H).

Example 7: preparation of *N*-(4-(2-amino-5-(1-(1-methylpiperidin-4-yl)-1*H*-pyrazol-4-yl)-pyridin-3-yl)-3-fluorophenyl)-6-cyano-5-(4-fluorophenyl)-1-isopropyl-4-oxo-1,4-dihydropyridine-3-carboxamide (**7**)

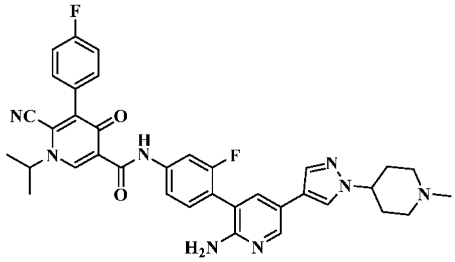

7

The same procedures as in Example 3 were performed, except that 3-bromo-5-(1-(1-methyl-piperidin-4-yl)-1*H*-pyrazol-4-yl)pyridin-2-amine (**z**) was used in place of 3-bromo-5-(1-ethyl-1*H*-pyrazol-4-yl)pyridin-2-amine (**v**), so as to give *N*-(4-(2-amino-5-(1-(1-methylpiperidin-4-yl)-1*H*-pyrazol-4-yl)pyridin-3-yl)-3-fluorophenyl)-6-cyano-5-(4-fluorophenyl)-1-isopropyl-4-oxo-1,4-dihydropyridine-3-carboxamide (**7**) (12 mg, yellow solid, yield: 60%).
LC-MS (ESI): *m/z* 649.3 [M+H$^+$].
$^1$H NMR (400 MHz, DMSO-$d_6$) 12.54 (s, 1H), δ 8.84 (s, 1H), 8.24 (s, 1H), 8.15 (d, *J* = 0.8 Hz, 1H), 7.89 (dd, *J* = 12.2, 2.1 Hz, 1H), 7.79 (d, *J* = 0.7 Hz, 1H), 7.64 - 7.58 (m, 2H), 7.54 (d, *J* = 2.4 Hz, 1H), 7.46 (dd, *J* = 8.4, 2.0 Hz, 1H), 7.38 (td, *J* = 8.7, 3.3 Hz, 3H), 5.54 (s, 2H), 4.87 (q, *J* = 6.6 Hz, 1H), 4.05 (dq, *J* = 10.1, 5.3, 4.7 Hz, 1H), 2.84 (d, *J* = 11.2 Hz, 2H), 2.20 (s, 3H), 1.99 (dtd, *J* = 22.9, 11.4, 8.4 Hz, 6H), 1.63 (d, *J* = 6.6 Hz, 6H).

Example 8: preparation of *N*-(4-(2-amino-5-(1-(piperidin-4-yl)-1*H*-pyrazol-4-yl)pyridin-3-yl)-3-fluorophenyl)-6-cyano-5-(4-fluorophenyl)-1-cyclobutyl-4-oxo-1,4-dihydropyridine-3-carboxamide (**8**)

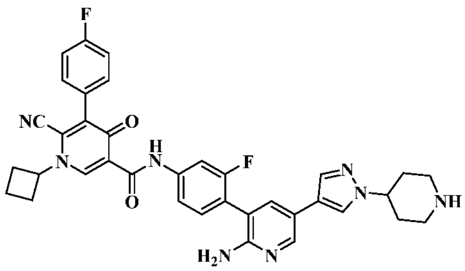

8

The same procedures as in Example 2 were performed, except that 6-cyano-1-cyclobutyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide (**g**) was used in place of 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (**f**), so as to give *N*-(4-(2-amino-5-(1-(piperidin-4-yl)-1*H*-pyrazol-4-yl)-pyridin-3-yl)-3-fluorophenyl)-6-cyano-5-(4-fluorophenyl)-1-cyclobutyl-4-oxo-1,4-dihydropyridine-3-carboxamide (**8**) (30 mg, yellow solid, two-step yield: 43.3%).
LC-MS (ESI): *m/z* 647.34[M+H$^+$].
$^1$H NMR (400 MHz, DMSO-$d_6$) *δ* 12.52 (s, 1H), 8.75 (s, 1H), 8.27 (dd, *J* = 10.5, 2.0 Hz, 2H), 8.14 (s, 1H), 7.90 (dd, *J* = 12.2, 2.0 Hz, 1H), 7.82 (s, 1H), 7.58 (ddd, *J* = 9.8, 6.0, 3.1 Hz, 3H), 7.46 (dd, *J* = 8.4, 2.0 Hz, 1H), 7.38 (td, *J* = 8.7, 3.1 Hz, 3H), 5.57 (s, 2H), 5.03 (q, *J* = 8.7 Hz, 1H), 4.27 (s, 1H), 3.18 (d, *J* = 12.4 Hz, 3H), 2.79 (t, *J* = 12.1 Hz, 2H), 2.69 - 2.53 (m, 4H), 2.06 (d, *J* = 13.0 Hz, 2H), 1.86 (td, *J* = 10.2, 5.2 Hz, 3H).

Example 9: preparation of *N*-(4-(2-amino-5-(1-ethyl-1*H*-pyrazol-4-yl)pyridin-3-yl)-3-fluorophenyl)-6-cyano-5-(4-fluorophenyl)-1-cyclobutyl-4-oxo-1,4-dihydropyridine-3-carboxamide (**9**)

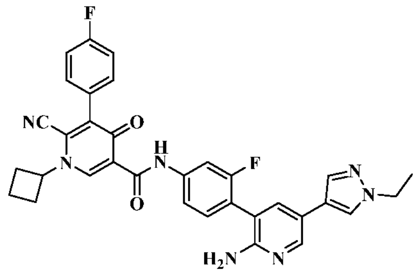

9

The same procedures as in Example 3 were performed, except that 6-cyano-1-cyclobutyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide (**g**) was used in place of 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (**f**), so as to give *N*-(4-(2-amino-5-(1-ethyl-1*H*-pyrazol-4-yl)pyridin-3-yl)-3-fluorophenyl)-6-cyano-5-(4-fluorophenyl)-1-cyclobutyl-4-oxo-1,4-dihydropyridine-3-carboxamide (**9**) (11 mg, yellow solid, yield: 69%).

LC-MS (ESI): *m/z* 592.27[M+H$^+$].

$^1$H NMR (400 MHz, DMSO-$d_6$) $\delta$ 12.52 (s, 1H), 8.75 (s, 1H), 8.24 (d, *J* = 2.3 Hz, 1H), 8.09 (s, 1H), 7.90 (dd, *J* = 12.2, 2.0 Hz, 1H), 7.78 (s, 1H), 7.61 - 7.55 (m, 2H), 7.52 (d, *J* = 2.3 Hz, 1H), 7.46 (dd, *J* = 8.4, 2.0 Hz, 1H), 7.42 - 7.35 (m, 3H), 5.56 (s, 2H), 5.03 (q, *J* = 8.6 Hz, 1H), 4.11 (q, *J* = 7.3 Hz, 2H), 2.67 - 2.57 (m, 3H), 2.02 - 1.96 (m, 1H), 1.87 (dq, *J*= 9.9, 5.3 Hz, 2H), 1.38 (t, *J*= 7.3 Hz, 3H).

Example 10: preparation of *N*-(4-(2-amino-5-(1-ethyl-1*H*-pyrazol-4-yl)pyridin-3-yl)-3-fluorophenyl)-6-cyano-5-(4-fluorophenyl)-1-cyclopropyl-4-oxo-1,4-dihydropyridine-3-carboxamide (**10**)

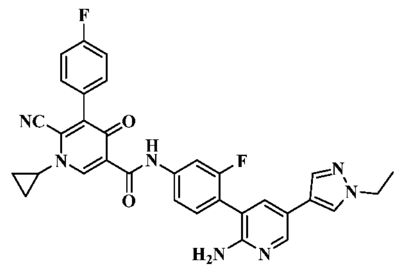

10

The same procedures as in Example 3 were performed, except that 6-cyano-1-cyclopropyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide (**e**) was used in place of 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (**f**), so as to give *N*-(4-(2-amino-5-(1-ethyl-1*H*-pyrazol-4-yl)pyridin-3-yl)-3-fluorophenyl)-6-cyano-5-(4-fluorophenyl)-1-cyclopropyl-4-oxo-1,4-dihydropyridine-3-carboxamide (**10**) (60 mg, yellow solid, yield: 46%).

LC-MS (ESI): *m/z* 578.3 [M+H$^+$].

$^1$H NMR (400 MHz, DMSO-$d_6$) $\delta$ 12.44 (s, 1H), 8.68 (s, 1H), 8.24 (d, *J* = 2.3 Hz, 1H), 8.09 (s, 1H), 7.88 (dd, *J* = 12.2, 2.0 Hz, 1H), 7.78 (s, 1H), 7.58 (ddd, *J* = 8.6, 5.4, 2.5 Hz, 2H), 7.51 (d, *J* = 2.4 Hz, 1H), 7.46 (dd, *J* = 8.4, 2.1 Hz, 1H), 7.38 (td, *J* = 8.6, 6.3 Hz, 3H), 5.56 (s, 2H), 4.10 (q, *J* = 7.2 Hz, 2H), 3.94 (tt, *J* = 7.3, 3.8 Hz, 1H), 2.92 (td, *J* = 12.5, 3.0 Hz, 2H), 1.38 (t, *J* = 7.3 Hz, 4H), 127 - 1.22 (m, 3H).

Example 11: preparation of *N*-(4-(2-amino-5-(1-(piperidin-4-yl)-1*H*-pyrazol-4-yl)pyridin-3-yl)-3-fluorophenyl)-6-cyano-5-(4-fluorophenyl)-1-cyclopropyl-4-oxo-1,4-dihydropyridine-3-carboxamide (**11**)

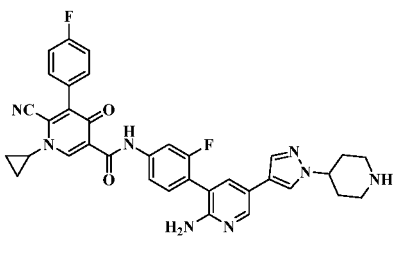

11

The same procedures as in Preparation Example 2 were performed, except that 6-cyano-1-cyclopropyl-*N*-(3-fluoro-4-(4,4,5,5-tetramethyl-1,3,2-dioxaborolan-2-yl)phenyl)-5-(4-fluorophenyl)-4-oxo-1,4-dihydropyridine-3-carboxamide (**e**) was used in place of 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (**f**), so as to give *N*-(4-(2-amino-5-(1-(piperidin-4-yl)-1*H*-pyrazol-4-yl)pyridin-3-yl)-3-fluorophenyl)-6-cyano-5-(4-fluorophenyl)-1-cyclopropyl-4-oxo-1,4-dihydropyridine-3-carboxamide (**11**) (56 mg, yellow solid, two-step yield: 37.6%).

LC-MS (ESI): *m/z* 633.36(M+H$^+$).

$^1$H NMR (400 MHz, DMSO-$d_6$) δ 12.43 (s, 1H), 8.68 (s, 1H), 8.26 (d, *J* = 2.3 Hz, 1H), 8.14 (s, 1H), 7.88 (dd, *J* = 12.2, 2.0 Hz, 1H), 7.83 (s, 1H), 7.61 - 7.54 (m, 3H), 7.46 (dd, *J* = 8.4, 2.0 Hz, 1H), 7.38 (td, *J* = 8.7, 6.8 Hz, 3H), 5.55 (s, 2H), 4.33 (s, 1H), 3.96 - 3.93 (m, 1H), 3.25 (s, 1H), 2.10 (s, 2H), 2.00 (d, *J*= 7.8 Hz, 3H), 1.42 (s, 2H), 1.26 - 1.21 (m, 3H).

Example 12: preparation of *N*-(4-(2-amino-5-(1-(piperidin-4-yl)-1H-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**12**)

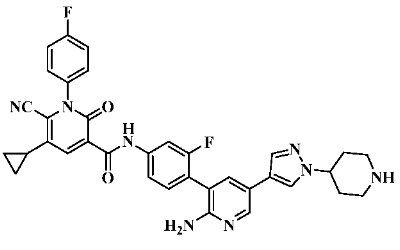

12

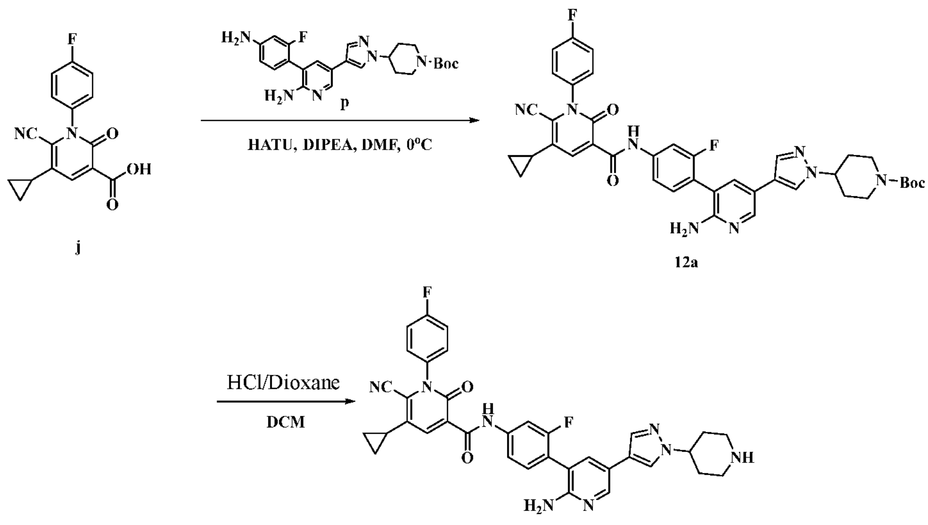


j

12a

12

Step 1: preparation of *tert-butyl* 4-(4-(6-amino-5-(4-(6-cyano-5-cyclopropyl-1-(4-fluorophenyl)-2-oxo-1,2-dihydropyridine-3-carboxamido)-2-fluorophenyl)pyridin-3-yl)-1*H*-pyrazol-1-yl)piperidine-1-carboxylate (**12a**)

6-cyano-5-cyclopropyl-1-(4-fluorophenyl)-2-oxo-1,2-dihydropyridine-3-carboxylic acid (intermediate **j**) (25 mg, 0.084 mmol), *tert*-butyl 4-(4-(6-amino-5-(4-amino-2-fluorophenyl)-pyridin-3-yl)-1*H*-pyrazol-1-yl)piperidine-1-carboxylate (intermediate **p**) (38 mg, 0.084 mmol), HATU (41 mg, 0.11 mmol) and DIPEA (33 mg, 0.25 mmol) were added to a reaction flask containing DMF (4 mL) at 0 °C. The reaction mixture was stirred for 1 h. After the reaction was completed, the reaction mixture was added with saturated sodium bicarbonate solution to quench the reaction, and then extracted with ethyl acetate (10 mL × 3). The organic phases were combined, washed with saturated brine, dried over anhydrous sodium sulfate and filtered, and the filtrate was concentrated under reduced pressure. The residue was purified by silica gel column chromatography (eluent: MeOH:DCM = 1:20) to give *tert*-butyl 4-(4-(6-amino-5-(4-(6-cyano-5-cyclopropyl-1-(4-fluorophenyl)-2-oxo-1,2-dihydropyridine-3-carboxamido)-2-fluorophenyl)-pyridin-3-yl)-1*H*-pyrazol-1-yl)piperidine-1-carboxylate (**12a**) (25 mg, yellow solid, yield: 40.6%).

LC-MS (ESI): *m*/*z* 733.41[M+H$^+$].

Step 2: preparation of *N*-(4-(2-amino-5-(1-(piperidin-4-yl)-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**12**)

*Tert*-butyl 4-(4-(6-amino-5-(4-(6-cyano-5-cyclopropyl-1-(4-fluorophenyl)-2-oxo-1,2-dihydropyridine-3-carboxamido)-2-fluorophenyl)pyridin-3-yl)-1*H*-pyrazol-1-yl)piperidine-1-carboxylate (**12a**) (25 mg, 0.034 mmol) and a solution of hydrochloric acid in dioxane (4 N, 1.5 mL) were added to a reaction flask containing DCM (3 mL). The reaction mixture was stirred at room temperature for 30 min. After the reaction was completed, the reaction mixture was concentrated under reduced pressure, and the residue was purified by preparative HPLC (C18, acetonitrile/water (0.1% formic acid): 10%-100%) to give *N*-(4-(2-amino-5-(1-(piperidin-4-yl)-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**12**) (10 mg, yellow solid, yield: 50.6%).

LC-MS (ESI): *m*/*z* 633.37(M+H$^+$].

$^1$H NMR (400 MHz, DMSO-$d_6$) δ 11.93 (s, 1H), 9.06 (d, *J* = 11.4 Hz, 1H), 8.89 (d, *J* = 10.7 Hz, 1H), 8.37 (d, *J* = 2.5 Hz, 2H), 8.19 (s, 1H), 8.12 (s, 1H), 8.03 (s, 1H), 7.96 (dd, *J* = 12.2, 2.1 Hz, 1H), 7.74 - 7.69 (m, 2H), 7.60 - 7.47 (m, 5H), 4.50 - 4.44 (m, 1H), 3.08 (d, *J* = 11.9 Hz, 2H), 2.28 - 2.05 (m, 6H), 2.04 - 1.95 (m, 1H), 1.19 - 1.14 (m, 2H), 0.93 (dt, *J* = 7.0, 3.3 Hz, 2H).

Example 13: preparation of *N*-(4-(2-amino-5-(1-(1-methylpiperidin-4-yl)-1*H*-pyrazol-4-yl)-pyridin-3-yl)-3-fluorophenyl)-6-cyano-5-cyclopropyl-1-(4-fluorophenyl)-2-oxo-1,2-dihydropyridine-3-carboxamide (**13**)

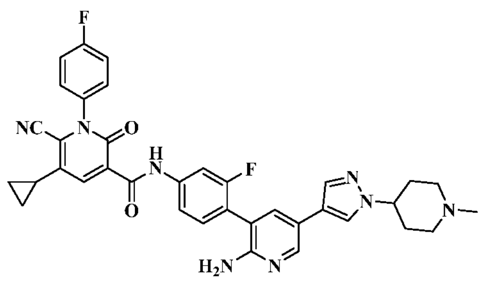

**13**

The same procedures as in Example 12 were performed, except that 3-(4-amino-2-fluorophenyl)-5-(1-(1-methylpiperidin-4-yl)-1*H*-pyrazol-4-yl)pyridin-2-amine (intermediate **u**) was used in place of *tert*-butyl 4-(4-(6-amino-5-(4-amino-2-fluorophenyl)pyridin-3-yl)-1*H*-pyrazol-1-yl)piperidine-1-carboxylate (intermediate **p**), so as to prepare *N*-(4-(2-amino-5-(1-(1-methyl-piperidin-4-yl)-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**13**) (yellow solid, one-step yield: 18.4%).

LC-MS (ESI): *m*/*z* 647.34[M+H$^+$].

$^1$H NMR (400 MHz, DMSO-$d_6$) δ11.85 (s, 1H), 8.25 (d, *J* = 2.4 Hz, 1H), 8.15 (s, 1H), 8.11 (s, 1H), 7.87 (dd, *J* = 12.1, 2.1 Hz, 1H), 7.79 (s, 1H), 7.74 - 7.69 (m, 2H), 7.56 - 7.46 (m, 4H), 7.38 (t, *J* = 8.4 Hz, 1H), 5.53 (s, 2H), 4.06 (dt, *J* = 10.7, 5.7 Hz, 1H), 2.84 (d, *J* = 11.0 Hz, 2H), 2.20 (s, 3H), 2.09 - 1.91 (m, 7H), 1.16 (dt, *J* = 8.3, 3.3 Hz, 2H), 0.94 (dt, *J* = 6.9, 4.8 Hz, 2H).

Example 14: preparation of *N*-(4-(2-amino-5-(1-ethyl-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**14**)

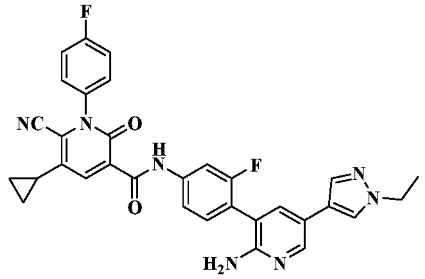

**14**

The same procedures as in Example 12 were performed, except that 3-(4-amino-2-fluorophenyl)-5-(1-ethyl-1*H*-pyrazol-4-yl)pyridin-2-amine (intermediate **q**) was used in place of *tert-butyl* 4-(4-(6-amino-5-(4-amino-2-fluorophenyl)pyridin-3-yl)-1*H*-pyrazol-1-yl)piperidine-1-carboxylate (intermediate **p**), so as to give *N*-(4-(2-amino-5-(1-ethyl-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**14**) (yellow solid, one-step yield: 20.3%).

LC-MS (ESI): *m/z* 578.36[M+H$^+$].

$^1$H NMR (400 MHz, DMSO-$d_6$) δ11.85 (s, 1H), 8.24 (d, *J* = 2.3 Hz, 1H), 8.11 (s, 1H), 8.08 (s, 1H), 7.87 (dd, *J* = 12.2, 2.1 Hz, 1H), 7.77 (s, 1H), 7.74 - 7.69 (m, 2H), 7.50 (ddd, *J* = 10.6, 5.9, 2.5 Hz, 4H), 7.38 (t, *J* = 8.4 Hz, 1H), 5.54 (s, 2H), 4.10 (q, *J* = 7.3 Hz, 2H), 2.10 (td, *J* = 8.3, 4.2 Hz, 1H), 1.38 (t, *J* = 7.3 Hz, 3H), 1.21 - 1.14 (m, 2H), 0.98 - 0.92 (m, 2H).

Example 15: preparation of *N*-(4-(2-amino-5-(1-methyl-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**15**)

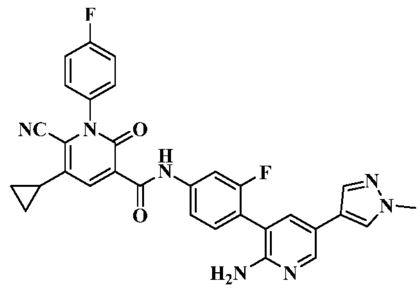

**15**

The same procedures as in Example 12 were performed, except that 3-(4-amino-2-fluorophenyl)-5-(1-methyl-1*H*-pyrazol-4-yl)pyridin-2-amine (intermediate **t**) was used in place of *tert*-butyl 4-(4-(6-amino-5-(4-amino-2-fluorophenyl)pyridin-3-yl)-1*H*-pyrazol-1-yl)piperidine-1-carboxylate (intermediate **p**), so as to give *N*-(4-(2-amino-5-(1-methyl-1*H*-pyrazol-4-yl)-pyridin-3-yl)-3-fluorophenyl)-6-cyano-5-cyclopropyl-1-(4-fluorophenyl)-2-oxo-1,2-dihydropyridine-3-carboxamide (**15**) (yellow solid, one-step yield: 22.4%).

LC-MS (ESI): *m/z* 564.37[M+H$^+$].

$^1$H NMR (400 MHz, DMSO-$d_6$) δ 11.85 (s, 1H), 8.23 (d, *J* = 2.3 Hz, 1H), 8.11 (s, 1H), 8.02 (s, 1H), 7.87 (dd, *J* = 12.2, 2.1 Hz, 1H), 7.76 (s, 1H), 7.74 - 7.68 (m, 2H), 7.50 (td, *J* = 7.8, 7.0, 2.2 Hz, 4H), 7.38 (t, *J* = 8.4 Hz, 1H), 5.54 (s, 2H), 3.82 (s, 3H), 2.10 (ddd, *J* = 8.3, 5.1, 3.3 Hz, 1H), 1.20 - 1.13 (m, 2H), 0.94 (dt, *J* = 6.9, 4.8 Hz, 2H).

Example 16: preparation of *N*-(4-(2-amino-5-(1-(tetrahydro-2*H*-pyran-4-yl)-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**16**)

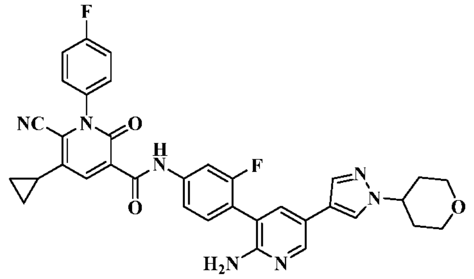

16

The same procedures as in Example 12 were performed, except that 3-(4-amino-2-fluorophenyl)-5-(1-(tetrahydro-2*H*-pyran-4-yl)-1*H*-pyrazol-4-yl)pyridin-2-amine (intermediate **r**) was used in place of *tert*-butyl 4-(4-(6-amino-5-(4-amino-2-fluorophenyl)pyridin-3-yl)-1*H*-pyrazol-1-yl)piperidine-1-carboxylate (intermediate **p**), so as to give *N*-(4-(2-amino-5-(1-(tetrahydro-2*H*-pyran-4-yl)-1*H*-pyrazol-4-yl)pyridin-3-yl)-3-fluorophenyl)-6-cyano-5-cyclopropyl-1-(4-fluorophenyl)-2-oxo-1,2-dihydropyridine-3-carboxamide (**16**) (yellow solid, one-step yield: 17.8%).

LC-MS (ESI): *m/z* 634.41[M+H$^+$].

$^1$H NMR (400 MHz, DMSO-$d_6$) δ 11.85 (s, 1H), 8.25 (d, *J*= 2.4 Hz, 1H), 8.17 (s, 1H), 8.11 (s, 1H), 7.87 (dd, *J* = 12.2, 2.1 Hz, 1H), 7.80 (s, 1H), 7.74 - 7.69 (m, 2H), 7.55 - 7.47 (m, 4H), 7.38 (t, *J* = 8.4 Hz, 1H), 5.54 (s, 2H), 4.34 (dt, *J* = 10.9, 4.7 Hz, 1H), 3.98 - 3.92 (m, 2H), 3.48 (dd, *J* = 11.6, 2.6 Hz, 2H), 2.12 - 2.06 (m, 1H), 1.96 (td, *J*= 12.7, 8.0 Hz, 4H), 1.20 - 1.14 (m, 2H), 0.97 - 0.92 (m, 2H).

Example 17: preparation of *N*-(4-(2-amino-5-(1-(piperidin-4-yl)-1*H*-pyrazol-4-yl)pyridin-3-yl)-3-fluorophenyl)-6-cyano-1-(4-fluorophenyl)-2-oxo-5-(prop-1-en-2-yl)-1,2-dihydropyridine-3-ca rboxamide (**17**)

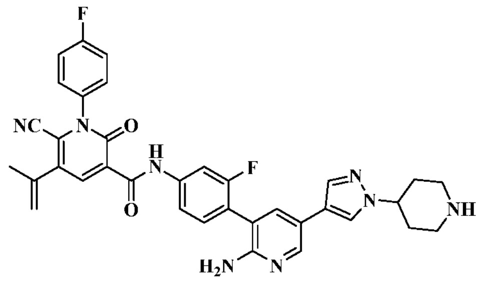

17

The same procedures as in Example 12 were performed, except that 6-cyano-1-(4-fluorophenyl)-2-oxo-5-(prop-1-en-2-yl)-1,2-dihydropyridine-3-carboxylic acid (intermediate **k**) was used in place of 6-cyano-5-cyclopropyl-1-(4-fluorophenyl)-2-oxo-1,2-dihydropyridine-3-carboxylic acid (intermediate **j**), so as to give *N*-(4-(2-amino-5-(1-(piperidin-4-yl)-1*H*-pyrazol-4-yl)pyridin-3-yl)-3-fluorophenyl)-6-cyano-1-(4-fluorophenyl)-2-oxo-5-(prop-1-en-2-yl)-1,2-dihydropyridine-3-carboxamide (**17**) (yellow solid, two-step yield: 9.3%).

LC-MS (ESI): *m/z* 633.38(M+H$^+$].

$^1$H NMR (400 MHz, DMSO-$d_6$) δ 11.78 (s, 1H), 8.54 (s, 1H), 8.26 (d, *J* = 2.3 Hz, 1H), 8.13 (s, 1H), 7.89 (dd, *J*= 12.2, 2.1 Hz, 1H), 7.81 (s, 1H), 7.76 - 7.71 (m, 2H), 7.55 - 7.47 (m, 4H), 7.39 (t, *J* = 8.4 Hz, 1H), 5.55 (s, 2H), 5.51 (t, *J*= 1.6 Hz, 1H), 5.43 (s, 1H), 4.21-4.27 (m, 1H),3.15 (d, *J*= 12.2 Hz, 2H), 2.76 (d, *J*= 12.4 Hz, 2H), 2.18 (s, 3H), 2.02 (t, *J*= 13.8 Hz, 3H), 1.90 (t, *J* = 11.7 Hz, 2H).

Example 18: preparation of *N*-(4-(2-amino-5-(1-ethyl-1*H*-pyrazol-4-yl)pyridin-3-yl)-3-fluorophenyl)-6-cyano-1-(4-fluorophenyl)-5-methyl-2-oxo-1,2-dihydropyridine-3-carboxamide (**18**)

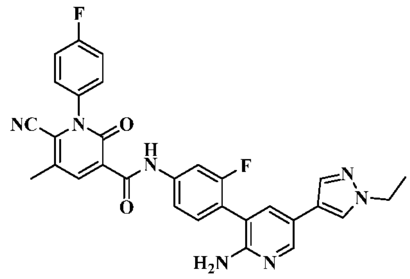

18

The same procedures as in Example 14 were performed, except that 6-cyano-1-(4-fluorophenyl)-5-methyl-2-oxo-1,2-dihydropyridine-3-carboxylic acid (intermediate **i**) was used in place of 6-cyano-5-cyclopropyl-1-(4-fluorophenyl)-2-oxo-1,2-dihydropyridine-3-carboxylic acid (intermediate **j**), so as to give *N*-(4-(2-amino-5-(1-ethyl-1*H*-pyrazol-4-yl)pyridin-3-yl)-3-fluorophenyl)-6-cyano-1-(4-fluorophenyl)-5-methyl-2-oxo-1,2-dihydropyridine-3-carboxamide (**18**) (yellow solid, one-step yield: 30.1%).

LC-MS (ESI): *m/z* 552.33[M+H$^+$],

$^1$H NMR (400 MHz, DMSO-$d_6$) δ 11.89 (s, 1H), 8.61 (s, 1H), 8.24 (d, *J* = 2.3 Hz, 1H), 8.08 (s, 1H), 7.88 (dd, *J*= 12.2, 2.1 Hz, 1H), 7.77 (d, *J*= 0.8 Hz, 1H), 7.72 - 7.67 (m, 2H), 7.54 - 7.46 (m, 4H), 7.39 (t, *J* = 8.4 Hz, 1H), 5.54 (s, 2H), 4.10 (q, *J* = 7.3 Hz, 2H), 2.45 (s, 3H), 1.38 (t, *J* = 7.3 Hz, 3H).

Example 19: preparation of *N*-(4-(2-amino-5-(1-ethyl-1*H*-pyrazol-4-yl)pyridin-3-yl)-3-fluorophenyl)-5-bromo-1-(4-fluorophenyl)-6-((methylamino)methyl)-2-oxo-1,2-dihydropyridine-3-ca rboxamide (**19**)

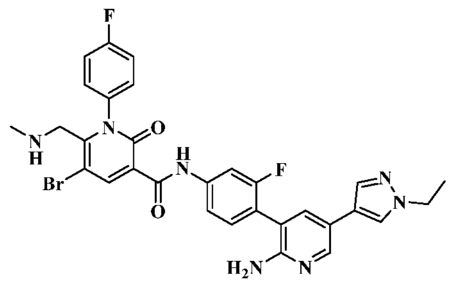

19

The same procedures as in Example 12 were performed, except that 5-bromo-6-(((*tert*-butoxycarbonyl)(methyl)amino)methyl)-1-(4-fluorophenyl)-2-oxo-1,2-dihydropyridine-3 - carboxylic acid (intermediate **l**) was used in place of 6-cyano-5-cyclopropyl-1-(4-fluorophenyl)-2-oxo-1,2-dihydropyridine-3-carboxylic acid (intermediate **j**), and 3-(4-amino-2-fluorophenyl)-5-(1-ethyl-1*H*-pyrazol-4-yl)pyridin-2-amine (intermediate **q**) was used in place of *tert-butyl* 4-(4-(6-amino-5-(4-amino-2-fluorophenyl)pyridin-3-yl)-1*H*-pyrazol-1-yl)piperidine-1-carboxylate (intermediate **p**), so as to give *N*-(4-(2-amino-5-(1-ethyl-1*H*-pyrazol-4-yl)pyridin-3-yl)-3-fluorophenyl)-5-bromo-1-(4-fluorophenyl)-6-((methylamino)methyl)-2-oxo-1,2-dihydropyridine-3-carboxamide (**19**) (yellow solid, two-step yield: 40.2%).

LC-MS (ESI): *m/z* 634.25/635.21[M+H$^+$].

$^1$H NMR (400 MHz, DMSO-$d_6$) δ 11.85 (s, 1H), 8.58 (s, 1H), 8.23 (d, *J* = 2.4 Hz, 1H), 8.08 (s, 1H), 7.87 (dd, *J*= 12.2, 2.1 Hz, 1H), 7.77 (s, 1H), 7.55 (ddt, *J*= 8.0, 5.1, 2.6 Hz, 2H), 7.52 - 7.43 (m, 4H), 7.36 (t, *J*= 8.4 Hz, 1H), 5.53 (s, 2H), 4.09 (t, *J*= 7.3 Hz, 2H), 2.09 (s, 3H), 1.38 (t, *J*= 7.3 Hz, 3H).

Example 20: preparation of *N*-(4-(2-amino-5-(1-(piperidin-4-yl)-1*H*-pyrazol-4-yl)pyridin-3-yl)-3-fluorophenyl)-3-(4-fluorophenyl)-1-isopropyl-2,4-dioxo-1,2,3,4-tetrahydropyrimidine-5-carboxamide (**20**)

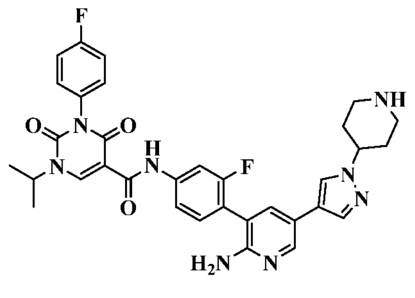

20

The same procedures as in Example 2 were performed, except that *N*-(3-fluoro-4-(4,4,5,5-tetramethyl-1,3,2-dioxaborolan-2-yl)phenyl)-3-(4-fluorophenyl)-1-isopropyl-2,4-dioxo-1,2 ,3,4-tetrahydropyrimidine-5-carboxamide (**bb**) was used in place of 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide (**f**), so as to give *N*-(4-(2-amino-5-(1-(piperidin-4-yl)-1*H*-pyrazol-4-yl)pyridin-3-yl)-3-fluorophenyl)-3-(4-fluorophenyl)-1-isopropyl-2,4-dioxo-1,2,3,4-tetrahydropyrimidine-5-carboxamide (**20**) (66 mg, white solid, two-step yield: 37%).

LC-MS (ESI): *m*/*z* 627.2(M+H$^+$).

$^1$H NMR (400 MHz, DMSO-$d_6$) δ 11.05 (s, 1H), 8.69 (s, 1H), 8.25 (d, *J* = 2.3 Hz, 1H), 8.13 (s, 1H), 7.86 (dd, *J* = 12.3, 2.1 Hz, 1H), 7.80 (s, 1H), 7.54 (d, *J* = 2.4 Hz, 1H), 7.48 - 7.41 (m, 3H), 7.36 (ddd, *J* = 8.9, 6.6, 2.5 Hz, 3H), 5.53 (s, 2H), 4.78 (p, *J* = 6.8 Hz, 1H), 4.26 - 4.21 (m, 1H), 3.16 - 3.11 (m, 2H), 2.72 (td, *J*= 12.4, 2.6 Hz, 3H), 2.06 - 1.99 (m, 2H), 1.90 - 1.82 (m, 2H), 1.43 (d, *J*= 6.8 Hz, 6H).

Example 21: preparation of 6-cyano-*N*-(4-((6,7-dimethoxyquinolin-4-yl)oxy)-3-fluorophenyl)-5-(4-fluorophenyl)-1-methyl-4-oxo-1,4-dihydropyridine-3-carboxamide (**21**)

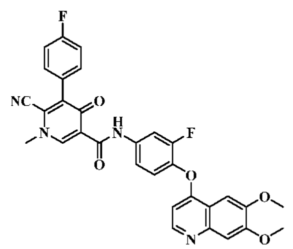

21

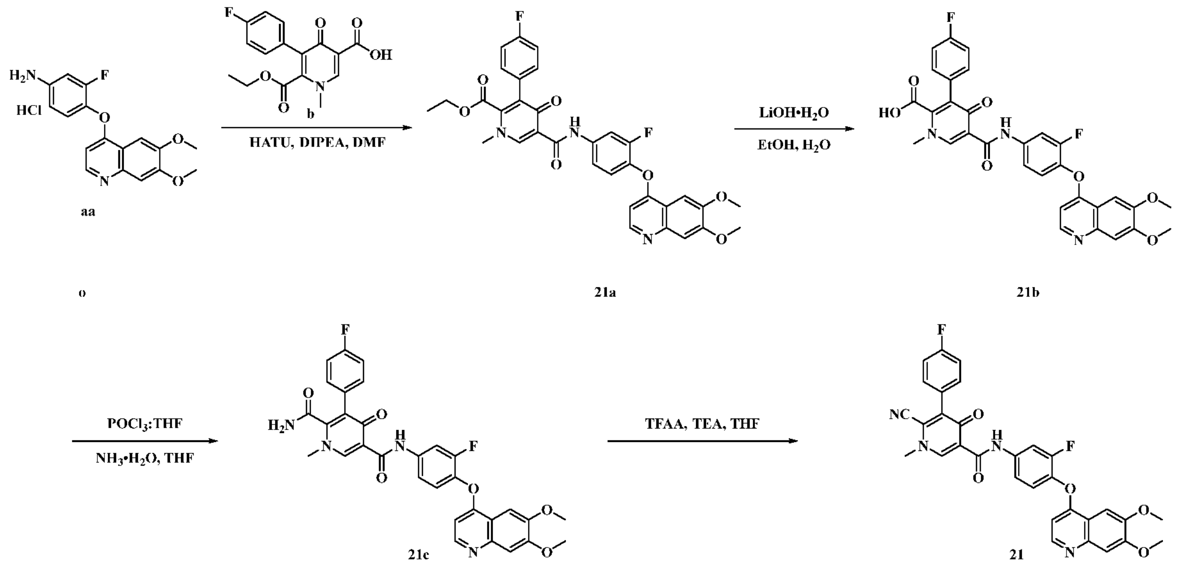


aa b o 21a 21b 21c 21

Step 1: preparation of ethyl 5-((4-((6,7-dimethoxyquinolin-4-yl)oxy)-3-fluorophenyl)-carbamoyl)-3-(4-fluorophenyl)-1-methyl-4-oxo-1,4-dihydropyridine-2-carboxylate (**21a**)

6-(ethoxycarbonyl)-5-(4-fluorophenyl)-1-methyl-4-oxo-1,4-dihydropyridine-3-carboxylic acid (**b**) (800 mg, 2.51 mmol), 4-((6,7-dimethoxyquinolin-4-yl)oxy)-3-fluoroaniline (**aa**) (790 mg, 2.51 mmol), HATU (1.43 g, 3.77 mmol) and DIPEA (973 mg, 7.53 mmol) were added to a reaction flask containing DMF (30 mL). The reaction mixture was stirred at room temperature for 30 min. After the reaction was completed, the reaction mixture was added with saturated sodium bicarbonate solution to quench the reaction, and then extracted with ethyl acetate (30 mL × 3). The organic phases were combined, washed with saturated brine, dried over anhydrous sodium sulfate and filtered, and the filtrate was concentrated under reduced pressure. The residue was purified by silica gel column chromatography (eluent: MeOH:DCM = 1:20) to give ethyl 5-((4-((6,7-dimethoxyquinolin-4-yl)oxy)-3-fluorophenyl)carbamoyl)-3-(4-fluorophenyl)-1-methyl-4-oxo-1,4-dihydropyridine-2-carboxylate (**21a**) (1.4 g, yellow solid, yield: 90.4%).
LC-MS (ESI): *m*/*z* 616.3[M+H$^+$].

Step 2: preparation of 5-((4-((6,7-dimethoxyquinolin-4-yl)oxy)-3-fluorophenyl)carbamoyl)-3-(4-fluorophenyl)-1-methyl-4-oxo-1,4-dihydropyridine-2-carboxylic acid (**21b**)

Ethyl 5-((4-((6,7-dimethoxyquinolin-4-yl)oxy)-3-fluorophenyl)carbamoyl)-3-(4-fluorophenyl)-1-methyl-4-oxo-1,4-dihydropyridine-2-carboxylate (**21a**) (1.4 g, 2.27 mmol), lithium hydroxide monohydrate (150 mg, 3.57 mmol) and water (4 mL) were added to a reaction flask containing ethanol (16 mL) at room temperature. The reaction mixture was warmed to 70 °C and stirred for 2 h. After the reaction was completed, the reaction mixture was concentrated under reduced pressure to give crude 5-((4-((6,7-dimethoxyquinolin-4-yl)oxy)-3-fluorophenyl)-carbamoyl)-3-(4-fluorophenyl)-1-methyl-4-oxo-1,4-dihydropyridine-2-carboxylic acid (**21b**) (1.4 g, grey solid), which was directly used in the next step without purification.
LC-MS (ESI): *m*/*z* 588.3[M+H$^+$].

Step 3: preparation of $N^5$-(4-((6,7-dimethoxyquinolin-4-yl)oxy)-3 -fluorophenyl)-3 -(4-fluorophenyl)-1-methyl-4-oxo-1,4-dihydropyridine-2,5-dicarboxamide (**21c**)

5-((4-((6,7-dimethoxyquinolin-4-yl)oxy)-3-fluorophenyl)carbamoyl)-3-(4-fluorophenyl)-1-methyl-4-oxo-1,4-dihydropyridine-2-carboxylic acid (**21b**) (200 mg, 0.3 mmol) was added to a reaction flask containing tetrahydrofuran (8 mL) and phosphorus oxychloride (4 mL) at room temperature. The reaction mixture was heated to reflux for 30 min, and after the reaction was completed, the reaction mixture was concentrated under reduced pressure to give the acyl chloride intermediate, which was directly used in the next step without purification.
The acyl chloride intermediate was added dropwise to a reaction flask containing tetrahydrofuran (10 mL) and aqueous ammonia (10 mL) at 0 °C. After the addition was completed, the reaction mixture was stirred for 20 min. After the reaction was completed, ethyl acetate (50 mL) was added to dilute the reaction mixture. The organic phase was washed successively with water and saturated brine, dried over anhydrous sodium sulfate, and filtered, and the filtrate was concentrated under reduced pressure. The residue was purified by silica gel column chromatography (eluent: MeOH:DCM = 1:10) to give $N^5$-(4-((6,7-dimethoxyquinolin-4-yl)oxy)-3-fluorophenyl)-3-(4-fluorophenyl)-1-methyl-4-oxo-1,4-dihydropyridine-2,5-dicarboxamide (**21c**) (118 mg, yellow solid, yield: 59.1%).
LC-MS (ESI): *m*/*z* 587.2[M+H$^+$].

Step 4: preparation of 6-cyano-*N*-(4-((6,7-dimethoxyquinolin-4-yl)oxy)-3-fluorophenyl)-5-(4-fluorophenyl)-1-methyl-4-oxo-1,4-dihydropyridine-3-carboxamide (**21**)

$N^5$-(4-((6,7-dimethoxyquinolin-4-yl)oxy)-3-fluorophenyl)-3-(4-fluorophenyl)-1-methyl-4-oxo-1,4-dihydropyridine-2,5-dicarboxamide (**21c**) (54 mg, 0.1 mol) was added to a reaction flask containing anhydrous tetrahydrofuran (4 mL). The reaction mixture was cooled to 0 °C and then added successively with triethylamine (81 mg, 0.8 mmol) and trifluoromethanesulfonic anhydride (84 mg, 0.4 mol). After the addition was completed, the reaction mixture was stirred for 30 min. After the reaction was completed, ethyl acetate (30 mL) was added to dilute the reaction mixture. The organic phase was washed successively with water and saturated brine, dried over anhydrous sodium sulfate, left to stand overnight and then filtered, and the filtrate was concentrated under reduced pressure. The residue was purified by preparative HPLC (C18, acetonitrile/water (0.1% formic acid): 10%-100%) to give 6-cyano-*N*-(4-((6,7-dimethoxyquinolin-4-yl)oxy)-3-fluorophenyl)-5-(4-fluorophenyl)-1-methyl-4-oxo-1,4-dihydropyridine-3-carboxamide (**21**) (18.4 mg, yellow solid, yield: 35.2%).
LC-MS (ESI): *m*/*z* 569.2[M+H$^+$].
$^1$H NMR (400 MHz, DMSO-$d_6$) $\delta$ 12.58 (s, 1H), 8.98 (s, 1H), 8.48 (d, *J*= 5.2 Hz, 1H), 8.34 (s, 1H), 8.04 (dd, *J*

= 12.9, 2.4 Hz, 1H), 7.60 - 7.54 (m, 2H), 7.53 (d, *J* = 2.5 Hz, 1H), 7.46 (t, *J* = 8.9 Hz, 1H), 7.42 - 7.36 (m, 3H), 6.48 (dd, *J*= 5.2, 1.0 Hz, 1H), 4.09 (s, 3H),3.95 (d, 6H).

Example 22: preparation of 6-cyano-*N*-(4-((6,7-dimethoxyquinolin-4-yl)oxy)-3-fluorophenyl)-5-(4-fluorophenyl)-1-cyclopropyl-4-oxo-1,4-dihydropyridine-3-carboxamide (**22**)

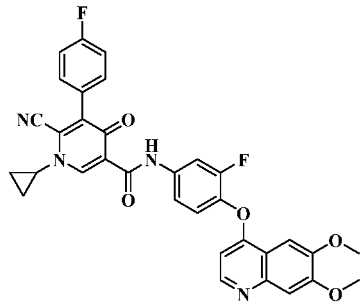

22

The same procedures as in Example 21 were performed, except that 1-cyclopropyl-6-(ethoxycarbonyl)-5-(4-fluorophenyl)-4-oxo-1,4-dihydropyridine-3-carboxylic acid (**d**) was used in place of 6-(ethoxycarbonyl)-5-(4-fluorophenyl)-1-methyl-4-oxo-1,4-dihydropyridine-3-carboxylic acid (**b**), so as to give 6-cyano-*N*-(4-((6,7-dimethoxyquinolin-4-yl)oxy)-3-fluorophenyl)-5-(4-fluorophenyl)-1-cyclopropyl-4-oxo-1,4-dihydropyridine-3-carboxamide (**22**) (38 mg, yellow solid, four-step yield: 18%).

LC-MS (ESI): *m*/*z* 595.2[M+H$^+$].

$^1$H NMR (400 MHz, DMSO-$d_6$) δ 12.43 (s, 1H), 8.67 (s, 1H), 8.48 (d, *J*= 5.2 Hz, 1H), 8.04 (dd, *J* = 12.8, 2.4 Hz, 1H), 7.63 - 7.50 (m, 4H), 7.49 - 7.35 (m, 4H), 6.48 (d, *J*= 5.2 Hz, 1H), 3.95 (d, 6H), 3.48 (d, *J* = 11.0 Hz, 1H), 1.41 (d, *J*= 3.9 Hz, 2H), 1.25 (s, 2H).

Example 23: preparation of 6-cyano-*N*-(4-((6,7-dimethoxyquinolin-4-yl)oxy)-3-fluorophenyl)-5-(4-fluorophenyl)-1-cyclobutyl-4-oxo-1,4-dihydropyridine-3-carboxamide (**23**)

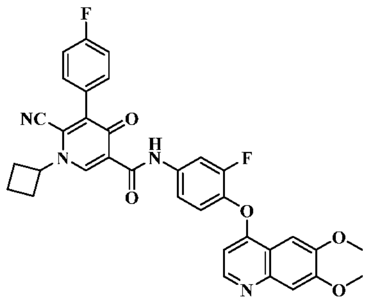

23

The same procedures as in Example 21 were performed, except that 1-cyclobutyl-6-(ethoxycarbonyl)-5-(4-fluorophenyl)-4-oxo-1,4-dihydropyridine-3-carboxylic acid (**c**) was used in place of 6-(ethoxycarbonyl)-5-(4-fluorophenyl)-1-methyl-4-oxo-1,4-dihydropyridine-3-carboxylic acid (**b**), so as to give 6-cyano-*N*-(4-((6,7-dimethoxyquinolin-4-yl)oxy)-3-fluorophenyl)-5-(4-fluorophenyl)-1-cyclobutyl-4-oxo-1,4-dihydropyridine-3-carboxamide (**23**) (25 mg, yellow solid, four-step yield: 21%).

LC-MS (ESI): *m*/*z* 609.2[M+H$^+$].

$^1$H NMR (400 MHz, DMSO-$d_6$) δ 12.52 (s, 1H), 8.74 (s, 1H), 8.48 (d, *J*= 5.2 Hz, 1H), 8.06 (dd, *J* = 12.9, 2.4 Hz, 1H), 7.61 - 7.51 (m, 4H), 7.46 (t, *J* = 8.9 Hz, 1H), 7.43 - 7.35 (m, 3H), 6.49 (d, *J* = 5.4 Hz, 1H), 5.03 (q, *J*= 8.4 Hz, 1H), 3.95 (d, *J*= 1.8 Hz, 6H), 2.68 - 2.53 (m, 4H), 1.87 (dq, *J*= 9.9, 5.3 Hz, 2H).

Example 24: preparation of 6-cyano-*N*-(4-((6,7-dimethoxyquinolin-4-yl)oxy)-3-fluorophenyl)-5-(4-fluorophenyl)-1-isopropyl-4-oxo-1,4-dihydropyridine-3-carboxamide (**24**)

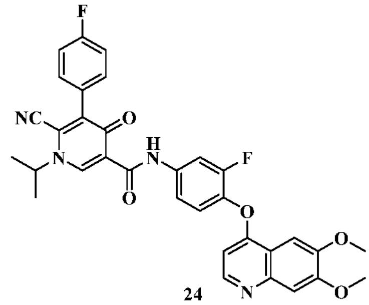

24

The same procedures as in Example 21 were performed, except that 6-(ethoxycarbonyl)-5-(4-fluorophenyl)-1-isopropyl-4-oxo-1,4-dihydropyridine-3-carboxylic acid (**a**) was used in place of 6-(ethoxycarbonyl)-5-(4-fluorophenyl)-1-methyl-4-oxo-1,4-dihydropyridine-3-carboxylic acid (**b**), so as to give 6-cyano-*N*-(4-((6,7-dimethoxyquinolin-4-yl)oxy)-3-fluorophenyl)-5-(4-fluorophenyl)-1-isopropyl-4-oxo-1,4-dihydropyridine-3-carboxamide (**24**) (38 mg, yellow solid, four-step yield: 23%).

LC-MS (ESI): *m*/*z* 597.3[M+H$^+$].

$^1$H NMR (400 MHz, DMSO-$d_6$) $\delta$ 12.54 (s, 1H), 8.84 (s, 1H), 8.48 (d, *J*= 5.2 Hz, 1H), 8.06 (dd, *J* = 12.9, 2.4 Hz, 1H), 7.64 - 7.58 (m, 2H), 7.56 - 7.51 (m, 2H), 7.45 (t, *J* = 8.9 Hz, 1H), 7.42 - 7.35 (m, 3H), 6.49 (dd, *J*= 5.2, 1.1 Hz, 1H), 4.85 (h, *J*= 6.6 Hz, 1H), 3.95 (d, *J*= 2.2 Hz, 6H), 1.63 (d, *J* = 6.6 Hz, 6H).

Example 25: preparation of 6-cyano-*N*-(4-((6-cyano-7-methoxyquinolin-4-yl)oxy)-3-fluorophenyl)-5-(4-fluorophenyl)-1-isopropyl-4-oxo-1,4-dihydropyridine-3-carboxamide (**25**)

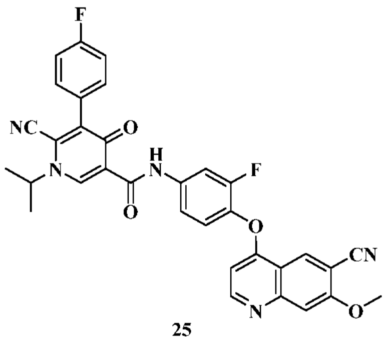

25

The same procedures as in Example 21 were performed, except that 4-(4-amino-2-fluoro-phenoxy)-7-methoxyquinoline-6-carboxamide (intermediate **cc**) was used in place of 4-((6,7-dimethoxyquinolin-4-yl)oxy)-3-fluoroaniline hydrochloride (intermediate **aa**), and 6-(ethoxycarbonyl)-5-(4-fluorophenyl)-1-isopropyl-4-oxo-1,4-dihydropyridine-3-carboxylic acid (**a**) was used in place of 6-(ethoxycarbonyl)-5-(4-fluorophenyl)-1-methyl-4-oxo-1,4-dihydropyridine-3-carboxylic acid (**b**), so as to give 6-cyano-*N*-(4-((6-cyano-7-methoxyquinolin-4-yl)-oxy)-3-fluorophenyl)-5-(4-fluorophenyl)-1-isopropyl-4-oxo-1,4-dihydropyridine-3-carboxamide (**25**) (30 mg, yellow solid, four-step yield: 19.3%).

LC-MS (ESI): *m*/*z* 592.21[M+H$^+$].

$^1$H NMR (400 MHz, DMSO-$d_6$) $\delta$ 12.57 (s, 1H), 8.84 (s, 1H), 8.81 (s, 1H), 8.77 (d, *J* = 5.3 Hz, 1H), 8.08 (dd, *J*= 12.8, 2.3 Hz, 1H), 7.66 - 7.48 (m, 5H), 7.42 - 7.35 (m, 2H), 6.63 (dd, *J*= 5.3, 1.1 Hz, 1H), 4.86 (p, *J*= 6.6 Hz, 1H), 4.08 (s, 3H), 1.63 (d, *J*= 6.6 Hz, 6H).

Example 26: preparation of 6-(aminomethyl)-*N*-(4-((6,7-dimethoxyquinolin-4-yl)oxy)-3-fluorophenyl)-1-(4-fluorophenyl)-5-methyl-2-oxo-1,2-dihydro-1,2-dihydropyridine-3 - carboxamide (**26**)

Step 1: preparation of *tert*-butyl ((3-bromo-5-((4-((6,7-dimethoxyquinolin-4-yl)oxy)-3-fluorophenyl)carbamoyl)-1-(4-fluorophenyl)-6-oxo-1,6-dihydropyridin-2-yl)methyl)carbamate (**26a**)

4-((6,7-dimethoxyquinolin-4-yl)oxy)-3-fluoroaniline (intermediate **aa**) (77 mg, 0.22 mmol), 6-((*tert*-butoxycarbonyl)amino)methyl)-5-bromo-1-(4-fluorophenyl)-2-oxo-1,2-dihydropyridine-3-carboxylic acid (intermediate **m**) (80 mg, 0.18 mmol), HATU (102 mg, 0.27 mmol) and DIPEA (70 mg, 0.54 mmol) were added to a reaction flask containing DMF (3 mL). The reaction mixture was stirred at room temperature for 30 min. After the reaction was completed, the reaction mixture was added with saturated sodium bicarbonate solution to quench the reaction, and then extracted with ethyl acetate (10 mL × 3). The organic phases were combined, washed with saturated brine, dried over anhydrous sodium sulfate and filtered, and the filtrate was concentrated under reduced pressure. The residue was purified by silica gel column chromatography (eluent: MeOH:DCM = 1:10) to give *tert*-butyl ((3-bromo-5-((4-((6,7-dimethoxyquinolin-4-yl)oxy)-3-fluorophenyl)carbamoyl)-1-(4-fluorophenyl)-6-oxo-1,6-dihydropyridin-2-yl)methyl)carbamate (**26a**) (50 mg, yellow solid, yield: 37.7%).

LC-MS (ESI): *m/z* 737.11/739.17[M+H$^+$].

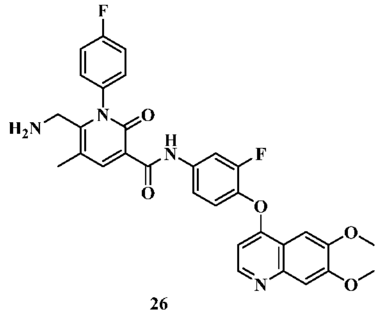

26

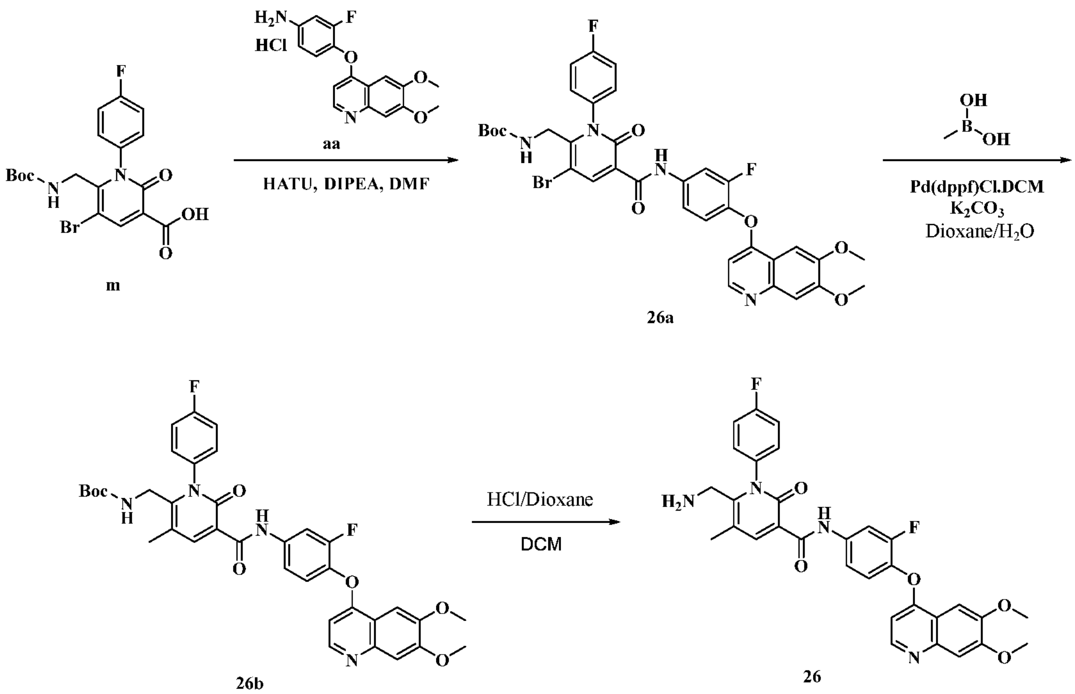


m

26a

26b

26

Step 2: preparation of *tert*-butyl ((5-((4-((6,7-dimethoxyquinolin-4-yl)oxy)-3-fluorophenyl)-carbamoyl)-1-(4-fluorophenyl)-3-methyl-6-oxo-1,6-dihydropyridin-2-yl)methyl)carbamate (**26b**)

*Tert*-butyl ((3-bromo-5-((4-((6,7-dimethoxyquinolin-4-yl)oxy)-3-fluorophenyl)carbamoyl)-1-(4-fluorophenyl)-6-oxo-1,6-dihydropyridin-2-yl)methyl)carbamate (**26a**) (50 mg, 0.07 mmol), methylboronic acid (40 mg, 0.70 mmol), potassium carbonate (28 mg, 0.21 mmol) and Pd(dppf)$Cl_2$.DCM (10 mg, 0.014 mmol) were added to a reaction flask containing 1,4-dioxane (4 mL) and water (1 mL) at room temperature. The reaction mixture was sealed in the flask, purged with nitrogen three times, heated to 100 °C in a microwave reactor and then stirred for 40 min. After being cooled to room temperature, the reaction mixture was diluted with water (15 mL) and extracted with ethyl acetate (15 mL × 3). The organic phases were combined, washed with saturated brine, dried over anhydrous sodium sulfate and filtered, and the filtrate was concentrated under reduced pressure. The residue was purified by silica gel column chromatography (eluent: MeOH:DCM = 1:10) to give *tert-butyl* ((5-((4-((6,7-dimethoxyquinolin-4-yl)oxy)-3-fluorophenyl) carbamoyl)-1-(4-fluorophenyl)-3-methyl-6-oxo-1,6-dihydropyridin-2-yl)methyl)carbamate (**26b**) (20 mg, yellow solid, yield: 42.5%).

LC-MS (ESI): *m/z* 673.30[M+H$^+$].

Step 3: preparation of 6-(aminomethyl)-*N*-(4-((6,7-dimethoxyquinolin-4-yl)oxy)-3-fluorophenyl)-1-(4-fluorophenyl)-5-methyl-2-oxo-1,2-dihydro-1,2-dihydropyridine-3-carboxamide (**26**)

*Tert*-butyl ((5-((4-((6,7-dimethoxyquinolin-4-yl)oxy)-3-fluorophenyl)carbamoyl)-1-(4-fluorophenyl)-3-methyl-6-oxo-1,6-dihydropyridin-2-yl)methyl)carbamate (**26b**) (20 mg, 0.03 mmol) and a solution of hydrochloric acid in dioxane (4 N, 1.5 mL) were added to a reaction flask containing DCM (3 mL). The reaction mixture was stirred at room temperature for 30 min. After the reaction was completed, the reaction mixture was concentrated under reduced pressure, and the residue was purified by preparative HPLC (C18, acetonitrile/water (0.1% formic acid): 10%-100%) to give 6-(aminomethyl)-*N*-(4-((6,7-dimethoxyquinolin-4-yl)oxy)-3-fluorophenyl)-1-(4-fluorophenyl)-5-methyl-2-oxo-1,2-dihydro-1,2-dihydropyridine-3-carboxamide (**26**) (9 mg, white solid, yield: 52.3%).

LC-MS (ESI): *m/z* 573.25[M+H$^+$].

$^1$H NMR (400 MHz, DMSO-$d_6$) δ 12.23 (s, 1H), 8.50 - 8.45 (m, 2H), 8.04 (dd, *J* = 13.0, 2.5 Hz, 1H), 7.55 - 7.50 (m, 4H), 7.47 - 7.40 (m, 4H), 6.47 (dd, *J* = 5.2, 1.1 Hz, 1H), 3.95 (d, *J* = 2.2 Hz, 6H), 2.34 (s, 3H).

Example 27: preparation of (4-((6,7-dimethoxyquinolin-4-yl)oxy)-3-fluorophenyl)-1-(4-fluorophenyl)-5-methyl-6-((methylamino)methyl)-2-oxo-1,2-dihydropyridine-3-carboxamide (**27**)

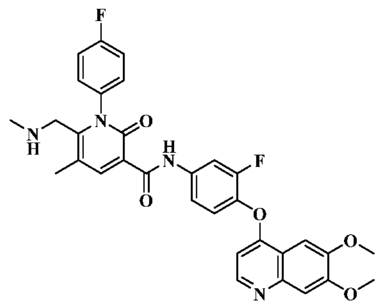

27

The same procedures as in Example 26 were performed, except that 5-bromo-6-(((*tert*-butoxycarbonyl)(methyl)amino)methyl)-1-(4-fluorophenyl)-2-oxo-1,2-dihydropyridine-3-carboxylic acid (intermediate **l**) was used in place of 5-bromo-6-(((*tert*-butoxycarbonyl)(methyl)-amino-1-(4-fluorophenyl)-2-oxo-1,2-dihydropyridine-3-carboxylic acid (intermediate **m**), so as to give (4-((6,7-dimethoxyquinolin-4-yl)oxy)-3-fluorophenyl)-1-(4-fluorophenyl)-5-methyl-6-((methylamino)methyl)-2-oxo-1,2-dihydropyridine-3-carboxamide (**27**) (yellow solid, three-step yield: 16.8%).

LC-MS (ESI): *m/z* 587.35[M+H$^+$].

$^1$H NMR (400 MHz, DMSO-$d_6$) δ 12.23 (s, 1H), 8.49 - 8.44 (m, 2H), 8.04 (dd, *J* = 12.9, 2.5 Hz, 1H), 7.58 - 7.48 (m, 4H), 7.46 - 7.39 (m, 4H), 6.47 (dd, *J*= 5.3, 1.1 Hz, 1H), 3.95 (d, *J*= 2.4 Hz, 6H), 3.26 (s, 2H), 2.34 (s, 3H), 2.04 (s, 3H).

Example 28: preparation of 5-methyl-*N*-(4-((6,7-dimethoxyquinolin-4-yl)oxy)-3-fluorophenyl)-1-(4-fluorophenyl)-6-(methoxymethyl)-2-oxo-1,2-dihydropyridine-3-carboxamide (**28**)

Step 1: preparation of 5-bromo-*N*-(4-((6,7-dimethoxyquinolin-4-yl)oxy)-3-fluorophenyl)-1-(4-fluorophenyl)-6-(methoxymethyl)-2-oxo-1,2-dihydropyridine-3-carboxamide (intermediate **28a**)

4-((6,7-dimethoxyquinolin-4-yl)oxy)-3-fluoroaniline hydrochloride (intermediate **aa**) (126 mg, 0.40 mmol), 5-bromo-1-(4-fluorophenyl)-6-(methoxymethyl)-2-oxo-1,2-dihydropyridine-3-carboxylic acid (intermediate **n**) (130 mg,

0.36 mmol), HATU (180 mg, 0.47 mmol) and DIPEA (140 mg, 1.0 mmol) were added to a reaction flask containing DMF (5 mL). The reaction mixture was stirred at room temperature for 30 min. After the reaction was completed, the reaction mixture was added with saturated sodium bicarbonate solution to quench the reaction, and then extracted with ethyl acetate (10 mL × 3). The organic phases were combined, washed with saturated brine, dried over anhydrous sodium sulfate and filtered, and the filtrate was concentrated under reduced pressure. The residue was purified by silica gel column chromatography (eluent: MeOH:DCM =1:10) to give 5-bromo-*N*-(4-((6,7-dimethoxyquinolin-4-yl)oxy)-3-fluorophenyl)-1-(4-fluorophenyl)-6-(methoxymethyl)-2-oxo-1,2-dihydropyridine-3-carboxamide (intermediate **28a**) (160 mg, yellow solid, yield: 68.1%).

LC-MS (ESI): *m/z* 652.11/654.08[M+H$^+$],

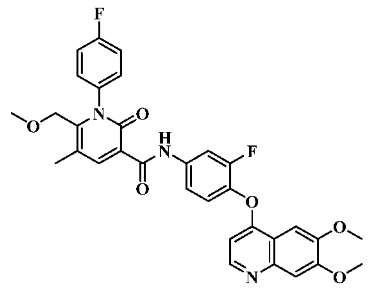

28

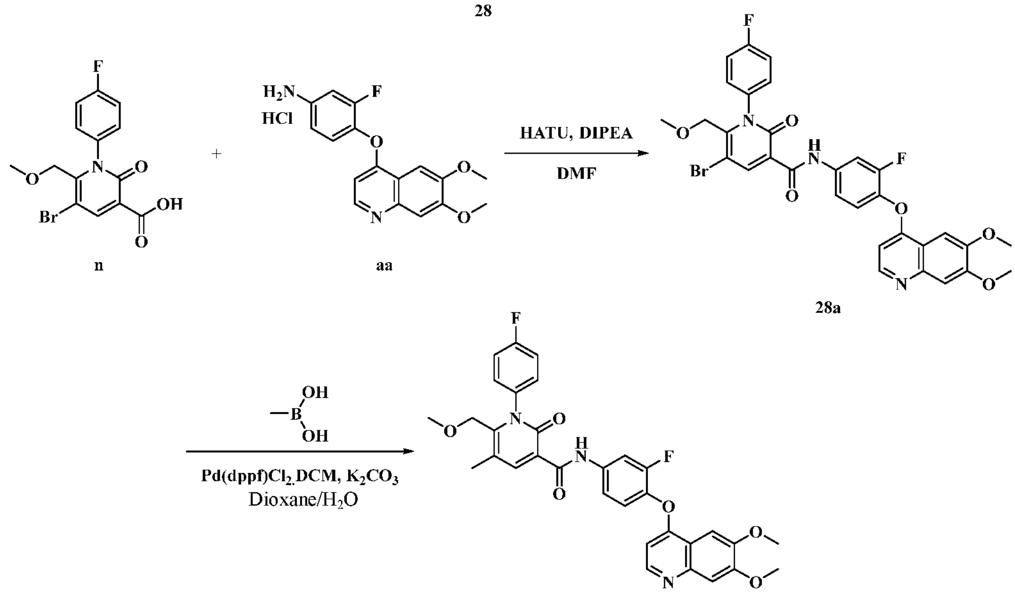


n aa 28a 28

Step 2: preparation of 5-methyl-*N*-(4-((6,7-dimethoxyquinolin-4-yl)oxy)-3-fluorophenyl)-1-(4-fluorophenyl)-6-(methoxymethyl)-2-oxo-1,2-dihydropyridine-3-carboxamide (**28**)

5-bromo-*N*-(4-((6,7-dimethoxyquinolin-4-yl)oxy)-3-fluorophenyl)-1-(4-fluorophenyl)-6-(methoxymethyl)-2-oxo-1,2-dihydropyridine-3-carboxamide (intermediate **28a**) (60 mg, 0.09 mmol), methylboronic acid (30 mg, 0.45 mmol), potassium carbonate (36 mg, 0.27 mmol), and Pd(dppf)$Cl_2$.DCM (15 mg, 0.018 mmol) were added to a reaction flask containing 1,4-dioxane (8 mL) and water (2 mL) at room temperature. The reaction mixture was sealed in the flask, purged with nitrogen three times, heated to 100 °C in a microwave reactor and then stirred for 40 min. After being cooled to room temperature, the reaction mixture was diluted with water (15 mL) and extracted with ethyl acetate (15 mL × 3). The organic phases were combined, washed with saturated brine, dried over anhydrous sodium sulfate and filtered, and the filtrate was concentrated under reduced pressure. The residue was purified by preparative HPLC (C18, acetonitrile/water (0.1% formic acid): 10%-100%) to give 5-methyl-*N*-(4-((6,7-dimethoxyquinolin-4-yl)oxy)-3-fluorophenyl)-1-(4-fluoro-phenyl)-6-(methoxymethyl)-2-oxo-1,2-dihydropyridine-3-carboxamide **(28)** (15 mg, yellow solid, yield: 28.3%).

LC-MS (ESI): *m/z* 588.20(M+H$^+$].

$^1$H NMR (400 MHz, DMSO-$d_6$) δ 12.19 (s, 1H), 8.52 - 8.47 (m, 2H), 8.04 (dd, *J* = 12.9, 2.4 Hz, 1H), 7.53 (s, 2H), 7.46 - 7.40 (m, 6H), 6.50 - 6.47 (m, 1H), 4.03 (s, 2H), 3.95 (d, *J* = 2.3 Hz, 6H), 3.01 (s, 3H), 2.33 (s, 3H).

Example 29: preparation of 6-cyano-*N*-(4-((6,7-dimethoxyquinolin-4-yl)oxy)-3-fluorophenyl)-1-(4-fluorophenyl)-5-methyl-2-oxo-1,2-dihydropyridine-3-carboxamide **(29A)** and 5-bromo-*N*-(4-((6,7-dimethoxyquinolin-4-yl)oxy)-3-fluorophenyl)-1-(4-fluorophenyl)-6-hydroxy-2-oxo -1,2-dihydropyridine-3-carboxamide **(29B)**

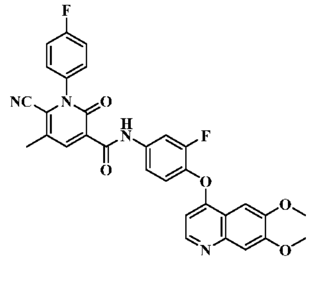

29A

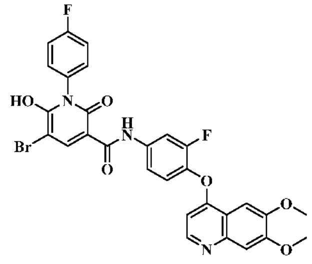

29B

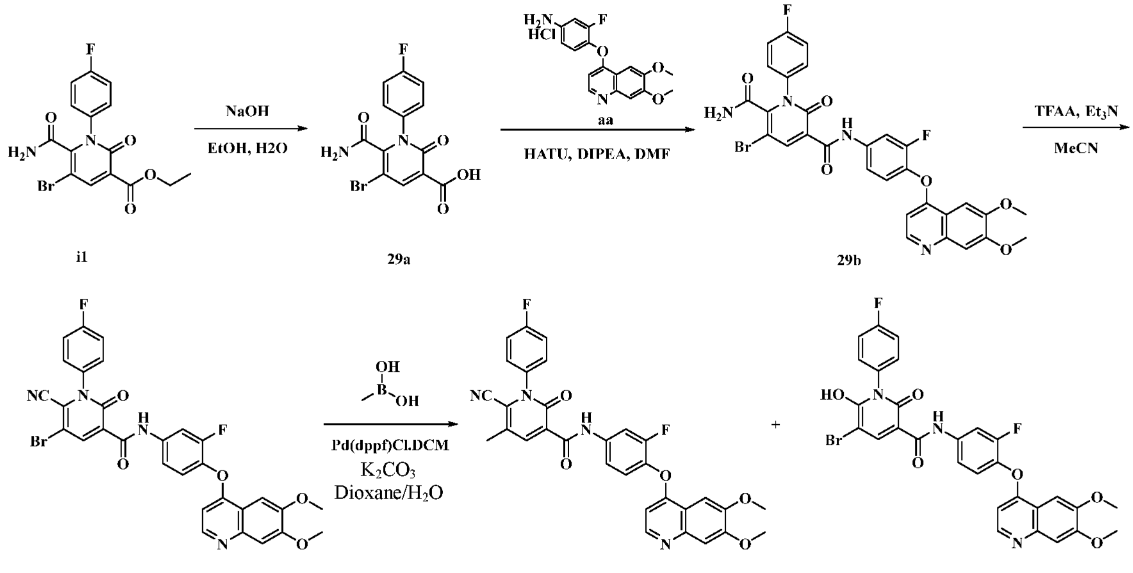


i1 29a 29b

29c 29A 29B

Step 1: preparation of 5-bromo-6-carbamoyl-1-(4-fluorophenyl)-2-oxo-1,2-dihydropyridine-3-carboxylic acid (intermediate **29a)**

Ethyl 5-bromo-6-carbamoyl-1-(4-fluorophenyl)-2-oxo-1,2-dihydropyridine-3-carboxylate (**i1**) (210 mg, 0.55 mmol) was added to a reaction flask containing ethanol (4.5 mL). After being cooled to 0 °C, the reaction mixture was added dropwise with aqueous sodium hydroxide solution (1.5 mL, 28.5 mg, 0.71 mmol) slowly, and after the addition was completed, the reaction mixture was warmed to room temperature and stirred for 30 min. After the reaction was completed, the reaction mixture was concentrated under reduced pressure to give crude 5-bromo-6-carbamoyl-1-(4-fluorophenyl)-2-oxo-1,2-dihydropyridine-3-carboxylic acid (intermediate **29a)** (210 mg, yellow solid, yield: 100%).

LC-MS (ESI): *m*/*z* 355-12/357.08(M+$H^+$].

Step 2: preparation of 3-bromo-*N*$^5$-(4-((6,7-dimethoxyquinolin-4-yl)oxy)-3-fluorophenyl)-1-(4-fluorophenyl)-6-oxo-1,6-dihydropyridine-2,5-dicarboxamide (intermediate **29b)**

4-((6,7-dimethoxyquinolin-4-yl)oxy)-3-fluoroaniline hydrochloride (intermediate **aa)** (193 mg, 0.62 mmol), 5-bromo-6-carbamoyl-1-(4-fluorophenyl)-2-oxo-1,2-dihydropyridine-3-carboxylic acid (intermediate **29a)** (200 mg, 0.56 mmol), HATU (320 mg, 0.84 mmol) and DIPEA (218 mg, 1.68 mmol) were added to a reaction flask containing DMF (5 mL). The reaction mixture was stirred at room temperature for 30 min. After the reaction was completed, the reaction mixture was added with saturated sodium bicarbonate solution to quench the reaction, and then extracted with ethyl acetate (10 mL × 3). The organic phases were combined, washed with saturated brine, dried over anhydrous sodium sulfate and filtered, and the filtrate was concentrated under reduced pressure. The residue was purified by silica gel

column chromatography (eluent: MeOH:DCM = 1:10) to give 3 -bromo-$N^5$-(4-((6,7-dimethoxyquinolin-4-yl)oxy)-3-fluorophenyl)-1-(4-fluorophenyl)-6-oxo-1,6-dihydropyridine-2,5-dicarboxamide (intermediate **29b)** (260 mg, yellow solid, yield: 71.3%).

LC-MS (ESI): *m/z* 651.21/653.17[M+H$^+$].

Step 3: preparation of 5-bromo-6-cyano-*N*-(4-((6,7-dimethoxyquinolin-4-yl)oxy)-3-fluorophenyl)-1-(4-fluorophenyl)-2-oxo-1,2-dihydropyridine-3-carboxamide **(29c)**

3-bromo-$N^5$-(4-((6,7-dimethoxyquinolin-4-yl)oxy)-3-fluorophenyl)-1-(4-fluorophenyl)-6-oxo-1,6-dihydropyridine-2,5-dicarboxamide (intermediate **29b)** (140 mg, 0.22 mol) was added to a reaction flask containing acetonitrile (5 mL). After being cooled to 0 °C, the reaction mixture was added dropwise with trifluoroacetic anhydride (180 mg, 0.84 mol) and triethylamine (130 mg, 0.84 mol) slowly and successively. The reaction mixture was stirred at 0 °C for 1 h. After the reaction was completed, the reaction mixture was diluted with water (10 mL) and extracted with ethyl acetate (10 mL × 3). The combined organic phases were washed with saturated brine, dried over anhydrous sodium sulfate and filtered, and the filtrate was concentrated under reduced pressure to give crude 5-bromo-6-cyano-*N*-(4-((6,7-dimethoxyquinolin-4-yl)oxy)-3-fluorophenyl)-1-(4-fluorophenyl)-2-oxo-1,2-dihydropyridine-3-carboxamide **(29c)** (130 mg, yellow solid, yield: 100%).

LC-MS (ESI): *m/z* 633.18/633.14[M+H$^+$].

Step 4: preparation of 6-cyano-*N*-(4-((6,7-dimethoxyquinolin-4-yl)oxy)-3-fluorophenyl)-1-(4-fluorophenyl)-5-methyl-2-oxo-1,2-dihydropyridine-3-carboxamide **(29A)** and 5-bromo-*N*-(4-((6,7-dimethoxyquinolin-4-yl)oxy)-3-fluorophenyl)-1-(4-fluorophenyl)-6-hydroxy-2-oxo-1,2-dihydropyridine-3-carboxamide **(29B)**

5-bromo-6-cyano-*N*-(4-((6,7-dimethoxyquinolin-4-yl)oxy)-3-fluorophenyl)-1-(4-fluorophenyl)-2-oxo-1,2-dihydropyridine-3-carboxamide **(28c)** (40 mg, 0.065 mmol), methylboronic acid (37 mg, 0.65 mmol), potassium carbonate (30 mg, 0.20 mmol) and Pd(dppf)$Cl_2$.DCM (10 mg, 0.013 mmol) were added to a reaction flask containing 1,4-dioxane (3 mL) and water (1 mL) at room temperature. The reaction mixture was sealed in the flask, purged with nitrogen three times, heated to 100 °C in a microwave reactor and then stirred for 40 min. After being cooled to room temperature, the reaction mixture was diluted with water (15 mL) and extracted with ethyl acetate (15 mL × 3). The organic phases were combined, washed with saturated brine, dried over anhydrous sodium sulfate and filtered, and the filtrate was concentrated under reduced pressure. The residue was purified by preparative HPLC (C18, acetonitrile/water (0.1% formic acid): 10%-100%) to give 6-cyano-*N*-(4-((6,7-dimethoxyquinolin-4-yl)oxy)-3-fluorophenyl)-1-(4-fluorophenyl)-5-methyl-2-oxo-1,2-dihydropyridine-3-carboxamide **(29A)** (4 mg, yellow solid, yield: 7.1%) and 5-bromo-*N*-(4-((6,7-dimethoxyquinolin-4-yl)oxy)-3-fluorophenyl)-1-(4-fluorophenyl)-6-hydroxy-2-oxo-1,2-dihydropyridine-3-carboxamide **(29B)** (by-product) (yellow solid, 15 mg).

29A: LC-MS (ESI): *m/z* 569.25[M+H$^+$]. $^1$H NMR (400 MHz, DMSO-$d_6$) δ 11.89 (s, 1H), 8.60 (s, 1H), 8.48 (d, *J*= 5.2 Hz, 1H), 8.04 (dd, *J*= 12.8, 2.5 Hz, 1H), 7.74 - 7.66 (m, 2H), 7.59 (ddd, *J*= 8.9, 2.5, 1.1 Hz, 1H), 7.54-7.44 (m, 4H), 7.41 (s, 1H), 6.48 (dd, *J* = 5.2, 1.1 Hz, 1H), 3.95 (d, *J* = 2.4 Hz, 6H), 2.45 (s, 3H).

29B: LC-MS (ESI): *m/z* 624.07/626.06[M+H$^+$]. $^1$H NMR (400 MHz, DMSO-$d_6$) δ 12.23 (s, 1H), 8.72 (d, *J*= 6.2 Hz, 1H), 8.16 (d, *J*= 18.5 Hz, 1H), 8.08 (dd, *J*= 13.6, 2.3 Hz, 1H), 7.70 (s, 1H), 7.48 (s, 1H), 7.44 - 7.34 (m, 2H), 7.28 - 7.22 (m, 2H), 7.16 (ddt, *J* = 7.0, 5.1, 2.7 Hz, 2H), 6.84 (d, *J* = 6.1 Hz, 1H), 4.01 (d, *J*= 3.2 Hz, 6H).

Example 30: preparation of 6-cyano-*N*-(4-((6,7-dimethoxyquinolin-4-yl)oxy)-3-fluorophenyl)-1-(4-fluorophenyl)-5-isopropyl-2-oxo-1,2-dihydropyridine-3-carboxamide **(30)**

Step 1: preparation of diethyl 3-bromo-1-(4-fluorophenyl)-6-oxo-1,6-dihydropyridine-2,5-dicarboxylate **(30a)**

5-bromo-6-(ethoxycarbonyl)-1-(4-fluorophenyl)-2-oxo-1,2-dihydropyridine-3-carboxylic acid (intermediate **h)** (1.5 g, 3.90 mmol), potassium carbonate (809 mg, 5.86 mmol) and iodoethane (910 mg, 5.86 mmol) were added to a reaction flask containing DMF (10 mL). The reaction mixture was stirred at room temperature for 6 h. After the reaction was completed, the reaction mixture was added with water (50 mL) and then extracted with ethyl acetate (20 mL × 3). The combined organic phases were washed with saturated brine, dried over anhydrous sodium sulfate and filtered, and the filtrate was concentrated under reduced pressure. The residue was purified by silica gel column chromatography (eluent: PE:EA= 2:1) to give diethyl 3-bromo-1-(4-fluorophenyl)-6-oxo-1,6-dihydropyridine-2,5-dicarboxylate **(30a)** (1.3 g, yellow solid, yield: 80.9%).

LC-MS (ESI): *m/z* 412.15/414.11[M+H$^+$].

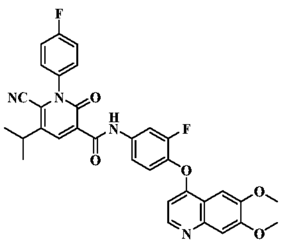

30

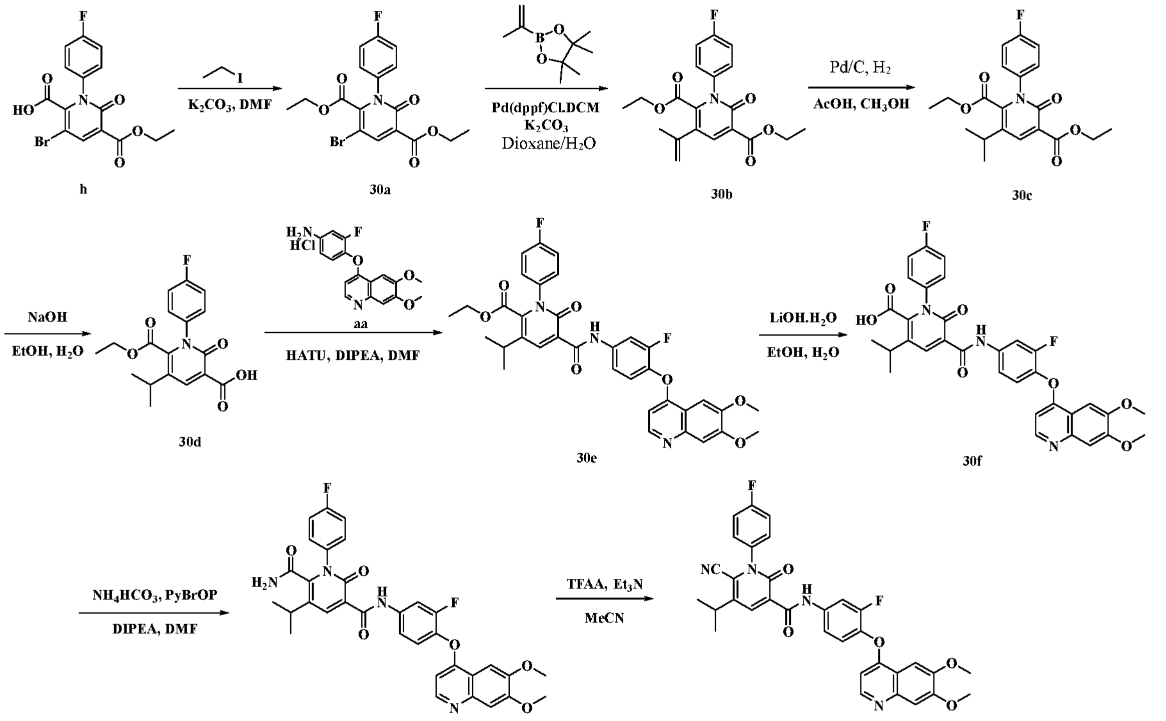


30g

30

Step 2: preparation of diethyl 1-(4-fluorophenyl)-6-oxo-3-(prop-1-en-2-yl)-1,6-dihydropyridine-2,5-dicarboxylate **(30b)**

Diethyl 3-bromo-1-(4-fluorophenyl)-6-oxo-1,6-dihydropyridine-2,5-dicarboxylate **(30a)** (300 mg, 0.73 mmol), pinacol isopropenylborate (611 mg, 3.64 mmol), potassium carbonate (302 mg, 2.19 mmol) and Pd(dppf)Cl·DCM (60 mg, 0.073 mmol) were added to a reaction flask containing 1,4-dioxane (16 mL) and water (4 mL) at room temperature. The reaction mixture was sealed in the flask, purged with nitrogen three times, heated to 100 °C in a microwave reactor and then stirred for 30 min. After being cooled to room temperature, the reaction mixture was diluted with water (10 mL) and then extracted with ethyl acetate (10 mL × 3). The combined organic phases were washed with saturated brine, dried over anhydrous sodium sulfate and filtered, and the filtrate was concentrated under reduced pressure. The residue was purified by silica gel column chromatography (eluent: PE:EA= 1:1) to give diethyl 1-(4-fluorophenyl)-6-oxo-3-(prop-1-en-2-yl)-1,6-dihydropyridine-2,5-dicarboxylate **(30b)** (220 mg, yellow solid, yield: 80.7%).

LC-MS (ESI): *m/z* 374.27(M+H$^+$).

Step 3: preparation of diethyl 1-(4-fluorophenyl)-3-isopropyl-6-oxo-1,6-dihydropyridine-2,5-dicarboxylate **(30c)**

Diethyl 1-(4-fluorophenyl)-6-oxo-3-(prop-1-en-2-yl)-1,6-dihydropyridine-2,5-dicarboxylate **(30b)** (220 mg, 0.59 mol), palladium on carbon (55 mg, 25%) and acetic acid (8 mg, 0.12 mol) were added to a reaction flask containing methanol (4 mL). After being sealed in the flask and purged with nitrogen three times, the reaction mixture was purged with hydrogen three times and then stirred for 30 min. The reaction mixture was filtered, and the filtrate was concentrated under reduced pressure to give crude diethyl 1-(4-fluorophenyl)-3-isopropyl-6-oxo-1,6-dihydropyridine-2,5-dicarboxylate **(30c)** (200 mg, white solid, yield: 91.4%).

LC-MS (ESI): *m/z* 376.30(M+H$^+$).

Step 4: preparation of 6-(ethoxycarbonyl)-1-(4-fluorophenyl)-5-isopropyl-2-oxo-1,2-dihydropyridine-3-carboxylic acid **(30d)**

Diethyl 1-(4-fluorophenyl)-3-isopropyl-6-oxo-1,6-dihydropyridine-2,5-dicarboxylate **(30c)** (200 mg, 0.53 mmol) was added to a reaction flask containing ethanol (4.5 mL). After being cooled to 0 °C, the reaction mixture was added dropwise with aqueous sodium hydroxide solution (1.5 mL, 28.5 mg, 0.71 mmol) slowly, and after the addition was completed, the reaction mixture was warmed to room temperature and stirred for 30 min. After the reaction was completed, the reaction mixture was concentrated under reduced pressure to give crude 6-(ethoxycarbonyl)-1-(4-fluorophenyl)-5-isopropyl-2-oxo-1,2-dihydropyridine-3-carboxylic acid **(30d)** (200 mg, yellow solid, yield: 100%).
LC-MS (ESI): *m/z* 348.27(M+H$^+$).

Step 5: preparation of ethyl 5-((4-((6,7-dimethoxyquinolin-4-yl)oxy)-3-fluorophenyl)-carbamoyl)-1-(4-fluorophenyl)-3-isopropyl-6-oxo-1,6-dihydropyridine-2-carboxylate **(30e)**

4-((6,7-dimethoxyquinolin-4-yl)oxy)-3-fluoroaniline hydrochloride (intermediate **aa)** (193 mg, 0.62 mmol), 6-(ethoxycarbonyl)-1-(4-fluorophenyl)-5-isopropyl-2-oxo-1,2-dihydropyridine-3-carboxylic acid **(30d)** (200 mg, 0.56 mmol), HATU (320 mg, 0.84 mmol) and DIPEA (218 mg, 1.68 mmol) were added to a reaction flask containing DMF (5 mL). The reaction mixture was stirred at room temperature for 30 min. After the reaction was completed, the reaction mixture was added with saturated sodium bicarbonate solution to quench the reaction, and then extracted with ethyl acetate (10 mL × 3). The organic phases were combined, washed with saturated brine, dried over anhydrous sodium sulfate and filtered, and the filtrate was concentrated under reduced pressure. The residue was purified by silica gel column chromatography (eluent: MeOH:DCM = 1:10) to give ethyl 5-((4-((6,7-dimethoxyquinolin-4-yl)oxy)-3-fluorophenyl)carbamoyl)-1-(4-fluorophenyl)-3-isopropyl-6-oxo-1,6-dihydropyridine-2-carboxylate **(30e)** (220 mg, yellow solid, yield: 60.7%).
LC-MS (ESI): *m/z* 644.36 [M+H$^+$].

Step 6: preparation of 5-((4-((6,7-dimethoxyquinolin-4-yl)oxy)-3-fluorophenyl)carbamoyl)-1-(4-fluorophenyl)-3-isopropyl-6-oxo-1,6-dihydropyridine-2-carboxylic acid **(30f)**

Ethyl 5-((4-((6,7-dimethoxyquinolin-4-yl)oxy)-3-fluorophenyl)carbamoyl)-1-(4-fluorophenyl)-3-isopropyl-6-oxo-1,6-dihydropyridine-2-carboxylate **(30e)** (220 mg, 0.34 mmol), lithium hydroxide monohydrate (22 mg, 0.51 mmol) and water (1.5 mL) were added to a reaction flask containing ethanol (4.5 mL). The reaction mixture was stirred at 70 °C for 12 h. After the reaction was completed, the reaction mixture was concentrated under reduced pressure, and the residue was purified by preparative HPLC (C18, acetonitrile/water (0.1% formic acid): 10%-100%) to give 5-((4-((6,7-dimethoxyquinolin-4-yl)oxy)-3-fluorophenyl)carbamoyl)-1-(4-fluorophenyl)-3-isopropyl-6-oxo-1,6-dihydropyridine-2-carboxylic acid **(30f)** (80 mg, white solid, yield: 38.2%).
LC-MS (ESI): *m/z* 616.35(M+H$^+$).

Step 7: preparation of *N*$^5$-(4-((6,7-dimethoxyquinolin-4-yl)oxy)-3-fluorophenyl)-1-(4-fluorophenyl)-3-isopropyl-6-oxo-1,6-dihydropyridine-2,5-dicarboxamide **(30g)**

5-((4-((6,7-dimethoxyquinolin-4-yl)oxy)-3-fluorophenyl)carbamoyl)-1-(4-fluorophenyl)-3-ispro pyl-6-oxo-1,6-dihydropyridine-2-carboxylic acid **(30f)** (80 mg, 0.13 mmol), ammonium bicarbonate (103 mg, 1.3 mmol), PyBrOP (91 mg, 0.20 mmol) and DIPEA (50 mg, 0.39 mol) were added to a reaction flask containing DMF (3 mL). The reaction mixture was stirred at room temperature for 1 h. After the reaction was completed, the reaction mixture was added with saturated aqueous sodium bicarbonate solution (10 mL) to quench the reaction, and then extracted with ethyl acetate (10 mL × 3). The combined organic phases were washed with saturated brine, dried over anhydrous sodium sulfate and filtered, and the filtrate was concentrated under reduced pressure. The residue was purified by silica gel column chromatography (eluent: DCM:$CH_3OH$ = 10:1) to give *N*$^5$-(4-((6,7-dimethoxyquinolin-4-yl)oxy)-3-fluorophenyl)-1-(4-fluorophenyl)-3-isopropyl-6-oxo-1,6-dihydropyridine-2,5-dicarboxamide **(30g)** (20 mg, white solid, yield: 25.0%).
LC-MS (ESI): *m/z* 615.34(M+H$^+$).

Step 8: preparation of 6-cyano-*N*-(4-((6,7-dimethoxyquinolin-4-yl)oxy)-3-fluorophenyl)-1-(4-fluorophenyl)-5-isopropyl-2-oxo-1,2-dihydropyridine-3-carboxamide **(30)**

*N*$^5$-(4-((6,7-dimethoxyquinolin-4-yl)oxy)-3-fluorophenyl)-1-(4-fluorophenyl)-3-isopropyl-6-oxo -1,6-dihydropyridine-2,5-dicarboxamide **(30g)** (12 mg, 0.02 mol) was added to a reaction flask containing acetonitrile (2 mL). After being cooled to 0 °C, the reaction mixture was added dropwise with trifluoroacetic anhydride (11 mg, 0.06 mol) and triethylamine

(12 mg, 0.12 mol) slowly and successively. The reaction mixture was stirred at 0 °C for 1 h. After the reaction was completed, the reaction mixture was diluted with water (10 mL) and extracted with ethyl acetate (10 mL × 3). The combined organic phases were washed with saturated brine, dried over anhydrous sodium sulfate and filtered, and the filtrate was concentrated under reduced pressure. The residue was purified by preparative HPLC (C18, acetonitrile/water (0.1% formic acid): 20%-100%) to give 6-cyano-*N*-(4-((6,7-dimethoxyquinolin-4-yl)oxy)-3-fluorophenyl)-1-(4-fluoro-phenyl)-5-isopropyl-2-oxo-1,2-dihydropyridine-3-carboxamide **(30)** (8 mg, yellow solid, yield: 67.0%).

LC-MS (ESI): *m/z* 597.33 [M+H$^+$].

$^1$H NMR (400 MHz, DMSO-$d_6$) δ 11.85 (s, 1H), 8.60 (s, 1H), 8.48 (d, *J*= 5.2 Hz, 1H), 8.05 (dd, *J*= 12.8, 2.5 Hz, 1H), 7.75 - 7.69 (m, 2H), 7.59 (ddd, J= 8.8, 2.5, 1.1 Hz, 1H), 7.54 - 7.45 (m, 4H), 7.41 (s, 1H), 6.49 (dd, *J* = 5.3, 1.1 Hz, 1H), 3.95 (d, *J* = 2.7 Hz, 6H), 2.00 (q, *J* = 7.0, 6.5 Hz, 1H), 1.33 (d, *J* = 6.8 Hz, 6H).

Example 31: preparation of 6-cyano-5-cyclopropyl-*N*-(4-((6,7-dimethoxyquinolin-4-yl)oxy)-3-fluorophenyl)-1-(4-fluorophenyl)-2-oxo-1,2-dihydropyridine-3-carboxamide **(31)**

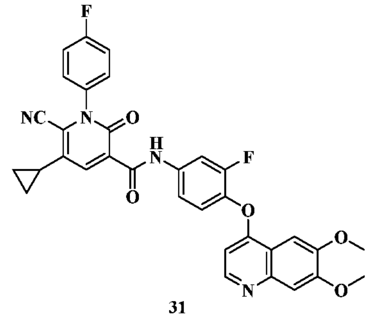

31

The same synthesis procedure as step 5 in Example 30 was performed, except that 6-cyano-5-cyclopropyl-1-(4-fluorophenyl)-2-oxo-1,2-dihydropyridine-3-carboxylic acid (intermediate j) was used in place of 6-(ethoxycarbonyl)-1-(4-fluorophenyl)-5-isopropyl-2-oxo-1,2-dihydropyridine-3-carboxylic acid **(30d),** so as to give 6-cyano-5-cyclopropyl-*N*-(4-((6,7-dimethoxyquinolin-4-yl)oxy)-3-fluorophenyl)-1-(4-fluorophenyl)-2-oxo-1,2-dihydropyridine-3-carboxamide (31) (yellow solid, 5.6 mg, yield: 16.5%).

LC-MS (ESI): *m/z* 595.33(M+H$^+$].

$^1$H NMR (400 MHz, DMSO-$d_6$) δ 11.84 (s, 1H), 8.48 (d, *J*= 5.2 Hz, 1H), 8.10 (s, 1H), 8.03 (dd, *J* = 12.8, 2.5 Hz, 1H), 7.75 - 7.68 (m, 2H), 7.61 - 7.56 (m, 1H), 7.55 - 7.44 (m, 4H), 7.41 (s, 1H), 6.48 (dd, *J*= 5.2, 1.0 Hz, 1H), 3.95 (d, *J* = 3.1 Hz, 6H), 2.10 (td, *J*= 8.3, 4.1 Hz, 1H), 1.19 - 1.13 (m, 2H), 0.93 (dt, *J*= 6.8, 4.8 Hz, 2H).

Example 32: preparation of 6-cyano-*N*-(4-((6,7-dimethoxyquinolin-4-yl)oxy)-3-fluorophenyl)-1-(4-fluorophenyl)-2-oxo-5-(prop-1-en-2-yl)-1,2-dihydropyridine-3-carboxamide (32)

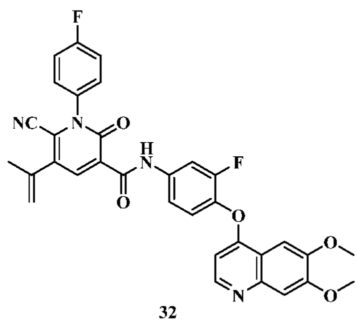

32

The same synthesis procedure as step 5 in Example 30 was performed, except that 6-cyano-1-(4-fluorophenyl)-2-oxo-5-(prop-1-en-2-yl)-1,2-dihydropyridine-3-carboxylic acid (intermediate **k)** was used in place of 6-(ethoxycarbonyl)-1-(4-fluorophenyl)-5-isopropyl-2-oxo-1,2-dihydropyridine-3-carboxylic acid **(30d),** so as to give 6-cyano-*N*-(4-((6,7-dimethoxyquinolin-4-yl)oxy)-3-fluorophenyl)-1-(4-fluorophenyl)-2-oxo-5-(prop-1-en-2-yl))-1,2-dihydropyridine-3-carboxamide (32) (yellow solid, 13 mg, yield: 19.8%).

LC-MS (ESI): *m/z* 595.32(M+H$^+$).

$^1$H NMR (400 MHz, DMSO-$d_6$) δ 11.77 (s, 1H), 8.57 - 8.50 (m, 2H), 8.06 (dd, *J*= 12.8, 2.5 Hz, 1H), 7.78 - 7.69 (m, 2H), 7.61 (dt, *J*= 8.9, 1.7 Hz, 1H), 7.57 - 7.45 (m, 4H), 7.43 (s, 1H), 6.55 (d, *J* = 5.4 Hz, 1H), 5.51 (t, *J*= 1.6 Hz, 1H), 5.44 (s, 1H), 3.96 (d, *J*= 2.8 Hz, 6H), 2.18 (s, 3H).

Example 33: preparation of *N*-(4-(2-amino-5-(1-ethyl-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide **(33)**

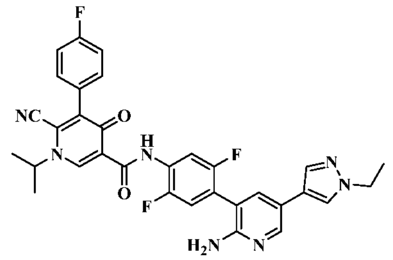

33

The same procedures as in Example 3 were performed, except that 6-cyano-*N*-(2,5-difluoro-4-(4,4,5,5-tetramethyl-1,3,2-dioxaborolan-2-yl)phenyl)-5-(4-fluorophenyl)-1-isopropyl-4-oxo-1,4-dih ydropyridine-3-carboxamide **(y)** was used in place of 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide **(f),** so as to give *N*-(4-(2-amino-5-(1-ethyl-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide **(33)** (25 mg, yellow solid, yield: 35%).

LC-MS (ESI): *m/z* 598.13[M+H$^+$].

$^1$H NMR (400 MHz, DMSO-d6) δ 12.86 (s, 1H), 8.86 (s, 1H), 8.25 (d, J = 2.3 Hz, 1H), 8.08 (d, *J* = 0.8 Hz, 1H), 7.77 (d, *J* = 0.8 Hz, 1H), 7.64 - 7.58 (m, 2H), 7.55 (d, *J* = 2.3 Hz, 1H), 7.45 - 7.36 (m, 3H), 5.68 (s, 2H), 4.87 (p, *J* = 6.5 Hz, 1H), 4.11 (q, *J* = 7.2 Hz, 2H), 1.63 (d, *J* = 6.6 Hz, 6H), 1.38 (t, *J*= 7.3 Hz, 3H).

Example 34: preparation of *N*-(4-(2-amino-5-(1-methyl-$d_3$-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide **(34)**

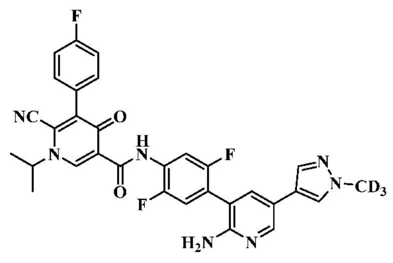

34

The same procedures as in Example 3 were performed, except that 6-cyano-*N*-(2,5-difluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide **(y)** was used in place of 6-cyano-*N*-(3-fluoro-4-(4,4,5,5-tetramethyl-1,3,2-dioxaborolan-2-yl)phenyl)-5-(4-fluorophenyl)-1-isopropyl-4-oxo-1,4-dihydropyridine-3-carboxamide **(f),** and 3-bromo-5-(1-(methyl-$d_3$)-1*H*-pyrazol-4-yl)pyridin-2-amine **(ee)** was used in place of 3-bromo-5-(1-ethyl-1*H*-pyrazol-4-yl)pyridin-2-amine **(v),** so as to give *N*-(4-(2-amino-5-(1-methyl-$d_3$-1*H*-pyrazol-4-yl)pyridin-3-yl)-2,5-difluorophenyl)-6-cyano-5-(4-fluorophenyl)-1-isopropyl-4-oxo-1,4-dihydropyridine-3-carboxamide **(34)** (32 mg, yellow solid, yield: 46%).

LC-MS (ESI): *m/z* 587.28[M+H$^+$].

$^1H$ NMR (400 MHz, DMSO-d6) δ 12.86 (s, 1H), 8.86 (s, 1H), 8.42 (dd, *J* = 11.6, 6.6 Hz, 1H), 8.24 (d, *J* = 2.4 Hz, 1H), 8.02 (d, *J* = 0.8 Hz, 1H), 7.77 (d, J = 0.8 Hz, 1H), 7.65 - 7.57 (m, 2H), 7.54 (d, *J* = 2.4 Hz, 1H), 7.47 - 7.36 (m, 3H), 5.69 (s, 2H), 4.88 (h, *J* = 6.7 Hz, 1H), 1.63 (d, *J* = 6.6 Hz, 6H).

Biological Evaluation of Compounds Disclosed Herein

Test Example 1: Evaluation of inhibitory activity ($IC_{50}$) of compounds disclosed herein against kinases Axl and c-MET

In this test, mobility shift assay was used to test the inhibitory activity of compounds when ATP concentrations correspond to the Km of the kinases. The control substances were staurosporine and cabozantinib.
The concentration of test compound was 10-fold diluted from an initial concentration of 10 μM. The test result ($IC_{50}$) was the average of two independent experiments.

Test materials:

Kinase Axl (Carna, Cat. No. 08-107, Lot. No. 06CBS-3408); kinase c-MET (Carna, Cat. No. 08-151, Lot. No.10CBS-1118M); substrate peptide FAM-P2(GL Biochem, Cat. No. 112394, Lot. No. P131014-XP112394); substrate peptide FAM-P22 (GL Biochem, Cat. No. 112393, Lot. No. P130408-ZB112393); ATP (Sigma, Cat. No. A7699-1G, CAS No. 987-65-5); DMSO (Sigma, Cat. No. D2650, Lot. No. 474382); EDTA (Sigma, Cat. No. E5134, CAS No. 60-00-4); HEPES (Sigma, Cat. No. V900477-500G, CAS No. 7365-45-9, Lot. No. WXBC4716V); DTT (Sigma, Cat. No. D0632-25g, CAS No. 3483-12-3, Lot. No. SLBF3964V); Brij-35 (Sigma, Cat. No. B4184, Lot. No. 018K61251); 96-well plate (Corning, Cat. No. 3365, Lot. No. 22008026); 384-well plate (Corning, Cat. No. 3573, Lot. No. 12608008); staurosporine (MCE, Cat. No. HY-15141, Lot. No. 19340); cabozantinib (prepared according to the method disclosed in patent WO 201101763A1).

Test procedures:

1) Preparation of a buffer: 50 mM HEPES, pH 7.5, 0.00015% Brij-35.
2) Preparation of control substance cabozantinib and test samples: cabozantinib and example compounds of the present invention were each serially diluted in 100% DMSO, then diluted to 10% DMSO with the above buffer, and added to a 384-well plate. For example, a compound at an initial concentration of 10 μM was adjusted to 500 μM with 100% DMSO, then serially diluted for 10 concentrations, and then subjected to 10-fold dilution with the buffer to prepare a diluted compound intermediate containing 10% DMSO, 5 μL of which was transferred to the 384-well plate.
3) The Axl and c-MET enzymes were each diluted to optimal concentrations with the following buffer: 50 mM HEPES, pH 7.5, 0.00015% Brij-35, 2 mM DTT. 10 μL of the two enzyme solutions each was added to the 384-well plate and co-incubated with the compound for 10-15 min at room temperature.
4) The substrate was diluted to optimal concentration with the following buffer: 50 mM HEPES, pH 7.5, 0.00015% Brij-35, 10 mM MgCl2, ATP at Km. 10 μL of the diluted substrate was added to the 384-well plate to initiate the reaction, which lasts for 1 h at 28 °C.
The reaction concentrations of the reagents in the test are shown in Table 1 below.

Table 1

| Kinase | Substrate | | Reaction concentration of kinase | Km: ATP concentration |
|---|---|---|---|---|
| | Peptide sequence | Reaction concentration | | |
| c-MET | Peptide FAM-P2 | 3 μM | 15 nM | 35 μM |
| Axl | Peptide FAM-P22 | 3 μM | 6 nM | 81 μM |

5) The conversion rate was read by Caliper Reader (Perkin Elmer) and the inhibition rate was calculated as the average of two tests.
6) $IC_{50}$ values were fitted with XL-fit software.
The inhibitory activity of the compounds disclosed herein against kinases Axl and c-MET is shown in Table 2 below.

Table 2: Inhibitory activity ($IC_{50}$) of compounds disclosed herein against tyrosine kinases Axl and c-MET

| Compounds | $IC_{50}$ (nM) | |
|---|---|---|
| | Axl | c-MET |
| Example 1 | 13 | 133 |
| Example 2 | 0.46 | 14 |
| Example 3 | 2.7 | 30 |
| Example 4 | 0.28 | 3.0 |
| Example 5 | 2.6 | 38 |
| Example 6 | 2.0 | 26 |
| Example 8 | 1.1 | 17 |
| Example 9 | 4.6 | 70 |
| Example 10 | 2.8 | 23 |
| Example 11 | 0.95 | 10 |
| Example 12 | 2.1 | 23 |
| Example 14 | 12 | 82 |
| Example 15 | 6.6 | 20 |
| Example 16 | 9.8 | 143 |
| Example 17 | 1.8 | 23 |
| Example 18 | 12 | 12 |
| Example 20 | 5.5 | 14 |
| Example 21 | 3.2 | |
| Example 22 | 5.9 | |
| Example 26 | 19 | 19 |
| Example 28 | 1.0 | 21 |
| Example 29A | 3.1 | |
| Example 29B | 3.9 | 30 |
| Cabozantinib | 14.0 | 21 |

As can be seen from Table 2 above, the compounds disclosed herein are effective in inhibiting the activity of kinases Axl and c-MET. Compared with the positive control drug cabozantinib, part of the compounds disclosed herein show higher inhibitory activity.

Test Example 2: Evaluation of inhibitory activity ($IC_{50}$) of compounds disclosed herein against tyrosine kinases Mer and Tyro3

In this test, HTRF method was used to test the inhibitory activity of compounds when ATP concentrations correspond to the Km of the kinases. The control substance was RXDX-106. The concentration of test compound was 3-fold diluted from an initial concentration of 10 $\mu$M, and two duplicate wells were set.

Test materials:

Kinase Mer (Carna, Cat. No. 08-108, Lot. No. 14CBS-0421H); Tyro3 (Carna, Cat. No. 08-109, Lot. No. 08CBS-1186H); HTRF kinase-TK kit (Cisbio, Cat. No. 62TK0PEC), the kit comprising: biotin-TK substrate lyophilized powder (Cisbio, Cat. No. 61TK0BLC, Lot. No. 07A), streptavidin-XL665 (Cisbio, Cat. No. 610SAXLG, Lot. No. 126A), TK antibody-cryptate (Cisbio, Cat. No. 610SAXLG, Lot. No. 04A), and detection buffer (including EDTA) (Cisbio, Cat. No. 62TK0PEC,

Lot. No. 12A); ATP (Sigma, Cat. No. A7699-5G, CAS No. 34369-07-8, Lot. No. SLBQ6014V); DMSO (Sigma, Cat. No. D5879-1L, Lot. No. SHBH9944); HEPES (Sigma, Cat. No. V900477-500G, CAS No. 7365-45-9, Lot. No. WXBC4716V); MgCl2 (Sigma, Cat. No. 208337-1KG, Lot. No. MKBX9508V); EGTA (Sigma, Cat. No. E3889-100g, CAS 67-42-5, CAS No. 7786-30-3, Lot. No. SLBG8546V); NP-40 (Beijing Dingguo, Cat. No. DH218, Lot. No. 36R00160); DTT (Sigma, Cat. No. D0632-25g, CAS No. 3483-12-3, Lot. No. SLBF3964V); compound plate (Labcyte, Cat. No. LP0200, Lot. No. 0006386836); assay plate (Greiner, Cat. No. 784075, Lot. No. E16123HM); control substance RXDX-106 (prepared according to the method disclosed in patent WO2013074633).

Test procedures:

1) Preparation of a buffer: 50 mM HEPES, 10 mM MgCl2, 1 mM EGTA, 0.01% NP-40, 2 mM DTT.
2) Preparation of control substance and test samples: RXDX-106 and the compounds disclosed herein were each dissolved in DMSO to 10 mM and then diluted to 1 mM or an appropriate concentration with DMSO, and then serial double dilution was performed using BRAVO (Agilent). 100 nL of each was transferred from a compound plate (Labcyte-LP0200) to an assay plate (Greiner-784075) using ECHO 555 (Labcyte). The final concentration of DMSO was 1%.
3) Mer and Tyro3 were each diluted to 0.5 nM and 1.2 nM with the detection buffer (Cisbio, Cat. No. 62TK0PEC, Lot. No. 12A). 5 μL of each was added to a 384-well plate and incubated with the compounds disclosed herein for 30 min at 22-25 °C. Final concentrations of Mer and Tyro3 were 0.25 nM and 0.6 nM, respectively.
4) Biotin-TK substrate and ATP were each diluted to a concentration twice the final concentration with 1 × kinase buffer made up of HEPES, $NaN_3$, BSA and orthovanadate. 5 μL of the mixture of substrate and ATP was added to the 384-well plate to initiate the reaction, which lasts for 1 h at 22-25 °C.
The reaction concentrations of the reagents in the test are shown in Table 3 below.

Table 3

| Enzyme | Enzyme concentration, nM | TK substrate concentration, μM | ATP concentration, μM |
|---|---|---|---|
| Mer (80 KD) | 0.25 | 2 | 30 |
| Tyro3 (76 KD) | 0.6 | 2 | 50 |

5) Streptavidin-XL665 and TK antibody-cryptate were diluted to 250 nM and 0.5 nM, respectively, with the detection buffer. 10 μL of each of the two solutions was added to the 384-well plate to initiate the reaction, which lasts for 1 h at 22-25 °C. The final concentrations of streptavidin-XL665 and TK antibody-cryptate were 125 nM and 0.25 nM, respectively.
6) The fluorescence intensities at 665 nm and 615 nm were read with an Envision Reader (PerkinElmer).
7) $\mathbf{IC_{50}}$ values of the compounds were fitted with XL-fit software.

The inhibitory activity of the compounds disclosed herein against the tyrosine kinases Mer and Tyro3 is shown in Table 4 below.

Table 4: Inhibitory activity ($IC_{50}$) of compounds disclosed herein against tyrosine kinases Mer and Tyro3

| Example | $IC_{50}$ (nM) | |
|---|---|---|
| | Mer | Tyro3 |
| Example 1 | 4.90 | 7.51 |
| Example 2 | 0.62 | 1.18 |
| Example 3 | 1.11 | 2.27 |
| Example 4 | 0.78 | 2.67 |
| Example 6 | 1.17 | 1.80 |
| Example 7 | 0.63 | 1.77 |
| Example 9 | 1.30 | 3.00 |
| Example 10 | 2.17 | 30.18 |
| Example 24 | 11.41 | 15.48 |

(continued)

| Example | $IC_{50}$ (nM) | |
|---|---|---|
| | Mer | Tyro3 |
| Example 33 | 0.92 | 0.50 |
| Example 34 | 1.16 | 1.16 |
| RXDX-106 | 6.40 | 3.30 |

As can be seen from Table 4 above, the compounds disclosed herein are effective in inhibiting the activity of kinases Mer and Tyro3. Compared with the control substance RXDX-106, part of the compounds disclosed herein show higher inhibitory activity.

Test Example 3: Inhibitory activity of compounds disclosed herein against EBC-1 cells

Test method: the inhibitory activity of the compounds against EBC-1 cell proliferation was evaluated using CellTiter-Glo® Luminescent Cell Viability Assay Kit (Promega).

Instruments: Spectramax M3 multi-functional microplate reader (Molecular Devices); Model 311 Series $CO_2$ incubator (Thermo Scientific); Model 1300 Series A2 biosafety cabinet (Thermo Scientific); CKX41SF inverted microscope (Olympus); IC1000 cell counter (Countstar); KK25E76TI refrigerator (SIEMENS); QB-9001 microporous quick shaker (Kylin-Bell).

Test materials: fetal bovine serum FBS (Thermo Fisher, Cat. No. 10099-141, Lot. No. 1966174C); CellTiter-Glo® fluorescent cell viability test reagent (Promega, Cat. No. G7572, Lot. No. 0000310975); 96-well transparent flat-bottom black-wall cell culture plate (Thermo Fisher, Cat. No., Lot. No. 1207365); RPMI1640 culture medium (GE, Cat. No. SH30809.01, Lot. No. AD17321266); MEM culture medium (GE, Cat. No. SH30024.01, Lot. No. AC10232463); NEAA (Thermo Fisher, Cat. No. 11140-050, Lot. No. 1872982); control substance cabozantinib (synthesized according to the method disclosed in WO201101763A1); EBC-1 cells (from Nanjing Cobioer Biotechnology Co., Ltd.; EBC-1 cells are human lung squamous carcinoma cells, which are cultured in complete culture medium (MEM+10% FBS+0.01 mM NEAA) at 37 °C/5%$CO_2$/95% humidity, the doubling time of growth is about 32 h, and the passage ratio is 1:6).

Test procedures: when thawing EBC-1 cells, the cell cryopreservation tube was shaken rapidly in a water bath at 37 °C to thaw the cells in 1 min. The cell suspension after thawing was mixed with RPMI1640 culture medium containing 10% FBS and centrifuged for 5 min at 1000 rpm, and the supernatant was discarded. The cell pellet was suspended in 5 mL of complete culture medium. The suspension was placed in a cell culture flask with a bottom area of 25 $cm^2$, and cultured in a cell incubator at 37 °C/95% humidity/5% $CO_2$. Cell passage was performed when cell confluence reached about 80%. When the cells were passaged, the original cell suspension was directly made uniform by pipetting. 1/6 of the cell suspension was kept, added with 5 mL of new complete culture medium and then made uniform by pipetting. The cell culture flask was then placed in a cell incubator for further culturing. Cell plating was performed when the cell confluence reached about 80% again. With reference to the method for cell passage, 1/6 cell suspension was kept for further culturing when the cells were plated, and the remaining 5/6 of cell suspension was placed in a 15 mL centrifuge tube. Cell viability was detected by trypan blue exclusion using an IC1000 cell counter (Countstar) to ensure that cell viability was above 90%. Cell suspension at a density of $3.33\times10^4$ viable cells/mL was prepared using complete culture medium, and 90 μL of the cell suspension was added into 96-well cell culture plates, so that the cell density in the cell culture plates (day 0 plate and test compound plate) was 3000 viable cells/well. A control group that contains no cell or compound but only complete culture medium and a control group that contains no compound but cells were set. The cell plates were incubated overnight in a cell incubator. When adding compound, the compounds disclosed herein and the control substance cabozantinib were each dissolved in DMSO and serially diluted to give a 10-fold solution. 10 μL of the above solution was added into a corresponding cell culture plate to ensure that the initial concentration of the compound was 10 μM, the dilution factor of adjacent concentration was 3.16, and the DMSO content in the cell culture plate was 0.1%. The cell plates were then incubated in the cell incubator for 72 h. During detection, CellTiter-Glo reagent (namely CellTiter-Glo® fluorescent cell viability test reagent, Promega, Cat. No. G7572, Lot. No. 0000310975) was melted, and the cell plate was equilibrated at room temperature for 30 min. The cell plate was added with the CellTiter-Glo reagent at 100 μL per well, and then shaken on a QB-9001 microporous quick shaker (Kylin-Bell) for 5 min to fully lyse the cells. The cell plate was left to stand at room temperature for 20 min to stabilize luminescence signals, and the luminescence value of each well was scanned by a Spectramax M3 multi-functional microplate reader (Molecular Devices) at full wavelength.

Test samples: example compounds of the present invention and cabozantinib (positive control compound).

Data analysis: cell viability was calculated for compounds at various concentrations using the following formula:

$$\text{Cell viability (\%)} = (\text{Lum}_{\text{test compound}} - \text{Lum}_{\text{culture solution control}}) / (\text{Lum}_{\text{cell control}} - \text{Lum}_{\text{culture solution control}}) \times 100\%,$$

where Lum refers to the luminescence value of each well of the test compound plate read by the multi-functional microplate reader.

The data were analyzed using GraphPad Prism 7.0 software, fitted with nonlinear S-curve regression to give dose-response curves, and **$IC_{50}$** values were calculated therefrom.

The inhibitory activity of the compounds disclosed herein against EBC-1 cells is shown in Table 5 below.

Table 5. Inhibitory activity ($IC_{50}$) of compounds disclosed herein against EBC-1 cells

| **Compounds** | **$IC_{50}$ (nM)** |
|---|---|
| Example 2 | 24 |
| Example 3 | 22 |
| Example 4 | 30 |
| Example 6 | 13 |
| Example 7 | 27 |
| Example 10 | 208 |
| Example 11 | 91 |
| Example 12 | 98 |
| Example 15 | 190 |
| Example 18 | 298 |
| Example 20 | 106 |
| Example 21 | 109 |
| Example 22 | 84 |
| Example 23 | 74 |
| Example 24 | 45 |
| Example 25 | 115 |
| Example 26 | 132 |
| Example 29A | 82.7 |
| Cabozantinib | 37 |

As can be seen from Table 5 above, the compounds disclosed herein are effective in inhibiting the activity of EBC-1 cells. Compared with the positive control drug cabozantinib, the compounds disclosed herein show similar inhibitory activity. Meanwhile, because EBC-1 is a lung cancer cell line driven by c-MET, the inhibition effect of the compounds disclosed herein against c-MET kinase targets is further verified due to their inhibition against activity of EBC-1 cells.

Test Example 4: Inhibitory activity of compounds disclosed herein against Ba/F3 Axl cells

Test method: the inhibitory activity of the compounds against Ba/F3 Axl cell proliferation was evaluated using CellTiter-Glo® Luminescent Cell Viability Assay Kit (Promega).

Instruments: Spectramax M3 multi-functional microplate reader (Molecular Devices); Model 311 Series $CO_2$ incubator (Thermo Scientific); Model 1300 Series A2 biosafety cabinet (Thermo Scientific); CKX41SF inverted microscope (Olympus); IC1000 cell counter (Countstar); KK25E76TI refrigerator (SIEMENS); QB-9001 microporous quick shaker (Kylin-Bell).

Test materials: fetal bovine serum FBS (Thermo Fisher, Cat. No. 10099-141, Lot. No. 1966174C); CellTiter-Glo® fluorescent cell viability test reagent (Promega, Cat. No. G7572, Lot. No. 0000310975); 96-well transparent flat-bottom black-wall cell culture plate (Thermo Fisher, Cat. No., Lot. No. 1207365); RPMI1640 culture medium (GE, Cat. No. SH30809.01, Lot. No. AD17321266); Murine IL-3 (PeproTech, Cat. No. 213-13, Lot. No. 120948); rhGas6 (R&D Systems, Cat. No. 885-GSB, Lot. No. DFGX0417081); control substance bemcentinib (also named BGB324, Shanghai Bidepharm, Cat. No. BD559084, Lot. No. AQU341); Ba/F3 Axl cells (constructed by KYinno Biotechnology Co., Ltd., and cultured in complete culture medium (RPMI1640+10% FBS+100 ng/mL rhGas6) at 37 °C/5%$CO_2$/95% humidity; the doubling time of growth is about 20 h, and the passage ratio is 1:10; see Oncogene. 2009, 28:3442-3455; Oncotarget. FASEB J. 2017, 31(4): 1382-1397).

Test procedures: when thawing Ba/F3 Axl cells, the cell cryopreservation tube was shaken rapidly in a water bath at 37 °C to thaw the cells in 1 min. The cell suspension after thawing was mixed with RPMI1640 culture medium containing 10% FBS and centrifuged for 5 min at 1000 rpm, and the supernatant was discarded. The cell pellet was suspended in 5 mL of complete culture medium. The suspension was placed in a cell culture flask with a bottom area of 25 $cm^2$, and cultured in a cell incubator at 37 °C/95% humidity/5% $CO_2$. Cell passage was performed at a cell density of $2\times10^6$ viable cells/mL. When the cells were passaged, the original cell suspension was directly made uniform by pipetting. 1/10 (namely 0.5 mL) of the cell suspension was kept, added with 4.5 mL of new complete culture medium and then made uniform by pipetting. The cell culture flask was then placed in a cell incubator for further culturing. Cell plating was performed when the cell confluence reached $2\times10^6$ viable cells/mL again. With reference to the method for cell passage, 1/10 (namely 0.5 mL) of the cell suspension was kept for further culturing when the cells were plated, and the remaining cell suspension was placed in a 15 mL centrifuge tube. The supernatant was discarded after centrifugation, and 5 mL of complete culture medium was used to resuspend the cells. Cell viability was detected by trypan blue exclusion using an IC1000 cell counter (Countstar) to ensure that cell viability was above 90%. Cell suspension at a density of $5.56\times10^4$ viable cells/mL was prepared using complete culture medium, and 90 μL of the cell suspension was added into 96-well cell culture plates, so that the cell density in the cell culture plates (day 0 plate and test compound plate) was 5000 viable cells/well. A control group that contains no cell or compound but only complete culture medium and a control group that contains no compound but cells were set. The cell plates were incubated overnight in a cell incubator. When adding compound, the compounds disclosed herein and the control substance bemcentinib were each dissolved in DMSO and serially diluted to give a 10-fold solution. 10 μL of the above solution was added into a corresponding cell culture plate to ensure that the initial concentration of the compound was 100 nM, the dilution factor of adjacent concentration was 3.16, and the DMSO content in the cell culture plate was 0.1%. The cell plates were then incubated in the cell incubator for 72 h. During detection, CellTiter-Glo reagent (namely CellTiter-Glo® fluorescent cell viability test reagent, Promega, Cat. No. G7572, Lot. No. 0000310975) was melted, and the cell plate was equilibrated at room temperature for 30 min. The cell plate was added with the CellTiter-Glo reagent at 100 μL per well, and then shaken on a QB-9001 microporous quick shaker (Kylin-Bell) for 5 min to fully lyse the cells. The cell plate was left to stand at room temperature for 20 min to stabilize luminescence signals, and the luminescence value of each well was scanned by a Spectramax M3 multi-functional microplate reader (Molecular Devices) at full wavelength.

Test samples: example compounds of the present invention and bemcentinib (positive control compound).

Data analysis: cell viability was calculated for compounds at various concentrations using the following formula:

$$\text{Cell viability (\%)} = (\text{Lum}_{\text{test compound}} - \text{Lum}_{\text{culture solution control}}) / (\text{Lum}_{\text{cell control}} - \text{Lum}_{\text{culture solution control}}) \times 100\%,$$

where Lum refers to the luminescence value of each well of the test compound plate read by the multi-functional microplate reader.

The data were analyzed using GraphPad Prism 7.0 software, fitted with nonlinear S-curve regression to give dose-response curves, and **$IC_{50}$** values were calculated therefrom.

The inhibitory activity of compounds disclosed herein against Ba/F3 Axl cells is shown in Table 6 below.

Table 6. Inhibitory activity ($IC_{50}$) of compounds disclosed herein against Ba/F3 Axl cells

| Compounds | $IC_{50}$ (nM) |
|---|---|
| Example 3 | 0.29 |
| Example 4 | 0.57 |
| Example 6 | 0.49 |
| Example 21 | 0.29 |
| Example 24 | 0.36 |

(continued)

| Compounds | $IC_{50}$ (nM) |
|---|---|
| Example 33 | 0.23 |
| Example 34 | 0.28 |
| Bemcentinib | 26.9 |

As shown in the Table 6 above, compared with the control substance bemcentinib, the compounds disclosed herein show higher inhibitory activity against Ba/F3 Axl cell proliferation.

Test Example 5: *In vivo* anti-tumor activity of compounds disclosed herein in model mice with ectopically grafted tumor cells

$3\times10^6$ cells of human non-small cell lung cancer tumor cell strain EBC-1 (ATCC) were inoculated subcutaneously into BALB/c-nude model mice (Beijing AniKeeper Biotech, 10 female mice). When the subcutaneous tumors in mice each grew to 175.5 $mm^3$, the mice were administered intragastrically with test samples.

The mice were divided into a negative control group and an example compound group (compound of Example 3) (30 mg/kg) with 5 mice per group. The mice in the negative control group were administered with 10% solutol HS-15, which was prepared by adding 10 mL of solutol HS-15 to 90 mL of $ddH_2O$ and then mixing well by vortexing. The mice in the example compound group were administered with a compound solution at a concentration of 1 mg/mL which was prepared by dissolving the compound of Example 3 in 10% solutol HS-15. The mice in both the solvent control group and the example compound group were subjected to intragastric administration once daily for 28 days at a dosage of 10 μL per gram of body weight.

After the start of the administration, the body weight and tumor size of each mice were measured twice a week. The calculation formula for tumor size is as follows:

$$\text{Tumor volume (mm}^3\text{)} = 0.5 \times (\text{long diameter of tumor} \times \text{short diameter of tumor}^2).$$

The anti-tumor efficacy was evaluated based on the growth curve of the tumor (i.e., tumor volume per measurement versus its treatment days) and relative tumor volume during treatment. The relative tumor inhibition (TGI) was calculated according to the following formula:

$$\text{TGI} = 1 - \text{T/C (\%)}.$$

T/C% is the relative tumor proliferation rate, i.e., the percentage of the relative tumor volume or tumor weight of the example compound group and the solvent control group at a certain time point. T and C are the relative tumor volumes (RTVs) of the example compound group and the solvent control group, respectively, at a particular time point. T/C% = $T_{RTV}/C_{RTV} \times 100\%$ ($T_{RTV}$: mean RTV of the example compound group; $C_{RTV}$: mean RTV of the solvent control group).

Relative tumor volume RTV was calculated as follows: RTV = Vt - V0, where V0 is the tumor volume of the animal at the time of grouping, and Vt is the tumor volume of the animal after treatment.

FIG. 1 shows the growth change in tumor volume of mice in the example compound group and the solvent control group. As shown in the figure, the compound disclosed herein can effectively inhibit the growth of tumor cells in model mice, and the tumor growth inhibition (TGI) is up to 108%.

FIG. 2 shows the change of body weight as a function of treatment time in mice of the example compound group and the solvent control group. As shown in the figure, the body weight of tumor-bearing mice does not change significantly during the experiment, indicating that the compound disclosed herein features good safety and tolerance.

In the present invention, it is proved by experiments that the compound disclosed herein can effectively inhibit the activity of tyrosine kinases such as Axl, Mer, Tyro3 or c-MET, and can highly inhibit the growth of tumor cells in mice. Therefore, the compound disclosed herein has wide application prospect in treating diseases related to tyrosine kinases such as Axl, Mer, Tyro3 or c-MET, and particularly in treating cancers.

## Claims

**1.** A compound of formula (I),

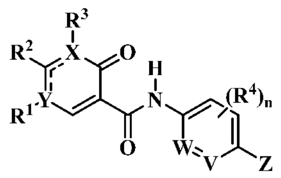

(I)

or a mesomer thereof, a racemate, an enantiomer, or a diastereomer thereof, or a mixture thereof, or a pharmaceutically acceptable salt thereof,
wherein,

"======" represents a single bond or a double bond;
X and Y are each independently C or N;
W and V are each independently CH or N;
Z is

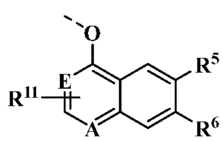 , 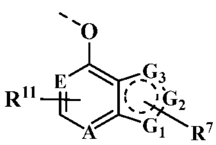 , 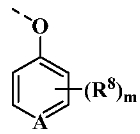 , or 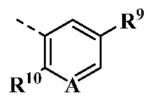 ;

A and E are each independently CH or N;
$G_1$, $G_2$, and $G_3$ are each independently C, N, O, or S;
$R^1$ is hydrogen, halogen, alkyl, alkenyl, alkynyl, cycloalkyl, or heterocyclyl, wherein the alkyl, alkenyl, alkynyl, cycloalkyl, or heterocyclyl is optionally further substituted with one or more groups, each independently selected from halogen, amino, nitro, cyano, hydroxyl, sulfydryl, carboxyl, an ester group, oxo, $NR^aR^b$, alkyl, alkoxy, cycloalkyl, heterocyclyl, aryl, and heteroaryl;
$R^2$ is hydrogen, halogen, oxo, hydroxyl, cyano, alkyl, cycloalkyl, heterocyclyl, $NR^aR^b$, $NHC(O)R^a$, and $NHS(O)_mR^a$, wherein the alkyl, cycloalkyl, or heterocyclyl is optionally further substituted with one or more groups, each independently selected from halogen, hydroxyl, sulfydryl, cyano, alkyl, $OR^a$, $SR^a$, $NR^aR^b$, and $C(O)NR^aR^b$;
$R^3$ is alkenyl, alkynyl, aryl, or heteroaryl, wherein the alkenyl, alkynyl, aryl, or heteroaryl is optionally further substituted with $R^a$;
$R^4$ is hydrogen, halogen, cyano, alkyl, haloalkyl, alkoxy, or haloalkoxy;
$R^5$ and $R^6$ are each independently hydrogen, halogen, cyano, $OR^a$, $SR^a$, $O(CH_2)_pNR^aR^b$, $O(CH_2)pOR^a$, $NR^aR^b$, $C(O)R^a$, $C(O)OR^a$, $OC(O)R^a$, $C(O)NR^aR^b$, or $OC(O)NR^aR^b$, or
$R^5$ and $R^6$ together with the atoms to which they are attached form oxacycloalkyl, in which the oxygen atom is attached to the phenyl ring;
$R^7$ is hydrogen, halogen, $NR^aR^b$, alkyl, cycloalkyl, heterocyclyl, aryl, or heteroaryl, wherein the alkyl, cycloalkyl, heterocyclyl, aryl, or heteroaryl is optionally further substituted with one or more groups, each independently selected from halogen, amino, nitro, cyano, hydroxyl, sulfydryl, carboxyl, an ester group, oxo, alkyl, alkoxy, cycloalkyl, heterocyclyl, aryl, and heteroaryl;
each $R^8$ is independently hydrogen, halogen, $NR^aR^b$, alkyl, cycloalkyl, heterocyclyl, aryl, and heteroaryl, wherein the alkyl, cycloalkyl, heterocyclyl, aryl, or heteroaryl is optionally further substituted with one or more groups, each independently selected from halogen, amino, nitro, cyano, hydroxyl, sulfydryl, carboxyl, an ester group, oxo, alkyl, alkoxy, cycloalkyl, heterocyclyl, aryl, and heteroaryl;
$R^9$ is aryl or heteroaryl, wherein the aryl or heteroaryl is optionally further substituted with one or more Q groups;
each Q is independently halogen, alkyl, cycloalkyl, heterocyclyl, aryl, heteroaryl, $OR^a$, $SR^a$, $O(CH_2)_pNR^aR^b$, $O(CH_2)pOR^a$, $NR^aR^b$, $C(O)R^a$, $C(O)OR^a$, $OC(O)R^a$, $C(O)NR^aR^b$, and $OC(O)NR^aR^b$, wherein the alkyl, cycloalkyl, heterocyclyl, aryl, or heteroaryl is optionally further substituted with one or more groups, each independently selected from halogen, amino, nitro, cyano, hydroxyl, sulfydryl, carboxyl, an ester group, oxo, alkyl,

alkoxy, cycloalkyl, heterocyclyl, aryl, and heteroaryl;

$R^{10}$ is hydrogen, halogen, alkyl, or $NR^aR^b$, wherein the alkyl is optionally further substituted with one or more halogens;

$R^{11}$ is hydrogen, halogen, cyano, amino, hydroxyl, alkyl, cycloalkyl, heterocyclyl, aryl, or heteroaryl, wherein the alkyl, cycloalkyl, heterocyclyl, aryl, or heteroaryl is optionally further substituted with one or more groups, each independently selected from halogen, amino, nitro, cyano, hydroxyl, sulfydryl, carboxyl, an ester group, oxo, alkyl, alkoxy, cycloalkyl, heterocyclyl, aryl, and heteroaryl;

$R^a$ and $R^b$ are each independently hydrogen, halogen, hydroxyl, nitro, cyano, oxo, alkyl, alkoxy, cycloalkyl, heterocyclyl, aryl, and heteroaryl, wherein the alkyl, alkoxy, cycloalkyl, heterocyclyl, aryl, or heteroaryl is optionally further substituted with one or more groups, each independently selected from halogen, amino, nitro, cyano, hydroxyl, sulfydryl, carboxyl, an ester group, oxo, alkyl, alkoxy, cycloalkyl, heterocyclyl, aryl, and heteroaryl, or

$R^a$ and $R^b$ together with the nitrogen atom to which they are attached form nitrogen-containing heterocyclyl, wherein the nitrogen-containing heterocyclyl is optionally further substituted with one or more groups, each independently selected from halogen, amino, nitro, cyano, oxo, hydroxyl, sulfydryl, carboxyl, an ester group, alkyl, alkoxy, cycloalkyl, heterocyclyl, aryl, and heteroaryl;

m is an integer from 1 to 4;

n is an integer from 0 to 4;

p is an integer from 1 to 6; and

any one or more H atoms in the compound of formula (I) are optionally further substituted with D atoms.

**2.** The compound of formula (I) according to claim 1, being a compound of formula (II),

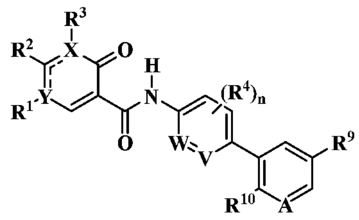

(II)

or a mesomer, a racemate, an enantiomer, or a diastereomer thereof, or a mixture thereof, or a pharmaceutically acceptable salt thereof, wherein, ------, $R^1$, $R^2$, $R^3$, $R^4$, $R^9$, $R^{10}$, X, Y, W, V, A and n are defined as in claim 1.

**3.** The compound of formula (I) according to claim 1, being a compound of formula (III),

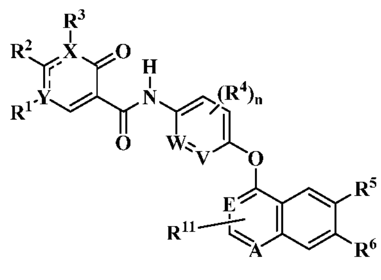

(III)

or a mesomer, a racemate, an enantiomer, or a diastereomer thereof, or a mixture thereof, or a pharmaceutically acceptable salt thereof, wherein, ------, $R^1$, $R^2$, $R^3$, $R^4$, $R^5$, $R^6$, $R^{11}$, X, Y, W, V, A, E and n are defined as in claim 1.

**4.** The compound of formula (I) according to claim 2, wherein W and V are CH, and A is N.

**5.** The compound of formula (I) according to claim 4, being a compound of formula (IV), (V), or (VI),

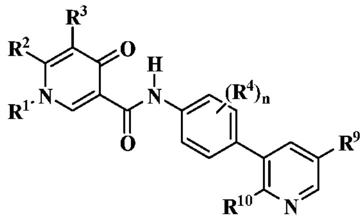

(IV)

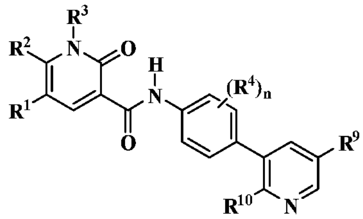

(V)

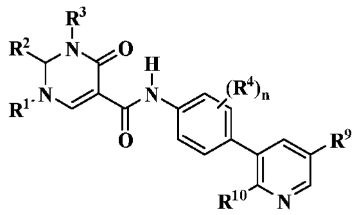

(VI)

wherein, $R^1$, $R^2$, $R^3$, $R^4$, $R^9$, $R^{10}$ and n are defined as in claim 1.

6. The compound of formula (I) according to claim 5, wherein
$R^9$ is aryl or heteroaryl, preferably $C_6$-$C_{10}$ aryl or 5-7 membered heteroaryl, wherein the aryl or heteroaryl is optionally further substituted with one or more Q groups;
each Q is independently alkyl, cycloalkyl, heterocyclyl, $OR^a$, $SR^a$, $O(CH_2)_pNR^aR^b$, $O(CH_2)pOR^a$, $NR^aR^b$, $OC(O)R^a$, or $OC(O)NR^aR^b$, wherein the alkyl, cycloalkyl, or heterocyclyl is optionally further substituted with one or more groups, each independently selected from halogen, amino, nitro, cyano, hydroxyl, sulfydryl, carboxyl, and ester group, oxo, alkyl, alkoxy, cycloalkyl, heterocyclyl, aryl. and heteroaryl;
$R^a$ and $R^b$ are each independently hydrogen or alkyl, wherein the alkyl is optionally further substituted with one or more groups, each independently selected from halogen, cycloalkyl, and heterocyclyl, or
$R^a$ and $R^b$ together with the nitrogen atom to which they are attached form nitrogen-containing heterocyclyl, preferably 5-7 membered nitrogen-containing heterocyclyl, wherein the nitrogen-containing heterocyclyl is optionally further substituted with one or more alkyl groups; and
p is an integer from 1 to 6.

7. The compound of formula (I) according to claim 5 or 6, wherein $R^{10}$ is amino.

8. The compound of formula (I) according to claim 3, wherein W and V are CH, E is CH, and A is N.

9. The compound of formula (I) according to claim 4, being a compound of formula (VII), (VIII), or (IX),

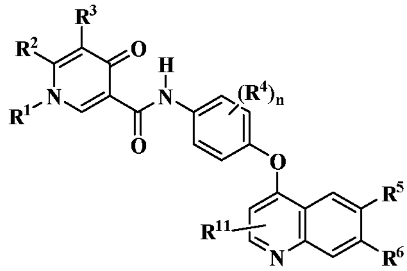

(VII)

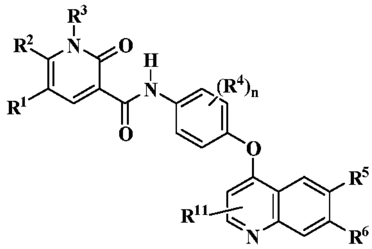

(VIII)

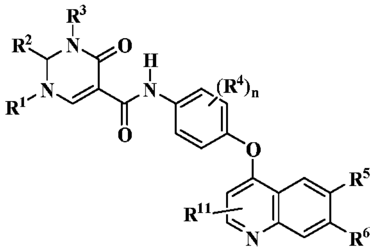

(IX)

wherein, $R^1$, $R^2$, $R^3$, $R^4$, $R^5$, $R^6$, $R^{11}$ and n are defined as in claim 1.

**10.** The compound of formula (I) according to claim 9, wherein
$R^5$ and $R^6$ are each independently cyano, $OR^a$, $SR^a$, $O(CH_2)_pNR^aR^b$, $O(CH_2)pOR^a$, $NR^aR^b$, $OC(O)R^a$, $C(O)NR^aR^b$, and $OC(O)NR^aR^b$, or
$R^5$ and $R^6$ together with the atoms they are attached form oxacycloalkyl, in which the oxygen atom is attached to the phenyl ring;
$R^a$ and $R^b$ are each independently hydrogen or alkyl, wherein the alkyl is optionally further substituted with one or more groups, each independently selected from halogen, cycloalkyl, and heterocyclyl, or
$R^a$ and $R^b$ together with the nitrogen atom to which they are attached form nitrogen-containing heterocyclyl, preferably 5-7 membered nitrogen-containing heterocyclyl, wherein the nitrogen-containing heterocyclyl is optionally further substituted with one or more alkyl groups; and
p is an integer from 1 to 6.

**11.** The compound of formula (I) according to claim 9 or 10, wherein $R^{11}$ is hydrogen or amino.

**12.** The compound of the formula (I) according to any one of claims 1 to 11, wherein
$R^1$ is halogen, alkyl, alkenyl, cycloalkyl, or heterocyclyl, wherein the alkyl, alkenyl, cycloalkyl, or heterocyclyl is optionally further substituted with one or more groups, each independently selected from halogen, amino, nitro, cyano, hydroxyl, sulfydryl, carboxyl, and ester group, oxo, $NR^aR^b$, alkyl, alkoxy, cycloalkyl, heterocyclyl, aryl, and heteroaryl; and
$R^a$ and $R^b$ are each independently hydrogen or alkyl, or
$R^a$ and $R^b$ together with the nitrogen atom to which they are attached form nitrogen-containing heterocyclyl, wherein the nitrogen-containing heterocyclyl is optionally further substituted with one or more alkyl groups.

**13.** The compound of formula (I) according to any one of claims 1 to 12, wherein
$R^2$ is hydrogen, oxo, cyano, hydroxyl, alkyl, cycloalkyl, or heterocyclyl, preferably oxo, cyano, hydroxyl, or alkyl, wherein the alkyl, cycloalkyl, or heterocyclyl is optionally further substituted with one or more groups, each independently selected from halogen, hydroxyl, sulfydryl, cyano, alkyl, $OR^a$, $SR^a$, $NR^aR^b$, and $C(O)NR^aR^b$; and
$R^a$ and $R^b$ are each independently hydrogen or alkyl, wherein the alkyl is optionally further substituted with one or more halogen groups, or
$R^a$ and $R^b$ together with the nitrogen atom to which they are attached form nitrogen-containing heterocyclyl, wherein the nitrogen-containing heterocyclyl is optionally further substituted with one or more alkyl groups.

**14.** The compound of formula (I) according to any one of claims 1 to 13, wherein
$R^3$ is alkynyl, aryl, or heteroaryl, wherein the alkynyl, aryl, or heteroaryl is optionally further substituted with $R^a$; and each $R^a$ is independently hydrogen, halogen, cyano, alkyl, alkoxy, or cycloalkyl, wherein the alkyl, alkoxy, or cycloalkyl is optionally further substituted with one or more halogen groups.

**15.** The compound of formula (I) according to any one of claims 1 to 14, wherein
$R^4$ is hydrogen, halogen, cyano, alkyl, haloalkyl, alkoxy, or haloalkoxy, preferably halogen; and
n is an integer from 0 to 2.

**16.** The compound of formula (I) according to any one of claims 1 to 14, wherein any one or more H atoms in the compound are substituted with D atoms.

**17.** The compound of formula (I) according to any one of claims 1 to 16, selected from:

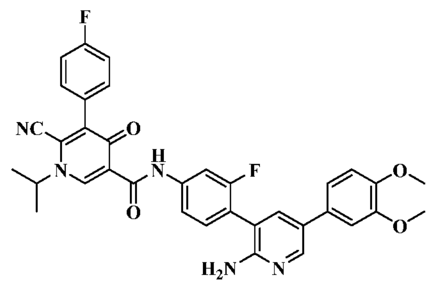
,
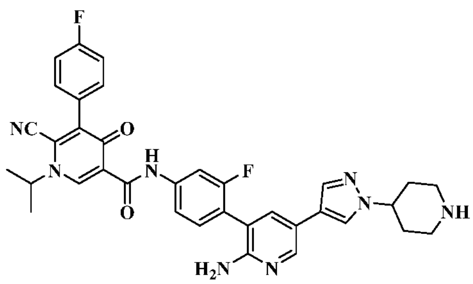
,
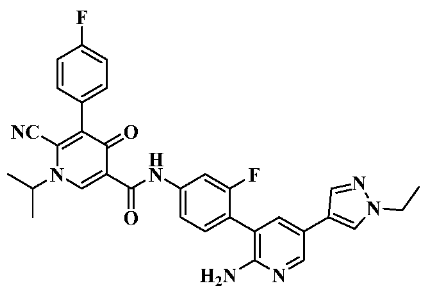
,
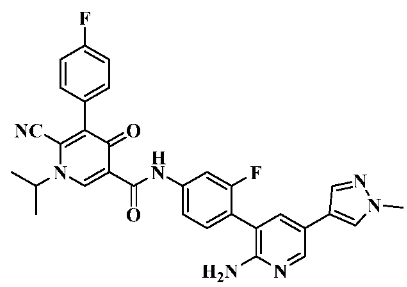
,
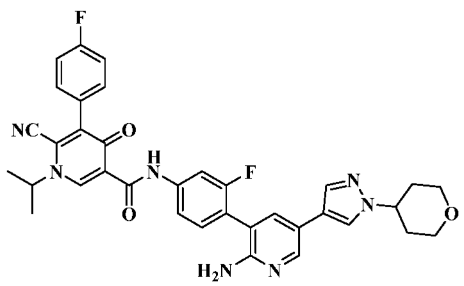
,
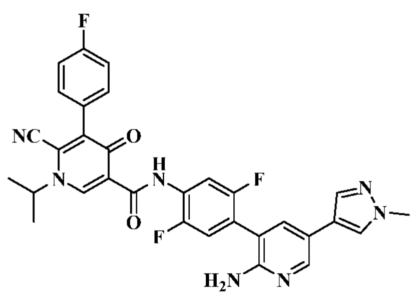
,

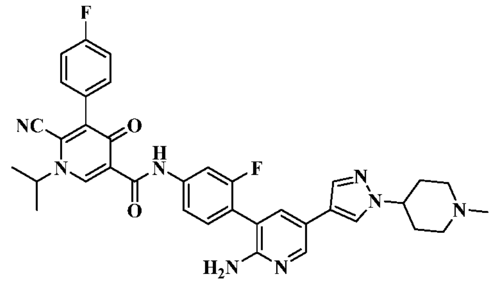
,
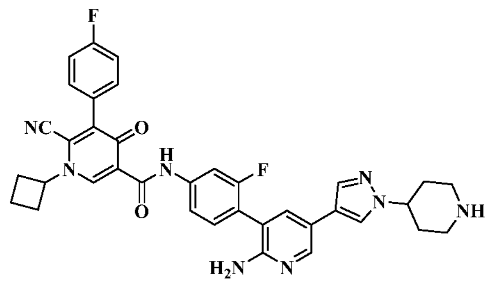
,
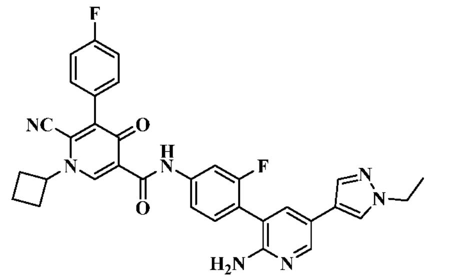
,
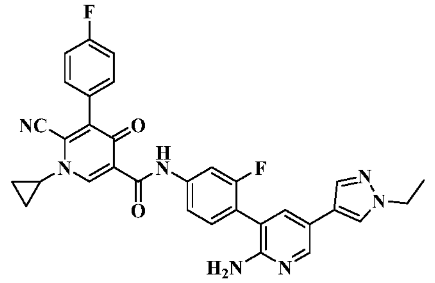
,
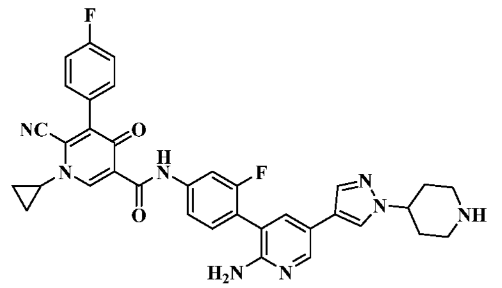
,
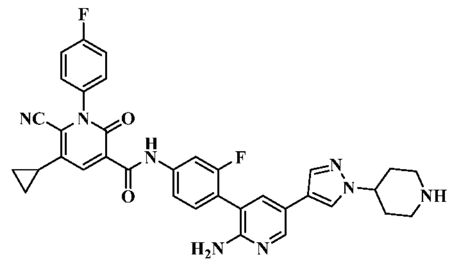
,
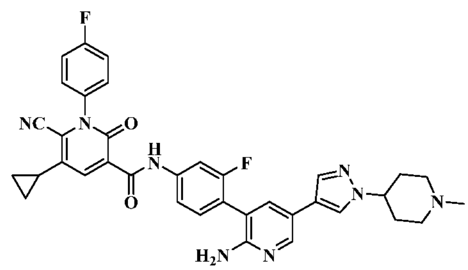
,
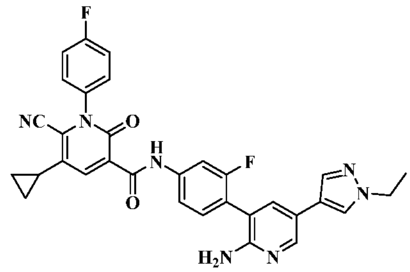
,
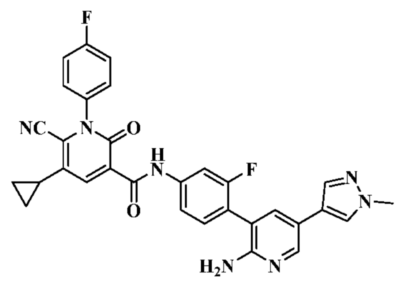
,
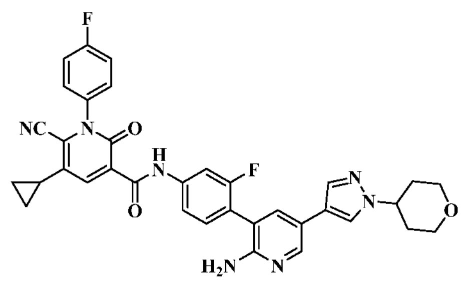
,

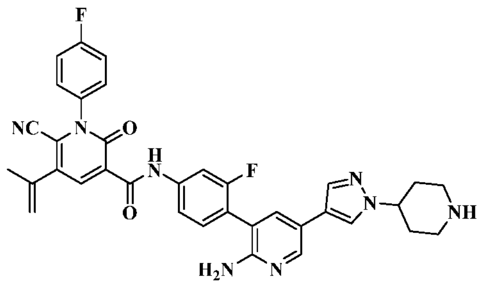

,

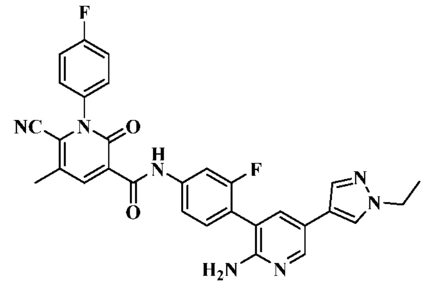

,

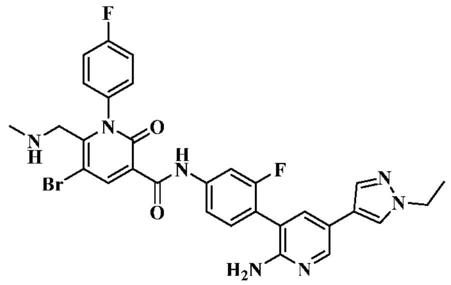

,

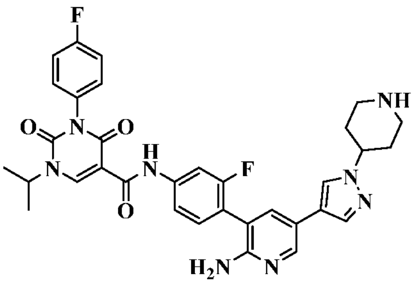

,

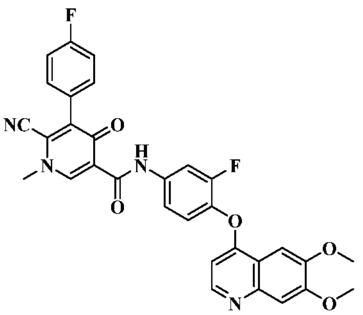

,

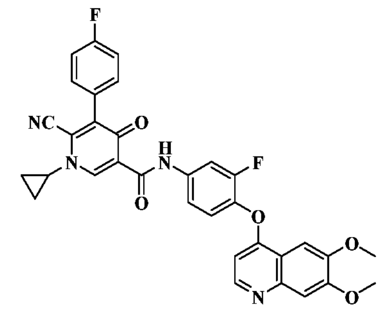

,

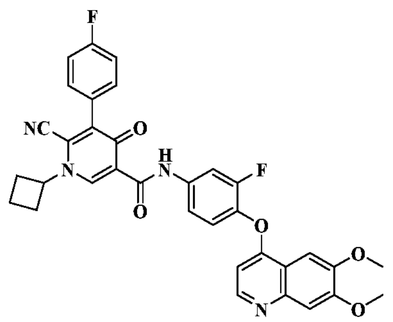

,

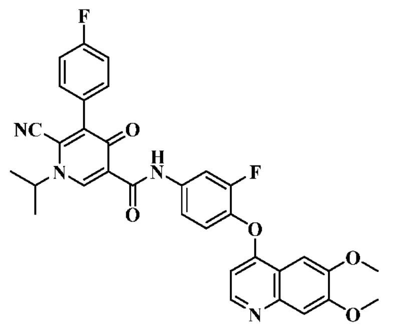

,

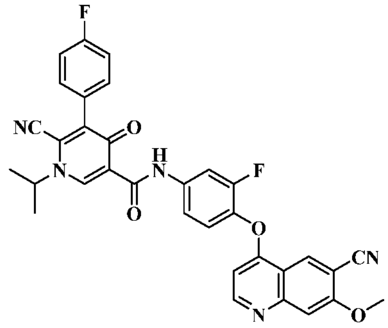

,

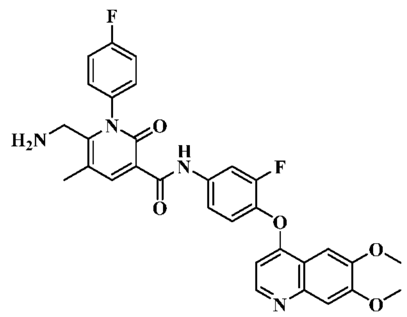

,

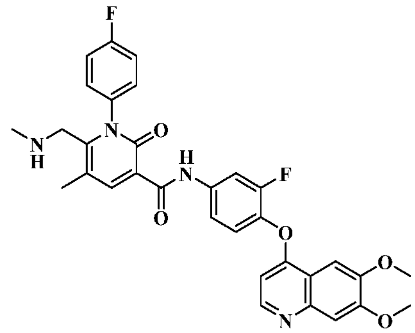
,

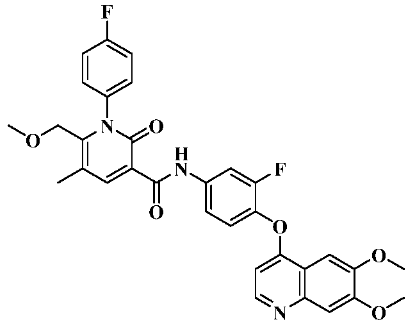
,

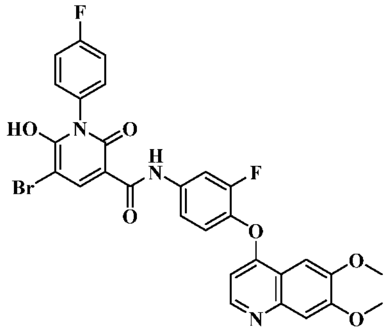
,

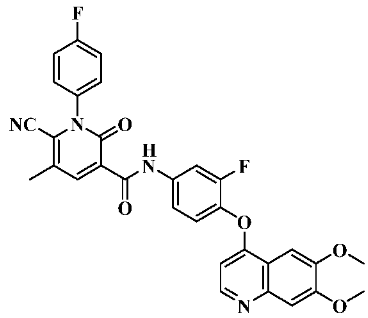
,

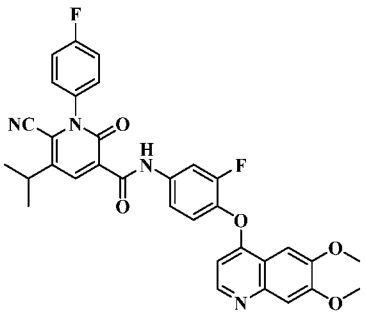
,

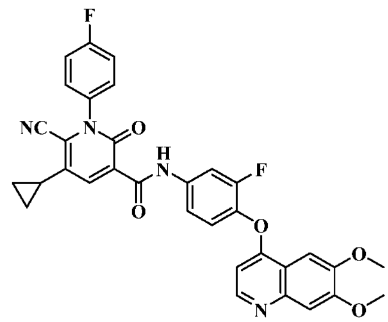
,

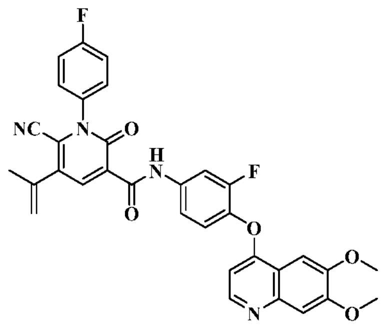
,

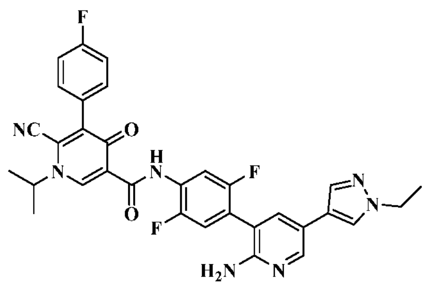
,

and

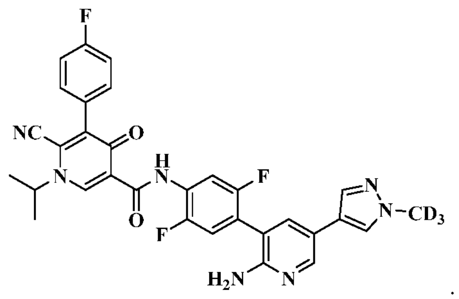

18. A method for preparing the compound of formula (I) according to any one of claims 1 to 17, comprising the step of:

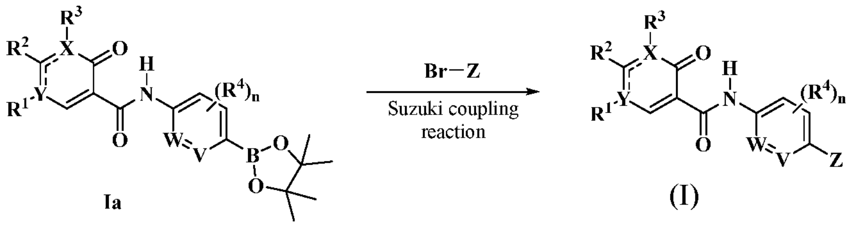


Ia

(I)

coupling pinacol borate Ia with aromatic bromide (Br-Z) *via* the Suzuki reaction in a solvent in the presence of a catalyst and a base to form the compound of formula (I), the catalyst being preferably Pd(dppf)$_2$, the base being preferably $K_2CO_3$, and the solvent being preferably dioxane and water;
wherein $R^1$, $R^2$, $R^3$, $R^4$, Z, X, Y, W, V and n are defined as in claim 1.

19. A method for preparing the compound of formula (I) according to any one of claims 1 to 17, comprising the step of:

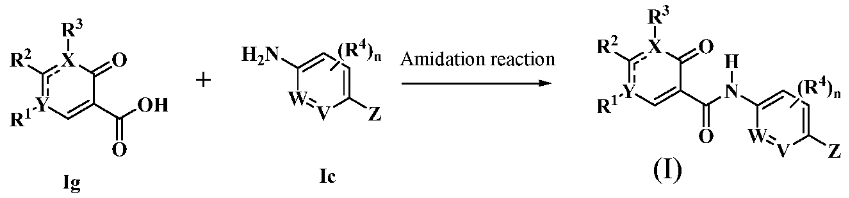


Ig Ic (I)

reacting carboxylic acid compound Ig and aromatic amine compound Ic in the presence of a coupling agent and a base to form the compound of formula (I), the coupling agent being preferably HATU, and the base being preferably triethylamine;
wherein $R^1$, $R^2$, $R^3$, $R^4$, Z, X, Y, W, V and n are defined as in claim 1.

20. A method for preparing the compound of formula (I) according to any one of claims 1 to 17, comprising the steps of:
when $R^2$ = CN,

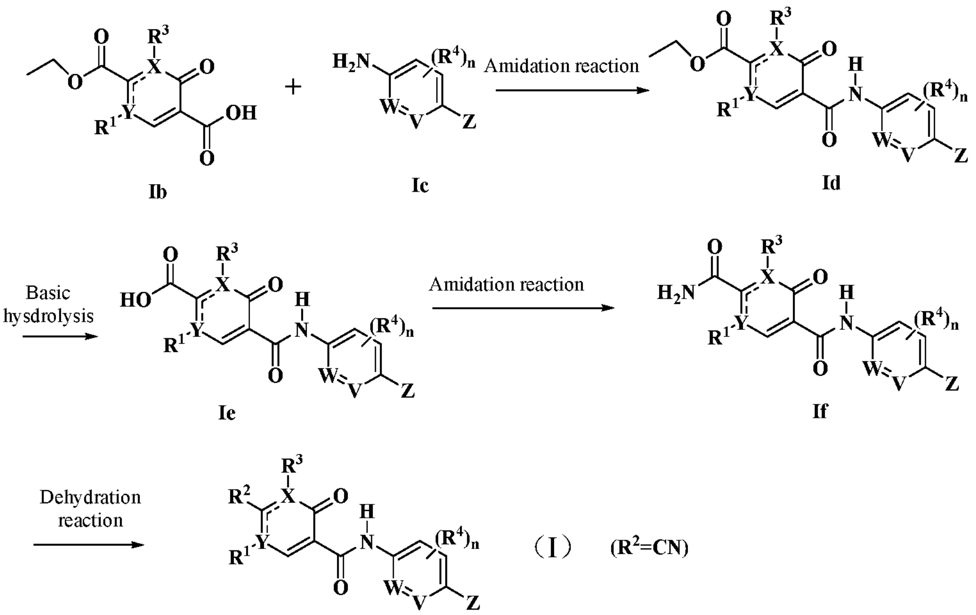

Ib Ic Id Ie If (I)

step 1: reacting carboxylic acid (Ib) with aromatic amine (Ic) in the presence of a coupling agent and a base to form arylamide intermediate (Id), the coupling agent being preferably HATU, and the base being preferably *N,N*-diisopropylethylamine;
step 2: hydrolyzing arylamide intermediate (Id) in a solvent in the presence of a base to form carboxylic acid intermediate (Ie), the base being preferably LiOH, and the solvent being preferably methanol-water solution;
step 3: reacting carboxylic acid intermediate (Ie) and ammonium chloride in the presence of a catalyst and a base to form dicarboxamide intermediate (If), the catalyst being preferably PyBrOP, and the base being preferably DIPEA; and
step 4: dehydrating dicarboxamide intermediate (If) in the presence of a dehydrating agent and a base to form the compound of formula (I), the dehydrating agent being preferably trifluoroacetic anhydride, and the base being preferably triethylamine;

wherein $R^1$, $R^2$, $R^3$, $R^4$, Z, X, Y, W, V and n are defined as in claim 1.

**21.** A pharmaceutical composition comprising the compound of formula (I) according to any one of claims 1 to 17 and a pharmaceutically acceptable carrier or excipient.

**22.** Use of the compound of formula (I) according to any one of claims 1 to 17 or the pharmaceutical composition according to claim 21 as a tyrosine kinase inhibitor, wherein the tyrosine kinase is preferably Axl, Mer, Tyro3, or c-MET.

**23.** Use of the compound of formula (I) according to any one of claims 1 to 17 or the pharmaceutical composition according to claim 21 in the manufacture of a medicament for treating a disease associated with tyrosine kinase activity, wherein the disease is preferably bladder cancer, breast cancer, cervical cancer, colorectal cancer, intestinal cancer, gastric cancer, head and neck cancer, kidney cancer, liver cancer, lung cancer, ovarian cancer, prostate cancer, testicular cancer, esophageal cancer, gallbladder cancer, pancreatic cancer, thyroid cancer, skin cancer, brain cancer, bone cancer, soft tissue cancer, leukemia, or lymph cancer, more preferably leukemia, liver cancer, lung cancer, kidney cancer, breast cancer, or colorectal cancer, and further more preferably leukemia, liver cancer, lung cancer, kidney cancer, breast cancer, gastric cancer, or colorectal cancer.

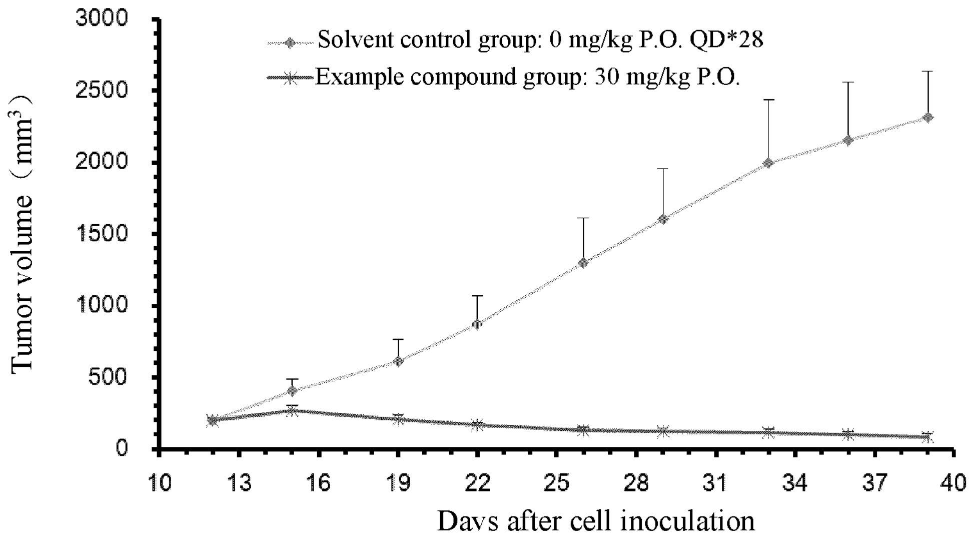
Solvent control group: 0 mg/kg P.O. QD*28
Example compound group: 30 mg/kg P.O.
Tumor volume（mm³）
3000
2500
2000
1500
1000
500
0
10
13
16
19
22
25
28
31
34
37
40
Days after cell inoculation

FIG. 1

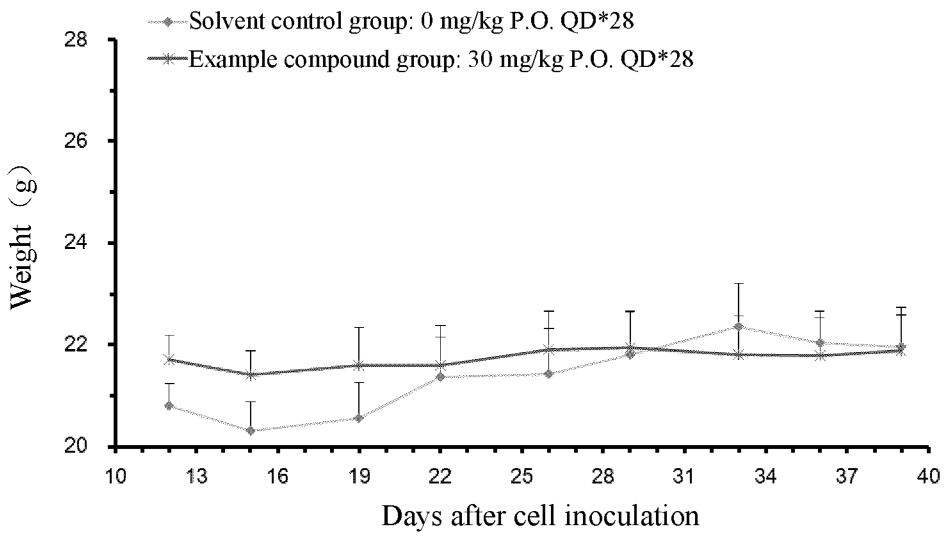
Solvent control group: 0 mg/kg P.O. QD*28
Example compound group: 30 mg/kg P.O. QD*28
Weight（g）
28
26
24
22
20
10
13
16
19
22
25
28
31
34
37
40
Days after cell inoculation

FIG. 2

## INTERNATIONAL SEARCH REPORT

International application No.
**PCT/CN2019/081987**

**A. CLASSIFICATION OF SUBJECT MATTER**

C07D 401/12(2006.01)i; C07D 491/04(2006.01)i; A61K 31/506(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, WPI, EPODOC, STN (REGISTRY, CAPLUS), MET, 抑制剂, 癌症, inhibitor?, cancer, tumor

**C. DOCUMENTS CONSIDERED TO BE RELEVANT**

| Category* | Citation of document, with indication, where appropriate, of the relevant passages | Relevant to claim No. |
|---|---|---|
| X | CN 101128199 A (BRISTOL-MYERS SQUIBB COMPANY) 20 February 2008 (2008-02-20) embodiments 55, 57, 101, 114, 115, 120, 130, 146, claim 1, and description, pages 26 and 27 | 1-23 |
| X | CN 101005843 A (BRISTOL-MYERS SQUIBB COMPANY) 25 July 2007 (2007-07-25) embodiments 242, 245, 248, 249, 253, 267, 290, 292, 297, 313, 314, 322-325 and 327-330, and claim 1 | 1-23 |
| X | CN 101528702 A (ARRAY BIOPHARMA INC.) 09 September 2009 (2009-09-09) embodiment 82, and claim 1 | 1-23 |
| A | CN 103958497 A (CEPHALON INC.) 30 July 2014 (2014-07-30) claims 1-16 | 1-23 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 June 2019** | **11 July 2019** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/081987**

| Patent document cited in search report | Publication date (day/month/year) | Patent family member(s) | Publication date (day/month/year) |
|---|---|---|---|
| CN 101128199 A | 20 February 2008 | CN 101128199 B | 24 July 2013 |
| | | UA 85087 C2 | 25 December 2008 |
| | | ES 2359836 T3 | 27 May 2011 |
| | | ZA 200608775 B | 25 June 2008 |
| | | ZA 200608775 A | 25 June 2008 |
| CN 101005843 A | 25 July 2007 | CN 1993130 A | 04 July 2007 |
| | | CN 1993130 B | 23 June 2010 |
| | | CN 100577663 C | 06 January 2010 |
| | | ZA 200610780 B | 26 November 2008 |
| | | ZA 200610780 A | 26 November 2008 |
| | | CN 101027305 A | 29 August 2007 |
| CN 101528702 A | 09 September 2009 | EP 2032538 A2 | 11 March 2009 |
| | | JP 2009539878 A | 19 November 2009 |
| | | CA 2655128 A1 | 21 December 2007 |
| | | US 2011053931 A1 | 03 March 2011 |
| | | WO 2007146824 A2 | 21 December 2007 |
| | | WO 2007146824 A3 | 10 April 2008 |
| CN 103958497 A | 30 July 2014 | ES 2614824 T3 | 02 June 2017 |
| | | AU 2012339640 A1 | 05 June 2014 |
| | | HK 1202528 A1 | 02 October 2015 |
| | | PH 12016501535 A1 | 22 May 2017 |
| | | PT 2780338 T | 17 February 2017 |
| | | EA 201490971 A1 | 30 September 2014 |
| | | JP 6051434 B2 | 27 December 2016 |
| | | MX 2014005766 A | 30 May 2014 |
| | | IL 245658 D0 | 30 June 2016 |
| | | IL 232405 D0 | 01 July 2014 |
| | | CA 2852697 A1 | 23 May 2013 |
| | | JP 6404299 B2 | 10 October 2018 |
| | | JP 2014533287 A | 11 December 2014 |
| | | UY 34451 A | 31 May 2013 |
| | | US 9522902 B2 | 20 December 2016 |
| | | BR 112014011548 A2 | 09 May 2017 |
| | | US 9120778 B2 | 01 September 2015 |
| | | AR 088872 A1 | 16 July 2014 |
| | | EP 2780338 A1 | 24 September 2014 |
| | | US 2017050949 A1 | 23 February 2017 |
| | | US 2017320853 A1 | 09 November 2017 |
| | | HU E033032 T2 | 28 November 2017 |
| | | US 2015209358 A1 | 30 July 2015 |
| | | TW 201332992 A | 16 August 2013 |
| | | WO 2013074633 A1 | 23 May 2013 |
| | | NZ 712194 A | 27 May 2016 |
| | | JP 2017071612 A | 13 April 2017 |
| | | SI 2780338 T1 | 26 April 2017 |
| | | US 9029538 B2 | 12 May 2015 |
| | | TW I562989 B | 21 December 2016 |
| | | KR 20140090678 A | 17 July 2014 |
| | | DK 2780338 T3 | 19 December 2016 |
| | | US 9745283 B2 | 29 August 2017 |

Form PCT/ISA/210 (patent family annex) (January 2015)

**INTERNATIONAL SEARCH REPORT**
**Information on patent family members**

International application No.
**PCT/CN2019/081987**

| Patent document cited in search report | Publication date (day/month/year) | Patent family member(s) | | | Publication date (day/month/year) |
|---|---|---|---|---|---|
| | | US | 10017496 | B2 | 10 July 2018 |
| | | IL | 245658 | A | 29 June 2017 |
| | | UA | 114900 | C2 | 28 August 2017 |
| | | SG | 10201510307W | A | 28 January 2016 |
| | | AU | 2012339640 | B2 | 05 January 2017 |
| | | CN | 108047201 | A | 18 May 2018 |
| | | MX | 342241 | B | 21 September 2016 |
| | | ZA | 201402940 | B | 30 August 2017 |
| | | CN | 103958497 | B | 01 September 2017 |
| | | US | 2014275077 | A1 | 18 September 2014 |
| | | EP | 2780338 | B1 | 09 November 2016 |
| | | PL | 2780338 | T3 | 28 April 2017 |
| | | EA | 023521 | B1 | 30 June 2016 |
| | | US | 2015210670 | A1 | 30 July 2015 |
| | | IL | 252517 | D0 | 31 July 2017 |
| | | SG | 11201402004Y | A | 29 May 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201101763 A1
- WO 2013074633 A


### Non-patent literature cited in the description


- **T. W. GREENE ; G. M. WUTS.** Protective Groups in Organic Synthesis. Wiley, 1999
- *Oncogene,* 2009, vol. 28, 3442-3455
- *Oncotarget. FASEB J.,* 2017, vol. 31 (4), 1382-1397